# EUROPEAN PATENT APPLICATION

(11) **EP 2 284 540 A1**
(43) Date of publication of application: **16.02.2011**
(21) Application number: 09167018.2
(22) Date of filing: 31.07.2009
(51) Int. Cl.: G01N 33/68

(54) **Method of diagnosing organ failure**

(71) Applicant: BIOCRATES Life Sciences AG, 6020 Innsbruck (AT)
(72) Inventor: Deigner, Hans-Peter, 6020 Innsbruck (AT); Kohl, Matthias, 95326 Kulmbach (DE); Enot, David, 6020 Innsbruck (AT); Koal, Therese, 6020 Innsbruck (AT); Keller, Matthias, 45219 Essen (DE)
(74) Representative: Winter, Brandl, Fürniss, Hübner, Röss, Kaiser, Polte - Partnerschaft

(57) **Abstract**

The present invention relates to a reliable and statistically significant method for predicting the likelihood of an onset of an inflammation associated organ failure from a biological sample of a mammalian subject in vitro, by means of a subject's quantitative metabolomics profile comprising a plurality of endogenous metabolites, and comparing it with a quantitative reference metabolomics profile of a plurality of endogenous organ failure predictive target metabolites in order to predict whether the subject is likely or unlikely to develop an organ failure. Furthermore, the invention relates to the usefulness of endogenous organ failure predictive target metabolites in such a method, and finally, the present invention relates to a Kit for carrying out said method.

## Description

The present invention relates to a method for predicting the likelihood of an onset of an infection associated organ failure from a biological sample of a mammalian subject in vitro in accordance with claim 1, a use of one or a plurality of endogenous metabolites for predicting the likelihood of an onset of an infection associated organ failure from a biological sample of a mammalian subject in vitro according to claim 12 and to a Kit in accordance with claim 15.

The invention generally relates to biomarkers for organ failure as tools in clinical diagnosis for early detection of organ failure, therapy monitoring and methods based on the same biomarkers.

### BACKGROUND of the Invention

Organ failure (OF) strikes an estimated 200 000 people in the U.S.
annually and kills 60% of them. While organ failure may arise from an infection and hospitals are seeing more cases in part due to increasing numbers of immunosuppressed cancer and transplant patients, an increasing number of hospital patients are at risk.

The mortality of multiorgan dysfinction syndrome (MODS) in hospitals is around 50%. The main etiological factors for MODS still are severe infection, major operations, trauma and severe pancreatitis. (Zhang SW, Wang C, Yin CH, Wang H, Wang BE, Zhongguo Wei Zhong Bing Ji Jiu Yi Xue. 2004, 16, 328-32. Multi-center clinical study on the diagnostic criteria for multiple organ dysfunction syndrome with illness severity score system).

Diagnostics of OF and MODS so far relies on clinical criteria and scores such as the Atlanta criteria and Sepsis-Related Organ Failure Assessment (SOFA)-score as well as on the use of few unreliable protein marker. For instance, severe acute pancreatitis with systemic organ dysfunctions develops in about 25% of patients with acute pancreatitis. Biochemical parameters are limited to protein markers such as procalcitonin (PCT), C reactive protein (CRP) and interleukins (Beger HG, Rau BM, Severe acute pancreatitis: Clinical course and management World J Gastroenterol. 2007, 13, 5043-51). Organ failure in acute pancreatitis was predicted by using a combination of plasma interleukin 10 and serum calcium measurements (Early Prediction of Organ Failure by Combined Markers in Patients With Acute Pancreatitis Mentula P, Kylänpää M-L, Kemppainen E, Br J Surg, 92, 68 - 75, 2005). In trauma patients, interleukin 6 and interleukin 10 were used for multiple OF prediction (Lausevic Z, Lausevic M, Trbojevic-Stankovic J, Krstic S, Stojimirovic, Predicting multiple organ failure in patients with severe trauma B Can J Surg. 2008, 51, 97-102).

Severe sepsis also includes OF and occurs when one or more vital organs are compromised. It can lead to septic shock, which is marked by low blood pressure that does not respond to standard treatment, problems in vital organs, and oxygen deprivation. About half of patients who suffer septic shock die.

Early diagnosis of beginning OF, however, is difficult because its clinical signs can mimic other conditions. The complexity of the host's response during the systemic inflammatory response has complicated efforts towards understanding disease pathogenesis (Reviewed in Healy, Annul. Pharmacother. 36: 648-54 (2002).). Early diagnosis, however, is the key to saving more lives, but available diagnostics so far do not indicate beginning organ failure. Consequently, some labs have started to offer faster tests for OF markers to speed diagnosis.

Besides critical care medicine therapy such as antibiotics therapy and symptomatic therapy, the treatment of organ failure is still limited to preventive measures and symptomatic supportive strategies.

Current diagnostics in clinical routine is limited to a) clinical information b) use of basic biochemical clinical parameters as outlined below in the definitions Or unspecific biomarkers like C-reactive protein (CRP) or procalcitonin (PCT) with low sensitivities and specificities (Critical Care Medicine 2006; 34:1996-2003, Archives of Surgery 2007; 142:134-142).

Sepsis by definition comprises systemic inflammatory response syndrome (SIRS) and infection with pathogens.

Systemic inflammatory response syndrome (SIRS) is considered to be present when two or more of the following clinical findings are present:
1. Body temperature >38°C or <36°C;
2. Heart rate >90 min⁻¹;
3. Hyperventilation evidenced by a respiratory rate of >20 min⁻¹ or a PaCO₂ of <32 mm Hg; and
4. White blood cell count of >12,000 cells µL⁻¹ or <4,000 µL⁻¹

The quantitative metabolomics profile of the endogenous organ failure predictive target metabolites can be combined with any of the above classical clinical laboratory parameters.

Organ failure includes a systemic inflammatory response syndrome (SIRS) together with an infection.

Sepsis (commonly called a "blood stream infection") denotes the presence of bacteria (bacteremia) or other infectious organisms or their toxins in the blood (septicemia) or in other tissue of the body and the immune response of the host. Organ failure due to sepsis is currently thought to start with the interaction between the host response and the presence of micro-organisms and/or their toxins within the body. The observed host responses include immune, coagulation, pro and anti-inflammatory responses. Septic organ failure thus comprises a systemic response to infection, defined as hypothermia or hyperthermia, tachycardia, tachypnea, a clinically evident focus of infection or positive blood cultures, one or more end organs with either dysfunction or inadequate perfusion, cerebral dysfunction, hypoxaemia, increased plasma lactate or unexplained metabolic acidosis, and oliguria.

While usually related to infection, it can also be associated with noninfectious insults such as trauma, burns, and pancreatitis. It is one of the most common causes of adult respiratory distress syndrome.

A precise definition of the term sepsis has been introduced by the ACCP/SCCM Consensus Conference Committee (1992): Definition for sepsis and guidelines for the use of innovative therapies in sepsis. Crit Care Med. 20(6):864-874. The 2001 International Organ failure Definitions Conference attempted to improve the above definition with the aim of increasing the accuracy of the diagnosis of sepsis Levy M, Fink M, Mitchell P, Marshall JC, Abraham E, et al. for the International Sepsis Definitions Conference. 2001 SCCM/ESICM/ACCP/ATS/SIS. The statement suggested that although the SIRS concept was valid, in the future if supported by further epidemiologic data, it may be possible to use purely biochemical and/or immunologic, rather than clinical criteria to identify the inflammatory response. It also defined infection as a pathologic process induced by a micro-organism, and that organ failure should be defined as a patient with documented or suspected 'infection' exhibiting some of the following variables:
1. General variables
   ○ Fever (core temperature >38.3°C)
   ○ Hypothermia (core temperature <36°C)
   ○ Heart rate >90 min⁻¹ or >2 SD above the normal value for age
   ○ Tachypnea
   ○ Altered mental status
   ○ Significant oedema or positive fluid balance (>20 mL/kg over 24 hrs)
   ○ Hyperglycemia (plasma glucose >7.7 mmol/L) in the absence of diabetes
2. Inflammatory variables
   ○ Leukocytosis - WBC count >12,000 µL⁻¹
   ○ Leukopaenia - WBC count <4000 µL⁻¹
   ○ Normal WBC count with >10% immature forms
   ○ Plasma C-reactive protein >2 SD above the normal value
   ○ Plasma procalcitonin >2 SD above the normal value
3. Hemodynamic variables
   ○ Arterial hypotension (SBP <90 mmHg, MAP <70 mmHg, or an SBP decrease >40 mmHg in adults)
   ○ SvO2a >70%
   ○ Cardiac index > 3.5 Lmin⁻¹M⁻²
4. Organ dysfunction variables
   ○ Arterial hypoxemia (PaO2/FIO2 <300)
   ○ Acute oliguria (urine output <0.5 mLkg⁻¹hr⁻¹ for at least 2hrs)
   ○ Creatinine increase >0.5 mg/dL
   ○ Coagulation abnormalities (INR >1.5 or aPTT >60 secs)
   ○ Ileus (absent bowel sounds)
   ○ Thrombocytopenia (platelet count <100µL)
   ○ Hyperbilirubinemia (plasma total bilirubin>4 mg/dL or 70 mmol/L)
5. Tissue perfusion variables
   ○ Hyperlactatemia (>1 mmol/L)
   ○ Decreased capillary refill or mottling
      (WBC, white blood cell; SBP, systolic blood pressure; MAP, mean arterial blood pressure; SvO2, mixed venous oxygen saturation; INR, international normalized ratio; aPTT, activated partial thromboplastin time; tachycardia (may be absent in hypothermic patients), and at least one of the following indications of altered organ function: altered mental status, hypoxemia, increased serum lactate level.

The definition of severe sepsis remained unchanged and refers to sepsis complicated by organ dysfunction. Organ dysfunction is defined using Multiple Organ Dysfunction score Marshall JC, Cook DJ, Christou NV, et al. Multiple organ dysfunction score: A reliable descriptor of a complex clinical outcome. Crit Care Med 1995; 23: 1638-1652 or the definitions used for the Sequential Organ Failure Assessment (SOFA) score Ferreira FL, Bota DP, Bross A, et al. Serial evaluation of the SOFA score to predict outcome in critically ill patients. JAMA 2002; 286: 1754-1758. Septic shock in adults refers to a state of acute circulatory failure characterized by persistent arterial hypotension unexplained by other causes. Hypotension is defined by a systolic arterial pressure below 90 mm Hg, a MAP <70 mmHg, or a reduction in systolic blood pressure of >40 mm Hg from baseline, despite adequate volume resuscitation, in the absence of other causes for hypotension.

The mortality rate associated with organ failure, severe sepsis and septic shock are high and reported as 25 to 30% and 40 to 70% respectively. Bernard GR, Vincent JL, Laterre PF, et al. Efficacy and safety of recombinant human activated protein C for severe sepsis. N Engl J Med 2001; 344: 699-709. Annane D, Aegerter P, Jars-Guincestre MC, Guidet B. Current epidemiology of septic shock: the CUB-Rea Network. Am J Respir Crit Care Med 2003; 168: 165-72.

Despite some advances in the management of severe sepsis and septic shock, problems remain regarding the usefulness of the currently used definitions and the often encountered delays in diagnosis. The reliable diagnosis of organ failure still remains a challenge.

The identification, let alone the quantification of pathogens or of nucleic acids from these pathogens in an ill subject is far from being reliable, validated or sufficient for diagnosis, a large body of scientific evidence supports diagnostics based on the molecular response and immune response of the host, actually reflecting the individual clinical state of the subject, regardless of the nature or quantities of the underlying pathogens, respectively fragments of these organisms.

In classical patient screening and diagnosis, the medical practitioner uses a number of diagnostic tools for diagnosing a patient suffering from a certain disease. Among these tools, measurement of a series of single routine parameters, e.g. in a blood sample, is a common diagnostic laboratory approach. These single parameters comprise for example enzyme activities and enzyme concentration and/or detection.

As far as such diseases are concerned which easily and unambiguously can be correlated with one single parameter or a few number of parameters achieved by clinical chemistry, these parameters have proved to be indispensable tools in modern laboratory medicine and diagnosis. However, in pathophysiological conditions, such as cancer or demyelinating diseases such as multiple sclerosis which share a lack of an unambiguously assignable single parameter or marker, differential diagnosis from blood or tissue samples is currently difficult to impossible.

Although RNA-based diagnosis of organ failure from blood cells has been explored recently, these approaches, however, suffer from several serious limitations:
The required sample size of usually several ml of blood is a problem for continuous monitoring of a critically ill subject; alternatives applying amplification of transcripts are lengthy and prone to error. The whole procedure affords numerous steps and due to laborious sample preparation and RNA isolation, transcription and array or PCR analysis still takes at least several hours and a large technological effort.

Currently used diagnostic methods thus require time and appropriate equipment with high costs and frequently unsatisfying sensitivities. However this used diagnostic means have major limitations either to reduced area under the curve (AUC) and/or delay of diagnosis or increased costs due to equipment required. Accordingly these procedures do not allow a timely assessment of an acute and rapidly evolving disease and overall the situation is far from satisfying and from providing a rapid and reliable diagnosis of severe sepsis and organ failure.

Therefore, there is still an urgent need for an early, rapid and reliable diagnosis of organ failure or any other state of health providing the unspecific clinical symptoms, ideally requiring only minute amounts of blood; there is an urgent need for timely treatment and early diagnosis of organ failure as well as, an urgent need for therapy monitoring. Further, there is an urgent need for early organ failure biomarkers enabling early and reliable diagnosis.

These needs are met by a method for in vitro predicting the likelihood of an onset of organ failure and disorders related thereto in accordance with claim 1, a use of a list of specified metabolites according to claim 12 and Kit in accordance with claim 15. In particular, the present invention provides a solution to these problems based on the application of a new technology in this context and on an unknown list of endogenous metabolites as diagnostic marker. Since metabolite concentration differences in biological fluids and tissues provide links to the various phenotypical responses, metabolites are suitable biomarker candidates.

The present invention allows for accurate, rapid, and sensitive prediction and diagnosis of OF through a measurement of one or plurality of endogenous metabolic biomarker (metabolites) taken from a biological sample at a single point in time. This is accomplished by obtaining a biomarker panel at a single point in time from an individual, particularly an individual at risk of developing OF, having OF, or suspected of having OF, and comparing the biomarker profile from the individual to reference biomarker values or scores. The reference biomarker values may be obtained from a population of individuals (a "reference population") who are, for example, afflicted with OF or who are suffering from either the onset of OF or a particular stage in the progression of OF. If the biomarker panel values or score from the individual contains appropriately characteristic features of the biomarker values or scores from the reference population, then the individual is diagnosed as having a more likely chance of getting OF, as being afflicted with OF or as being at the particular stage in the progression of OF as the reference population.

Accordingly, the present invention provides, inter alia, methods of predicting the likelihood of an onset of OF in an individual. The methods comprise obtaining a biomarker score at a single point in time from the individual and comparing the individual's biomarker profile to a reference biomarker profile. Comparison of the biomarker profiles can predict the onset of OF in the individual preferably with an accuracy of at least about . This method may be repeated again at any time prior to the onset of OF.

The present invention further provides a method of determining the progression (i.e., the stage) of sepsis in an individual towards OF. This method comprises obtaining a biomarker profile at a single point in time from the individual and comparing the individual's biomarker profile to a reference biomarker score. Comparison of the biomarker scores can determine the progression of sepsis in the individual preferably with an accuracy of at least about 90 %. This method may also be repeated on the individual at any time.

Additionally, the present invention provides a method of diagnosing OF in an individual having or suspected of having OF. This method comprises obtaining a biomarker score at a single point in time from the individual and comparing the individual's biomarker score to a reference biomarker score. Comparison of the biomarker profiles can diagnose OF in the individual with an accuracy of at least about 90 %. This method may also be repeated on the individual at any time.

In another embodiment, the invention provides, inter alia, a method of determining the status of OF or diagnosing OF in an individual comprising applying a decision rule. The decision rule comprises comparing (i) a biomarker score generated from a biological sample taken from the individual at a single point in time with (ii) a biomarker score generated from a reference population. Application of the decision rule determines the status of sepsis or diagnoses OF in the individual. The method may be repeated on the individual at one or more separate, single points in time.

The present invention further provides, inter alia, a method of determining the status of OF or diagnosing OF in an individual comprising obtaining a biomarker score from a biological sample taken from the individual and comparing the individual's biomarker score to a reference biomarker score. A single such comparison is capable of classifying the individual as having membership in the reference population. Comparison of the biomarker scores determines the status of OF or diagnoses OF in the individual.

In yet another embodiment, the present invention provides, inter alia, a method of determining the status of OF or diagnosing OF in an individual. The method comprises comparing a measurable characteristic of at least one biomarker between a biomarker panel or biomarker score composed by (processed or unprocessed) values of this panel obtained from a biological sample from the individual and a biomarker score obtained from biological samples from a reference population. Based on this comparison, the individual is classified as belonging to or not belonging to the reference population. The comparison, therefore, determines the likelihood of OF or diagnoses OF in the individual. The biomarkers, in one embodiment, are selected from the group of biomarkers shown in any one of TABLES 1 to 3.

The present invention provides methods for predicting organ failure . Such methods comprise the steps of: analyzing a biological sample from a subject to determine the level(s) of one or more biomarkers for organ failure in the sample, where the one or more biomarkers are selected from Table 1 and comparing the level(s) of the one or more biomarkers - respectively a composed value / score generated by subjecting the concentrations of individual biomarkers in the sample to a classification method such as affording an equation processing single concentration values - to obtain a separation between both (diseased and healthy) groups or comparing the level(s) of the one or more biomarkers in the sample to organ failure positive or organ failure negative reference levels of the one or more biomarkers in order to determine whether the subject is developing organ failure.

The present invention provides a solution to the problem described above, and generally relates to the use of metabolomics data, generated by quantitation of endogenous metabolites by but not limited to mass spectrometry (MS), in particular MS-technologies such as MALDI, ESI, atmospheric pressure pressure chemical ionization (APCI), and other methods, determination of metabolite concentrations by use of MS-technologies or alternative methods coupled to separation (LC-MS, GC-MS, CE-MS), subsequent feature selection and /or the combination of features to classifiers including molecular data of at least two molecules.

The concentrations of the individual markers, analytes, metabolites thus are measured and compared to reference values or data combined and processed to scores, classifiers and compared to reference values thus indicating diseased states etc. with superior sensitivities and specificities compared to known procedures, clinical parameters and biomarkers.

Those skilled in the art will understand that for the quantitation of certain metabolites, also chemically modified metabolites may be used. For example, it is a well established practice to use the phenylisothiocyanates of amino acids for a more sensitive (sensitivity enhancement up to 100 fold) and preciser quantification, as one gets a better separation on the column material used prior to the MS-technologies.

Furthermore, in some embodiments, the present invention provides a method of diagnosing organ failure and/or duration/severity comprising: detecting the presence or absence of one or more (e.g., 2 or more, 3 or more, 5 or more, 10 or more, etc. measured together in a multiplex or panel format) organ failure specific metabolites in a sample (e.g., a tissue (e.g., biopsy) sample, a blood sample, a serum sample, or a urine sample) from a subject; and diagnosing organ failure based on the presence of the organ failure specific metabolite.

The present invention further provides a method of screening compounds, comprising: contacting an animal, a tissue, a cell containing a organ failure-specific metabolite with a test compound; and detecting the level of the organ failure specific metabolite. In some embodiments, the method further comprises the step of comparing the level of the organ failure specific metabolite in the presence of the test compound or therapeutic intervention to the level of the organ failure specific metabolite in the absence of the organ failure specific metabolite. In some embodiments, the cell is in vitro, in a non-human mammal, or ex vivo. In some embodiments, the test compound is a small molecule or a nucleic acid (e.g., antisense nucleic acid, a siRNA, or a miRNA) or oxygen/xenon or any neuroprotective drug that inhibits the expression of an enzyme involved in the synthesis or breakdown of an organ failure specific metabolite. In some embodiments, the organ failure specific metabolite groups given in Tables 2 and 3. In some embodiments, the method is a high throughput method.

In particular, the present invention relates to:
A method for predicting the likelihood of onset of an inflammation associated organ failure from a biological sample of a mammalian subject in vitro, wherein
   a. the subject's quantitative metabolomics profile comprising one or a plurality of endogenous metabolites, is detected in the biological sample by means of quantitative metabolomics analysis, and
   b. the quantitative metabolomics profile of the subject's sample is compared with a quantitative reference metabolomics profile of one or a plurality of endogenous organ failure predictive target metabolites in order to predict whether the subject is likely or unlikely to develop an organ failure; and
   c. wherein said endogenous organ failure predictive target metabolites have a molecular mass less than 1500 Da and are selected from the group consisting of: Amino acids, in particular, arginine, aspartic acid, citrulline, glutamic acid (glutamate), glutamine, leucine, isoleucine, histidine, ornithine, proline, phenylalanine, serine, tryptophane, tyrosine, valine, kynurenine;
      phenylthio carbamyl amino acids (PTC-amino acids), in particular, PCT-arginine, PTC-glutamine, PTC-histidine, PTC-methionine, PTC-ornithine, PTC-phenylalanine, PTC-proline, PTC-serine, PTC-tryptophane, PTC-tyrosine, PTC-valine;
      dimethylarginine, in particular N,N-dimethyl-L-arginine;
      carboxylic acids, namely 15(S)-hydroxy-5Z,8Z,11Z,13E-eicosatetraenoic acid [(5Z,8Z,11 Z,13E,15S)-15-Hydroxyicosa-5,8,11,13-tetraenoic acid], succinic acid (succinate);
      Ceramides, with an N-acyl residue having from 2 to 30 Carbon atoms in the acyl residue and having from 0 to 5 double bonds and having from 0 to 5 hydroxy groups;
      carnitine; acylcarnitines having from 1 to 20 carbon atoms in the acyl residue; acylcarnitines having from 3 to 20 carbon atoms in the acyl residue and having 1 to 4 double bonds in the acyl residue; acylcarnitines having from 1 to 20 carbon atoms in the acyl residue and having from 1 to 3 OH-groups in the acyl residue; acylcarnitines having from 3 to 20 carbon atoms in the acyl residue with 1 to 4 double bonds and 1 to 3 OH-groups in the acyl residue;
      phospholipides, in particular lysophosphatidylcholines (monoacylphospha-tidylcholines) having from 1 to 30 carbon atoms in the acyl residue; lysophosphatidylcholines having from 3 to 30 carbon atoms in the acyl residue and having 1 to 6 double bonds in the acyl residue;
      phosphatidylcholines (diacylphosphatidylcholines) having a total of from 1 to 50 carbon atoms in the acyl residues; phosphatidylcholines having a total from 3 to 50 carbon atoms in the acyl residues and having a total of 1 to 8 double bonds in the acyl residues;
      sphingolipids, in particular sphingomyelines having a total number of carbon atoms in the acyl chains from 10 to 30; sphingomyelines having a total number of carbon atoms in the acyl chains from 10 to 30 and 1 to 5 double bonds; hydroxysphinogomyelines having a total number of carbon atoms in the acyl residues from 10 to 30;
      hydroxysphingoyelines having a total number of carbon atoms in the acyl residues from 10 to 30 and 1 to 5 double bonds;
      prostaglandines, namely 6-keto-prostaglandin F1alpha, prostaglandin D2, thromboxane B2;
      putrescine;
      oxysterols, namely 22-R-hydroxycholesterol, 24-S-hydroxycholesterol, 25-hydroxycholesterol, 27- hydroxycholesterol, 20α- hydroxycholesterol, 22-S-hydroxycholesterol, 24,25- epoxycholesterol,3β,5α,6β- trihydroxycholesterol, 7α-hydroxycholesterol, 7-Ketocholesterol, 5β,6β- epoxycholesterol, 5α,6α-epoxycholesterol, 4β- hydroxycholesterol, desmosterol (vitamin D3), 7-dehydrocholesterol, cholestenone, lanosterol, 24-dehydrolanosterol;
      bile acids, namely cholic acid, chenodeoxycholic acid, deoxycholic acid, glycocholic acid, glycochenodeoxycholic acid, glycodeoxycholic acid, glycolithocholic acid, glycolithocholic acid sulfate, glycoursodeoxycholic acid, lithocholic acid, taurocholic acid, taurochenodeoxycholic acid taurodeoxycholic acid, taurolithocholic acid, taurolithocholic acid sulfate, tauroursodeoxycholic acid, ursodeoxycholic acid;
      biogenic amines, namely histamine, serotonine, palmitoyl ethanolamine.

According to the present invention, the term "inflammation associated organ failure" comprises "infection associated organ failure" and/or "sepsis associated organ failure".

A preferred method is one, wherein the biological sample is selected from the group consisting of stool; body fluids, in particular blood, liquor, cerebrospinal fluid, urine, ascitic fluid, seminal fluid, saliva, puncture fluid, cell content, tissue samples, in particular liver biopsy material; or a mixture thereof.

Advantageously, a preferred embodiment of the method according to the present invention is one, wherein said quantitative metabolomics profile is achieved by a quantitative metabolomics profile analysis method comprising the generation of intensity data for the quantitation of endogenous metabolites by mass spectrometry (MS), in particular, by high- throughput mass spectrometry, preferably by MS-technologies such as Matrix Assisted Laser Desorption/lonisation (MALDI), Electro Spray Ionization (ESI), Atmospheric Pressure Chemical Ionization (APCI), ¹H-, ¹³C- and/or ³¹P- Nuclear Magnetic Resonance spectroscopy (NMR), optionally coupled to MS, determination of metabolite concentrations by use of MS-technologies and/or methods coupled to separation, in particular Liquid Chromatography (LC-MS), Gas Chromatography (GC-MS), or Capillary Electrophoresis (CE-MS).

Furthermore, preferably, intensity data of said metabolomics profile are normalized with a set of endogenous housekeeper metabolites by relating detected intensities of the selected endogenous organ failure predictive target metabolites to intensities of said endogenous housekeeper metabolites.

A particularly preferred method according to the present invention is one, wherein said endogenous housekeeper metabolites are selected from the group consisting of such endogeneous metabolites which show stability in accordance with statistical stability measures being selected from the group consisting of coefficient of variation (CV) of raw intensity data, standard deviation (SD) of logarithmic intensity data, stability measure (M) of geNorm - algorithm or stability measure value (rho) of NormFinder-algorithm.

Additionally, said quantitative metabolomics profile comprises the results of measuring at least one of the parameters selected from the group consisting of: concentration, level or amount of each individual endogenous metabolite of said plurality of endogenous metabolites in said sample, qualitative and/or quantitative molecular pattern and/or molecular signature; and using and storing the obtained set of values in a database.

A panel of reference endogenous organ failure predictive target metabolites or derivatives thereof is established by:
a) mathematically preprocessing intensity values obtained for generating the metabolomics profiles in order to reduce technical errors being inherent to the measuring procedures used to generate the metabolomics profiles;
b) selecting at least one suitable classifying algorithm from the group consisting of logistic regression, (diagonal) linear or quadratic discriminant analysis (LDA, QDA, DLDA, DQDA), perceptron, shrunken centroids regularized discriminant analysis (RDA), random forests (RF), neural networks (NN), Bayesian networks, hidden Markov models, support vector machines (SVM), generalized partial least squares (GPLS), partitioning around medoids (PAM), inductive logic programming (ILP), generalized additive models, gaussian processes, regularized least square regression, self organizing maps (SOM), recursive partitioning and regression trees, K-nearest neighbour classifiers (K-NN), fuzzy classifiers, bagging, boosting, and naïve Bayes; and applying said selected classifier algorithm to said preprocessed data of step a);
c) said classifier algorithms of step b) being trained on at least one training data set containing preprocessed data from subjects being divided into classes according to their likelihood to develop an organ failure, in order to select a classifier function to map said preprocessed data to said likelihood;
d) applying said trained classifier algorithms of step c) to a preprocessed data set of a subject with unknown organ failure likelihood, and using the trained classifier algorithms to predict the class label of said data set in order to predict the likelihood for a subject to develop an organ failure.

The endogenous organ failure predictive target metabolites for easier and/or more sensitive detection are preferably detected by means of chemically modified derivatives thereof, such as phenylisothiocyanates for amino acids.
In a preferred embodiment of the present invention, said endogenous organ failure predictive target metabolites are selected from the group consisting of:
Carnitin, acylcarnitines (C chain length:total number of double bonds), in particular, C12-DC, C14:1, C14:1-OH, C14:2, C14:2-OH, C18, C6:1;
sphingomyelins (SM chain length:total number of double bonds), in particular, SM C16:0, SM C17:0, SM C18:0, SM C19:0, SM C21:1, SM C21:3, SM C22:2, SM C23:0, SM C23:1, SM C23:2, SM C23:3, SM C24:0, SM C24:1, SM C24:2, SM C24:3, SM C24:4, SM C26:4, SM C3:0, SM (OH) C22:1, SM (OH) C22:2, SM (OH) C24:1, SM C26:0, SM C26:1;
phosphatidylcholines, (diacylphosphatidylcholines, PC aa chain length:total number of double bonds or PC ae) in particular, PC aa C28:1, PC aa C38:0, PC aa C42:0, PC aa C42:1, PC ae C40:1, PC ae C40:2, PC ae C40:6, PC ae C42:2, PC ae C42:3, PC ae C42:4, PC ae C44:5, PC ae C44:6, PC aa C36:4, PC aa C38:1, PC aa C38:2, PC aa C38:4, PC aa C38:5, PC aa C38:6, PC aa C40:5, PC aa C40:6, PC aa C40:7, PC aa C40:8, PC ae C36:4, PC ae C36:5, PC ae C38:4, PC ae C38:6;
lysophosphatidylcholines (monoacylphosphatidylcholines, PC a chain length:total number of double bonds), in particular, PC a C18:2, PC a C20:4, PC a C20:3, PC a C26:0;
Phe;
oxycholesterols, in particular, 3β,5α,6β-trihydroxycholestan, 7-ketocholesterol, 5α,6α-epoxycholesterol;
lysophosphatidylethanolamins (monoacylphosphatidylcholins, PE a chain length:total number of double bonds), in particular, PE a C18:1, PE a C18:2, PE a C20:4, PE a C22:5, PE a C22:6;
phosphatidylethanolamins, (diacylphosphatidylcholins, PE aa chain length:total number of double bonds),in particular, PE aa C38:0, PE aa C38:2;
ceramids, (N-chain length:total number of double bonds),in particular, N-C2:0-Cer, N-C7:0-Cer, N-C9:3-Cer, N-C17:1-Cer, N-C22:1-Cer, N-C25:0-Cer, N-C27:1-Cer, N-C5:1-Cer(2H), N-C7:1-Cer(2H), N-C8:1-Cer(2H), N-C11:1-Cer(2H), N-C20:0-Cer(2H), N-C21:0-Cer(2H), N-C22:1-Cer(2H), N-C25:1-Cer(2H), N-C26:1-Cer(2H), N-C24:0(OH)-Cer, N-C26:0(OH)-Cer, N-C6:0(OH)-Cer, N-C8:0(OH)-Cer(2H), N-C10:0(OH)-Cer(2H), N-C25:0(OH)-Cer(2H), N-C26:0(OH)-Cer(2H), N-C27:0(OH)-Cer(2H), N-C28:0(OH)-Cer(2H).

For generating a metabolomics analysis profile, said plurality of endogenous organ failure predictive target metabolites or derivatives thereof comprises 2 to 80, in particular 2 to 60, preferably 2 to 50, preferred 2 to 30, more preferred 2 to 20, particularly preferred 2 to 10 endogenous metabolites

A particular embodiment of the present invention is the use of one or a plurality of endogenous metabolites for predicting of an onset of an infection associated organ failure from a biological sample of a mammalian subject in vitro, wherein the metabolites are selected from the group consisting of : Amino acids, in particular, arginine, aspartic acid, citrulline, glutamic acid (glutamate), glutamine, leucine, isoleucine, histidine, ornithine, proline, phenylalanine, serine, tryptophane, tyrosine, valine, kynurenine;
phenylthio carbamyl amino acids (PTC-amino acids), in particular, PCT-arginine, PTC-glutamine, PTC-histidine, PTC-methionine, PTC-ornithine, PTC-phenylalanine, PTC-proline, PTC-serine, PTC-tryptophane, PTC-tyrosine, PTC-valine;
dimethylarginine, in particular N,N-dimethyl-L-arginine;
carboxylic acids, namely 15(S)-hydroxy-5Z,8Z,11Z,13E-eicosatetraenoic acid [(5Z,8Z,11 Z,13E,15S)-15-Hydroxyicosa-5,8,11,13-tetraenoic acid], succinic acid (succinate);
Ceramides, with an N-acyl residue having from 2 to 30 Carbon atoms in the acyl residue and having from 0 to 5 double bonds and having from 0 to 5 hydroxy groups;
carnitine; acylcarnitines having from 1 to 20 carbon atoms in the acyl residue; acylcarnitines having from 3 to 20 carbon atoms in the acyl residue and having 1 to 4 double bonds in the acyl residue; acylcarnitines having from 1 to 20 carbon atoms in the acyl residue and having from 1 to 3 OH-groups in the acyl residue; acylcarnitines having from 3 to 20 carbon atoms in the acyl residue with 1 to 4 double bonds and 1 to 3 OH-groups in the acyl residue;
phospholipides, in particular lysophosphatidylcholines (monoacylphospha-tidylcholines) having from 1 to 30 carbon atoms in the acyl residue; lysophosphatidylcholines having from 3 to 30 carbon atoms in the acyl residue and having 1 to 6 double bonds in the acyl residue;
phosphatidylcholines (diacylphosphatidylcholines) having a total of from 1 to 50 carbon atoms in the acyl residues; phosphatidylcholines having a total from 3 to 50 carbon atoms in the acyl residues and having a total of 1 to 8 double bonds in the acyl residues;
sphingolipids, in particular sphingomyelines having a total number of carbon atoms in the acyl chains from 10 to 30; sphingomyelines having a total number of carbon atoms in the acyl chains from 10 to 30 and 1 to 5 double bonds; hydroxysphinogomyelines having a total number of carbon atoms in the acyl residues from 10 to 30; hydroxysphingoyelines having a total number of carbon atoms in the acyl residues from 10 to 30 and 1 to 5 double bonds;
prostaglandines, namely 6-keto-prostaglandin F1alpha, prostaglandin D2, thromboxane B2;
putrescine;
oxysterols, namely 22-R-hydroxycholesterol, 24-S-hydroxycholesterol, 25-hydroxycholesterol, 27- hydroxycholesterol, 20α- hydroxycholesterol, 22-S-hydroxycholesterol, 24,25- epoxychoiesterol,3β,5α,6β- trihydroxycholesterol, 7α-hydroxycholesterol, 7-Ketocholesterol, 5β,6β- epoxycholesterol, 5α,6α-epoxycholesterol, 4β- hydroxycholesterol, desmosterol (vitamin D3), 7-dehydrocholesterol, cholestenone, lanosterol, 24-dehydrolanosterol;
bile acids, namely cholic acid, chenodeoxycholic acid, deoxycholic acid, glycocholic acid, glycochenodeoxycholic acid, glycodeoxycholic acid, glycolithocholic acid, glycolithocholic acid sulfate, glycoursodeoxycholic acid, lithocholic acid, taurocholic acid, taurochenodeoxycholic acid taurodeoxycholic acid, taurolithocholic acid, taurolithocholic acid sulfate, tauroursodeoxycholic acid, ursodeoxycholic acid;
biogenic amines, namely histamine, serotonine, palmitoyl ethanolamine.

Particularly preferred endogenous organ failure predictive target metabolites are selected from the group consisting of:
Carnitin, acylcarnitines (C chain length:total number of double bonds), in particular, C12-DC, C14:1, C14:1-OH, C14:2, C14:2-OH, C18, C6:1;
sphingomyelins (SM chain length:total number of double bonds), in particular, SM C16:0, SM C17:0, SM C18:0, SM C19:0, SM C21:1, SM C21:3, SM C22:2, SM C23:0, SM C23:1, SM C23:2, SM C23:3, SM C24:0, SM C24:1, SM C24:2, SM C24:3, SM C24:4, SM C26:4, SM C3:0, SM (OH) C22:1, SM (OH) C22:2, SM (OH) C24:1, SM C26:0, SM C26:1;
phosphatidylcholines, (diacylphosphatidylcholines, PC aa chain length:total number of double bonds or PC ae) in particular, PC aa C28:1, PC aa C38:0, PC aa C42:0, PC aa C42:1, PC ae C40:1, PC ae C40:2, PC ae C40:6, PC ae C42:2, PC ae C42:3, PC ae C42:4, PC ae C44:5, PC ae C44:6, PC aa C36:4, PC aa C38:1, PC aa C38:2, PC aa C38:4, PC aa C38:5, PC aa C38:6, PC aa C40:5, PC aa C40:6, PC aa C40:7, PC aa C40:8, PC ae C36:4, PC ae C36:5, PC ae C38:4, PC ae C38:6;
lysophosphatidylcholines (monoacylphosphatidylcholines, PC a chain length:total number of double bonds), in particular, PC a C18:2, PC a C20:4, PC a C20:3, PC a C26:0;
Phe;
oxycholesterols, in particular, 3β,5α,6β-trihydroxycholestan, 7-ketocholesterol, 5α,6α-epoxycholesterol;
lysophosphatidylethanolamins (monoacylphosphatidylcholins, PE a chain length:total number of double bonds), in particular, PE a C18:1, PE a C18:2, PE a C20:4, PE a C22:5, PE a C22:6;
phosphatidylethanolamins, (diacylphosphatidylcholins, PE aa chain length:total number of double bonds),in particular, PE aa C38:0, PE aa C38:2;
ceramids, (N-chain length:total number of double bonds),in particular, N-C2:0-Cer, N-C7:0-Cer, N-C9:3-Cer, N-C17:1-Cer, N-C22:1-Cer, N-C25:0-Cer, N-C27:1-Cer, N-C5:1-Cer(2H), N-C7:1-Cer(2H), N-C8:1-Cer(2H), N-C11:1-Cer(2H), N-C20:0-Cer(2H), N-C21:0-Cer(2H), N-C22:1-Cer(2H), N-C25:1-Cer(2H), N-C26:1-Cer(2H), N-C24:0(OH)-Cer, N-C26:0(OH)-Cer, N-C6:0(OH)-Cer, N-C8:0(OH)-Cer(2H), N-C10:0(OH)-Cer(2H), N-C25:0(OH)-Cer(2H), N-C26:0(OH)-Cer(2H), N-C27:0(OH)-Cer(2H), N-C28:0(OH)-Cer(2H).

Furthermore, the present invention includes a kit for carrying out a method for predicting the likelihood of an onset of an infection associated organ failure from a biological sample of a mammalian subject in vitro, in a biological sample, comprising:
a) calibration agents for the quantitative detection of endogenous organ failure predictive target metabolites, wherein said metabolites are selected from the group consisting of: Amino acids, in particular, arginine, aspartic acid, citrulline, glutamic acid (glutamate), glutamine, leucine, isoleucine, histidine, ornithine, proline, phenylalanine, serine, tryptophane, tyrosine, valine, kynurenine;
   phenylthio carbamyl amino acids (PTC-amino acids), in particular, PCT-arginine, PTC-glutamine, PTC-histidine, PTC-methionine, PTC-ornithine, PTC-phenylalanine, PTC-proline, PTC-serine, PTC-tryptophane, PTC-tyrosine, PTC-valine;
   dimethylarginine, in particular N,N-dimethyl-L-arginine;
   carboxylic acids, namely 15(S)-hydroxy-5Z,8Z,11Z,13E-eicosatetraenoic acid [(5Z,8Z,11 Z,13E,15S)-15-Hydroxyicosa-5,8,11,13-tetraenoic acid], succinic acid (succinate);
   Ceramides, with an N-acyl residue having from 2 to 30 Carbon atoms in the acyl residue and having from 0 to 5 double bonds and having from 0 to 5 hydroxy groups;
   carnitine; acylcarnitines having from 1 to 20 carbon atoms in the acyl residue; acylcarnitines having from 3 to 20 carbon atoms in the acyl residue and having 1 to 4 double bonds in the acyl residue; acylcarnitines having from 1 to 20 carbon atoms in the acyl residue and having from 1 to 3 OH-groups in the acyl residue; acylcarnitines having from 3 to 20 carbon atoms in the acyl residue with 1 to 4 double bonds and 1 to 3 OH-groups in the acyl residue;
   phospholipides, in particular lysophosphatidylcholines (monoacylphospha-tidylcholines) having from 1 to 30 carbon atoms in the acyl residue; lysophosphatidylcholines having from 3 to 30 carbon atoms in the acyl residue and having 1 to 6 double bonds in the acyl residue;
   phosphatidylcholines (diacylphosphatidylcholines) having a total of from 1 to 50 carbon atoms in the acyl residues; phosphatidylcholines having a total from 3 to 50 carbon atoms in the acyl residues and having a total of 1 to 8 double bonds in the acyl residues;
   sphingolipids, in particular sphingomyelines having a total number of carbon atoms in the acyl chains from 10 to 30; sphingomyelines having a total number of carbon atoms in the acyl chains from 10 to 30 and 1 to 5 double bonds; hydroxysphinogomyelines having a total number of carbon atoms in the acyl residues from 10 to 30; hydroxysphingoyelines having a total number of carbon atoms in the acyl residues from 10 to 30 and 1 to 5 double bonds;
   prostaglandines, namely 6-keto-prostaglandin F1alpha, prostaglandin D2, thromboxane B2;
   putrescine;
   oxysterols, namely 22-R-hydroxycholesterol, 24-S-hydroxycholesterol, 25-hydroxycholesterol, 27- hydroxycholesterol, 20α- hydroxycholesterol, 22-S-hydroxycholesterol, 24,25- epoxycholesterol,3β,5α6β- trihydroxycholesterol, 7α-hydroxycholesterol, 7-Ketocholesterol, 5β,6β- epoxycholesterol, 5α,6α-epoxycholesterol, 4β- hydroxycholesterol, desmosterol (vitamin D3), 7-dehydrocholesterol, cholestenone, lanosterol, 24-dehydrolanosterol;
   bile acids, namely cholic acid, chenodeoxycholic acid, deoxycholic acid, glycocholic acid, glycochenodeoxycholic acid, glycodeoxycholic acid, glycolithocholic acid, glycolithocholic acid sulfate, glycoursodeoxycholic acid, lithocholic acid, taurocholic acid, taurochenodeoxycholic acid taurodeoxycholic acid, taurolithocholic acid, taurolithocholic acid sulfate, tauroursodeoxycholic acid, ursodeoxycholic acid;
   biogenic amines, namely histamine, serotonine, palmitoyl ethanolamine;
b) data base with processed data from healthy patients and patients who developed an infection associated organ failure;
c) classification software for generating the quantitative metabolomics profiles achieved with said calibration agents of step a) and classifying the results based on the processed data of step b).

Data classification is the categorization of data for its most effective and efficient use. Classifiers are typically deterministic functions that map a multi-dimensional vector of biological measurements to a binary (or n-ary) outcome variable that encodes the absence or existence of a clinically-relevant class, phenotype, distinct physiological state or distinct state of disease. To achieve this various classification methods such as, but not limited to, logistic regression, (diagonal) linear or quadratic discriminant analysis (LDA, QDA, DLDA, DQDA), perceptron, shrunken centroids regularized discriminant analysis (RDA), random forests (RF), neural networks (NN), Bayesian networks, hidden Markov models, support vector machines (SVM), generalized partial least squares (GPLS), partitioning around medoids (PAM), inductive logic programming (ILP), generalized additive models, gaussian processes, regularized least square regression, self organizing maps (SOM), recursive partitioning and regression trees, K-nearest neighbor classifiers (K-NN), fuzzy classifiers, bagging, boosting, and naive Bayes and many more can be used.

Further aspects, advantages and embodiments of the present invention will become evident by the description of examples, from the experimental sections below and by means of the drawings.

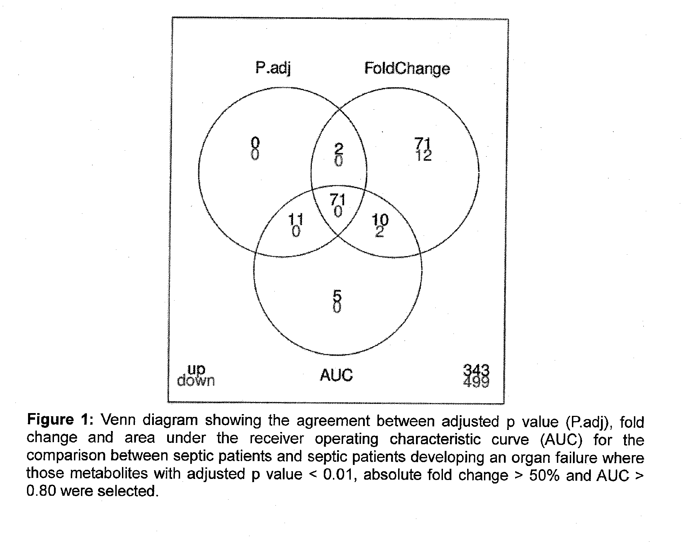

Fig.1 is a Venn diagram showing the agreement between adjusted p value (P.adj), fold change and area under the receiver operating characteristic curve (AUC) for the comparison between septic patients and septic patients developing an organ failure where those metabolites with adjusted p value < 0.01, absolute fold change > 50% and AUC > 0.80 were selected;

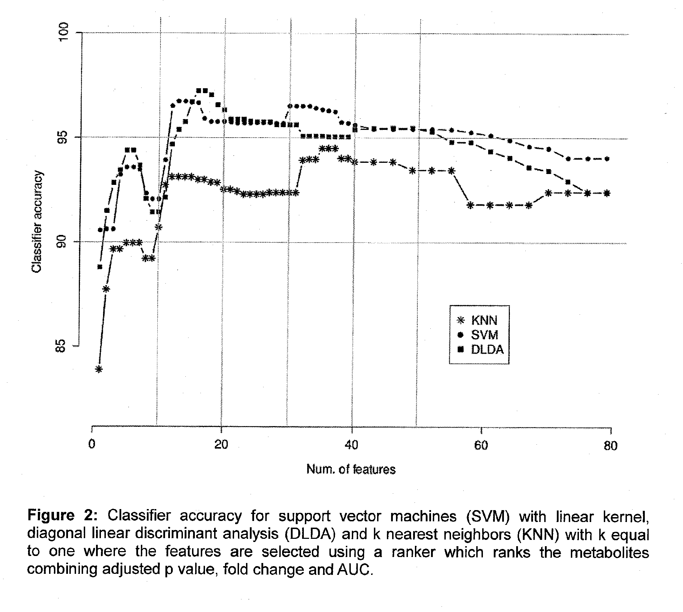

Fig. 2 is a graph showing classifier accuracy for support vector machines (SVM) with linear kernel, diagonal linear discriminant analysis (DLDA) and k nearest neighbors (KNN) with k equal to one where the features are selected using a ranker which ranks the metabolites combining adjusted p value, fold change and AUC;

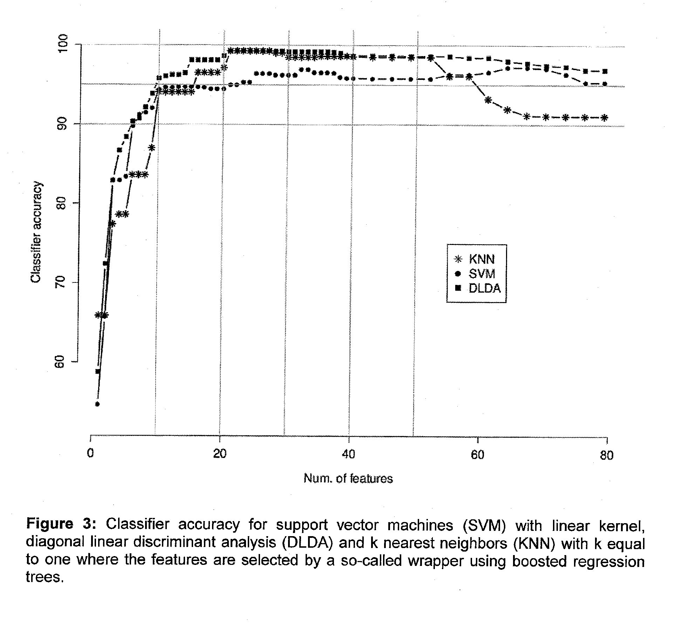

Fig. 3 is a graph showing classifier accuracy for support vector machines (SVM) with linear kernel, diagonal linear discriminant analysis (DLDA) and k nearest neighbors (KNN) with k equal to one where the features are selected by a so-called wrapper using boosted regression trees;

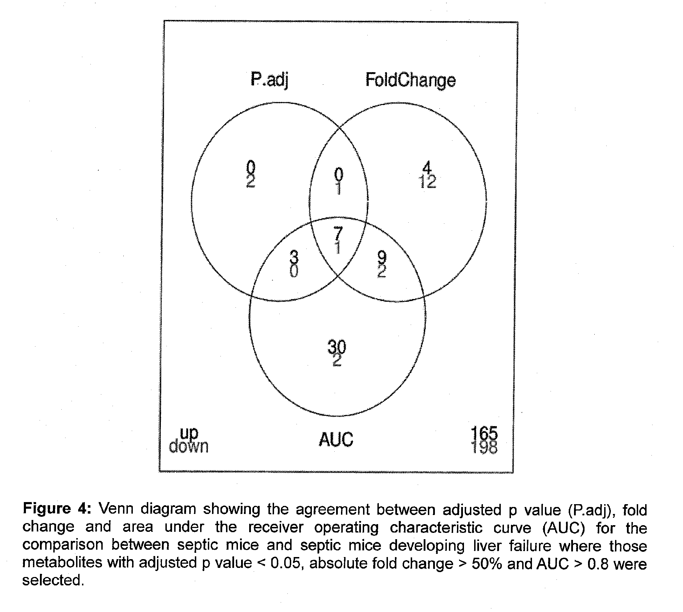

Fig. 4 is a Venn diagram showing the agreement between adjusted p value (P.adj), fold change and area under the receiver operating characteristic curve (AUC) for the comparison between septic mice and septic mice developing liver failure where those metabolites with adjusted p value < 0.05, absolute fold change > 50% and AUC > 0.8 were selected;

"Organ failure" (OF) in this context relates to any diseased state, however, particularly addresses an infection associated organ failure.

"Severe sepsis" refers to sepsis associated with organ dysfunction, hypoperfusion abnormalities, or sepsis-induced hypotension. Hypoperfusion abnormalities include, but are not limited to, lactic acidosis, oliguria, or an acute alteration in mental status. "Septic shock" refers to sepsis-induced hypotension that is not responsive to adequate intravenous fluid challenge and with manifestations of peripheral hypoperfusion. A "converter patient" refers to a SIRS-positive patient who progresses to clinical suspicion of sepsis during the period the patient is monitored, typically during an ICU stay. A "non-converter patient" refers to a SIRS-positive patient who does not progress to clinical suspicion of sepsis during the period the patient is monitored, typically during an ICU stay.

A patient with OF has a clinical presentation that is classified as OF, as defined above, but is not clinically deemed to have OF. Individuals who are at risk of developing OF include patients in an ICU and those who have otherwise suffered from a physiological trauma, such as a burn or other insult.

As used herein, "organ failure" (OF) includes all stages of OF including, but not limited to, the onset of OF and multi organ failure (MOD), e.g. associated with the end stages of sepsis.

"Sepsis" refers to a SIRS-positive condition that is associated with a confirmed infectious process. Clinical suspicion of sepsis arises from the suspicion that the SIRS-positive condition of a SIRS patient is a result of an infectious process.

The "onset of OF" refers to an early stage of OF, i.e., prior to a stage when the clinical manifestations are sufficient to support a clinical suspicion of OF. Because the methods of the present invention are used to detect OF prior to a time that OF would be suspected using conventional techniques, the patient's disease status at early OF can only be confirmed retrospectively, when the manifestation of OF is more clinically obvious. The exact mechanism by which a patient acquires OF is not a critical aspect of the invention. The methods of the present invention can detect changes in the biomarker score independent of the origin of the OF. Regardless of how OF arises, the methods of the present invention allow for determining the status of a patient having, or suspected of having, OF, as classified by previously used criteria.

As used herein, the term "organ failure specific metabolite" refers to a metabolite that is differentially present or differentially concentrated in septic organisms compared to non-septic organisms. For example, in some embodiments, organ failure specific metabolites are present in septic tissues but not in non- in septic tissues.

In other embodiments, organ failure-specific metabolites are absent in septic tissues but present in non-septic cells, tissues, body liquids. In still further embodiments, organ failure specific metabolites are present at different levels (e.g., higher or lower) in septic tissue/cells as compared to non-septic cells. For example, an organ failure specific metabolite may be differentially present at any level, but is generally present at a level that is increased by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more; or is generally present at a level that is decreased by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, or by 100% (i.e., absent).

An organ failure-specific metabolite is preferably differentially present at a level that is statistically significant (e.g., an adjusted p-value less than 0.05 as determined using either Analysis of Variance, Welch's t-test or its non parametric equivalent versions). Exemplary organ failure-specific metabolites are described in the detailed description and experimental sections below.

The term "sample" in the present specification and claims is used in its broadest sense. On the one hand it is meant to include a specimen or culture. On the other hand, it is meant to include both biological and environmental samples. A sample may include a specimen of synthetic origin.

Biological samples may be animal, including human, fluid, solid (e.g., stool) or tissue, such biological samples may be obtained from all of the various families of domestic animals, as well as feral or wild animals, including, but not limited to, such animals as ungulates, bear, fish, rodents, etc. A biological sample may contain any biological material suitable for detecting the desired biomarkers, and may comprise cellular and/or non-cellular material from a subject. The sample can be isolated from any suitable biological tissue or fluid such as, for example, tissue, blood, blood plasma, urine, or cerebral spinal fluid (CSF).

A "reference level" of a metabolite means a level of the metabolite that is indicative of a particular disease state, phenotype, or lack thereof, as well as combinations of disease states, phenotypes, or lack thereof. A "positive" reference level of a metabolite means a level that is indicative of a particular disease state or phenotype. A "negative" reference level of a metabolite means a level that is indicative of a lack of a particular disease state or phenotype. For example, a "organ failure-positive reference level" of a metabolite means a level of a metabolite that is indicative of a positive diagnosis of organ failure in a subject, and an "organ failure-negative reference level" of a metabolite means a level of a metabolite that is indicative of a negative diagnosis of organ failure in a subject. A "reference level" of a metabolite may be an absolute or relative amount or concentration of the metabolite, a presence or absence of the metabolite, a range of amount or concentration of the metabolite, a minimum and/or maximum amount or concentration of the metabolite, a mean amount or concentration of the metabolite, and/or a median amount or concentration of the metabolite; and, in addition, "reference levels" of combinations of metabolites may also be ratios of absolute or relative amounts or concentrations of two or more metabolites with respect to each other or a composed value / score obtained by classification.

Appropriate positive and negative reference levels of metabolites for a particular disease state, phenotype, or lack thereof may be determined by measuring levels of desired metabolites in one or more appropriate subjects, and such reference levels may be tailored to specific populations of subjects (e.g., a reference level may be age-matched so that comparisons may be made between metabolite levels in samples from subjects of a certain age and reference levels for a particular disease state, phenotype, or lack thereof in a certain age group). Such reference levels may also be tailored to specific techniques that are used to measure levels of metabolites in biological samples (e.g., LC-MS, GC-MS, etc.), where the levels of metabolites may differ based on the specific technique that is used.

As used herein, the term "cell" refers to any eukaryotic or prokaryotic cell (e.g., bacterial cells such as E. coli, yeast cells, mammalian cells, avian cells, amphibian cells, plant cells, fish cells, and insect cells), whether located in vitro or in vivo.

As used herein, the term "processor" refers to a device that performs a set of steps according to a program (e.g., a digital computer). Processors, for example, include Central Processing Units ("CPUs"), electronic devices, or systems for receiving, transmitting, storing and/or manipulating data under programmed control.

As used herein, the term "memory device," or "computer memory" refers to any data storage device that is readable by a computer, including, but not limited to, random access memory, hard disks, magnetic (floppy) disks, compact discs, DVDs, magnetic tape, flash memory, and the like.

"Mass Spectrometry" (MS) is a technique for measuring and analyzing molecules that involves fragmenting a target molecule, then analyzing the fragments, based on their mass/charge ratios, to produce a mass spectrum that serves as a "molecular fingerprint". Determining the mass/charge ratio of an object is done through means of determining the wavelengths at which electromagnetic energy is absorbed by that object. There are several commonly used methods to determine the mass to charge ration of an ion, some measuring the interaction of the ion trajectory with electromagnetic waves, others measuring the time an ion takes to travel a given distance, or a combination of both. The data from these fragment mass measurements can be searched against databases to obtain definitive identifications of target molecules. Mass spectrometry is also widely used in other areas of chemistry, like petrochemistry or pharmaceutical quality control, among many others.

As used here, the term "metabolite" denotes endogenous organic compounds of a cell, an organism, a tissue or being present in body liquids and in extracts obtained from the aforementioned sources with a molecular weight typically below 1500 Dalton. Typical examples of metabolites are carbohydrates, lipids, phospholipids, sphingolipids and sphingophospholipids, amino acids, cholesterol, steroid hormones and oxidized sterols and other compounds such as collected in the Human Metabolite database [Wishart DS et al., HMDB: the Human Metabolome Database. Nucleic Acids Res. 2007 Jan;35(Database issue):D521-6(see http://www.hmdb.ca/)*]* and other databases and literature. This includes any substance produced by metabolism or by a metabolic process and any substance involved in metabolism.

"Metabolomics" as understood within the scope of the present invention designates the comprehensive quantitative measurement of several (2-thousands) metabolites by, but not limited to, methods such as mass spectroscopy, coupling of liquid chromatography, gas chromatography and other separation methods chromatography with mass spectroscopy.

The term "separation" refers to separating a complex mixture into its component proteins or metabolites. Common laboratory separation techniques include gel electrophoresis and chromatography.

The term "capillary electrophoresis" refers to an automated analytical technique that separates molecules in a solution by applying voltage across buffer-filled capillaries. Capillary electrophoresis is generally used for separating ions, which move at different speeds when the voltage is applied, depending upon the size and charge of the ions. The solutes (ions) are seen as peaks as they pass through a detector and the area of each peak is proportional to the concentration of ions in the solute, which allows quantitative determinations of the ions.

The term "chromatography" refers to a physical method of separation in which the components to be separated are distributed between two phases, one of which is stationary (stationary phase) while the other (the mobile phase) moves in a definite direction. Chromatographic output data may be used for manipulation by the present invention.

An "ion" is a charged object formed by adding electrons to or removing electrons from an atom.

A "mass spectrum" is a plot of data produced by a mass spectrometer, typically containing m/z values on x-axis and intensity values on y-axis.

A "peak" is a point on a mass spectrum with a relatively high y-value.

The term "m/z" refers to the dimensionless quantity formed by dividing the mass number of an ion by its charge number. It has long been called the "mass-to-charge" ratio.

The term "metabolism" refers to the chemical changes that occur within the tissues of an organism, including "anabolism" and "catabolism". Anabolism refers to biosynthesis or the buildup of molecules and catabolism refers to the breakdown of molecules.

As used herein, the term "post-surgical tissue" refers to tissue that has been removed from a subject during a surgical procedure. Examples include, but are not limited to, biopsy samples, excised organs, and excised portions of organs.

As used herein, the terms "detect", "detecting", or "detection" may describe either the general act of discovering or discerning or the specific observation of a detectably labeled composition.

As used herein, the term "clinical failure" refers to a negative outcome following organ failure treatment.

A biomarker in this context is a characteristic, comprising data of at least one metabolite that is measured and evaluated as an indicator of biologic processes, pathogenic processes, or responses to a therapeutic intervention associated with organ failure or related to organ failure treatment. A combined biomarker as used here may be selected from at least two small endogenous molecules and metabolites.

### DETAILED DESCRIPTION OF THE INVENTION

The present invention relates to markers of Organ failure and its duration/severity as well of the effect of therapeutic interventions. In particular embodiments, the present invention provides metabolites that are differentially present in Organ failure. Experiments conducted during the course of development of embodiments of the present invention identified a series of metabolites as being differentially present in Tables 2 and 3 provide additional metabolites present in plasma serum or other body liquids. The disclosed markers find use as diagnostic and therapeutic targets.

### Diagnostic Applications

In some embodiments, the present invention provides methods and compositions for diagnosing organ failure, including but not limited to, characterizing risk of organ failure, stage of organ failure,duration and severity etc. based on the presence of organ failure specific metabolites or their derivatives, precursors, metabolites, etc. Exemplary diagnostic methods are described below.

Thus, for example, a method of diagnosing (or aiding in diagnosing) whether a subject has organ failure comprises (1) detecting the presence or absence or a differential level of one or more organ failure specific metabolites selected from table # and b) diagnosing organ failure based on the presence, absence or differential level of the organ failure specific metabolite. When such a method is used to aid in the diagnosis of organ failure, the results of the method may be used along with other methods (or the results thereof) useful in the clinical determination of whether a subject has organ failure.

Any mammalian sample suspected of containing organ failure specific metabolites is tested according to the methods described herein. By way of non-limiting examples, the sample may be tissue (e.g., a biopsy sample or post-surgical tissue), blood, urine, or a fraction thereof (e.g., plasma, serum, urine supernatant, urine cell pellet).

In some embodiments, the patient sample undergoes preliminary processing designed to isolate or enrich the sample for organ failure specific metabolites or cells that contain organ failure specific metabolites. A variety of techniques known to those of ordinary skill in the art may be used for this purpose, including but not limited: centrifugation; immunocapture; and cell lysis.

Metabolites may be detected using any suitable method including, but not limited to, liquid and gas phase chromatography, alone or coupled to mass spectrometry (See e.g., experimental section below), NMR, immunoassays, chemical assays, spectroscopy and the like. In some embodiments, commercial systems for chromatography and NMR analysis are utilized.

In other embodiments, metabolites (i.e. biomarkers and derivatives thereof) are detected using optical imaging techniques such as magnetic resonance spectroscopy (MRS), magnetic resonance imaging (MRI), CAT scans, ultra sound, MS-based tissue imaging or X-ray detection methods (e.g., energy dispersive x-ray fluorescence detection).

Any suitable method may be used to analyze the biological sample in order to determine the presence, absence or level(s) of the one or more metabolites in the sample. Suitable methods include chromatography (e.g., HPLC, gas chromatography, liquid chromatography), mass spectrometry (e.g., MS, MS-MS), enzyme-linked immunosorbent assay (ELISA), antibody linkage, other immunochemical techniques, biochemical or enzymatic reactions or assays, and combinations thereof. Further, the level(s) of the one or more metabolites may be measured indirectly, for example, by using an assay that measures the level of a compound (or compounds) that correlates with the level of the biomarker(s) that are desired to be measured.

The levels of one or more of the recited metabolites may be determined in the methods of the present invention. For example, the level(s) of one metabolites, two or more metabolites, three or more metabolites, four or more metabolites, five or more metabolites, six or more metabolites, seven or more metabolites, eight or more metabolites, nine or more metabolites, ten or more metabolites, etc., including a combination of some or all of the metabolites including, but not limited to those listed in table 2, may be determined and used in such methods.

Determining levels of combinations of the metabolites may allow greater sensitivity and specificity in the methods, such as diagnosing organ failure and aiding in the diagnosis of organ failure, and may allow better differentiation or characterization of organ failure from other disorders or other organ failure that may have similar or overlapping metabolites to organ failure (as compared to a subject not having organ failure). For example, ratios of the levels of certain metabolites in biological samples may allow greater sensitivity and specificity in diagnosing organ failure and aiding in the diagnosis of organ failure and allow better differentiation or characterization of organ failure from other organ failure or other disorders of the that may have similar or overlapping metabolites to organ failure (as compared to a subject not having organ failure).

### Data Analysis

In some embodiments, a computer-based analysis program is used to translate the raw data generated by the detection assay (e.g., the presence, absence, or amount of an organ failure specific metabolite) into data of predictive value for a clinician. The clinician can access the predictive data using any suitable means. Thus, in some embodiments, the present invention provides the further benefit that the clinician, who is not likely to be trained in metabolite analysis, need not understand the raw data. The data is presented directly to the clinician in its most useful form. The clinician is then able to immediately utilize the information in order to optimize the care of the subject.

The present invention contemplates any method capable of receiving, processing, and transmitting the information to and from laboratories conducting the assays, information provides, medical personal, and subjects. For example, in some embodiments of the present invention, a sample (e.g., a biopsy or a blood, urine or serum sample) is obtained from a subject and submitted to a profiling service (e.g., clinical lab at a medical facility, etc.), located in any part of the world (e.g., in a country different than the country where the subject resides or where the information is ultimately used) to generate raw data. Where the sample comprises a tissue or other biological sample, the subject may visit a medical center to have the sample obtained and sent to the profiling center, or subjects may collect the sample themselves (e.g., a plasma sample) and directly send it to a profiling center. Where the sample comprises previously determined biological information, the information may be directly sent to the profiling service by the subject (e.g., an information card containing the information may be scanned by a computer and the data transmitted to a computer of the profiling center using an electronic communication systems). Once received by the profiling service, the sample is processed and a profile is produced (i.e., metabolic profile), specific for the diagnostic or prognostic information desired for the subject.

The profile data is then prepared in a format suitable for interpretation by a treating clinician. For example, rather than providing raw data, the prepared format may represent a diagnosis or risk assessment (e.g., likelihood of organ failure being present) for the subject, along with recommendations for particular treatment options. The data may be displayed to the clinician by any suitable method. For example, in some embodiments, the profiling service generates a report that can be printed for the clinician (e.g., at the point of care) or displayed to the clinician on a computer monitor.

In some embodiments, the information is first analyzed at the point of care or at a regional facility. The raw data is then sent to a central processing facility for further analysis and/or to convert the raw data to information useful for a clinician or patient. The central processing facility provides the advantage of privacy (all data is stored in a central facility with uniform security protocols), speed, and uniformity of data analysis. The central processing facility can then control the fate of the data following treatment of the subject. For example, using an electronic communication system, the central facility can provide data to the clinician, the subject, or researchers.

In some embodiments, the subject is able to directly access the data using the electronic communication system. The subject may chose further intervention or counseling based on the results. In some embodiments, the data is used for research use. For example, the data may be used to further optimize the inclusion or elimination of markers as useful indicators of a particular condition or stage of disease.

When the amount(s) or level(s) of the one or more metabolites in the sample are determined, the amount(s) or level(s) may be compared to organ failure metabolite-reference levels, such as -organ failure-positive and/or organ failure-negative reference levels to aid in diagnosing or to diagnose whether the subject has organ failure. Levels of the one or more metabolites in a sample corresponding to the organ failure-positive reference levels (e.g., levels that are the same as the reference levels, substantially the same as the reference levels, above and/or below the minimum and/or maximum of the reference levels, and/or within the range of the reference levels) are indicative of a diagnosis of organ failure in the subject. Levels of the one or more metabolites in a sample corresponding to the organ failure-negative reference levels (e.g., levels that are the same as the reference levels, substantially the same as the reference levels, above and/or below the minimum and/or maximum of the reference levels, and/or within the range of the reference levels) are indicative of a diagnosis of no organ failure in the subject. In addition, levels of the one or more metabolites that are differentially present (especially at a level that is statistically significant) in the sample as compared to organ failure-negative reference levels are indicative of a diagnosis of organ failure in the subject. Levels of the one or more metabolites that are differentially present (especially at a level that is statistically significant) in the sample as compared to organ failure-positive reference levels are indicative of a diagnosis of no organ failure in the subject.

The level(s) of the one or more metabolites may be compared to organ failure-positive and/or organ failure-negative reference levels using various techniques, including a simple comparison (e.g., a manual comparison) of the level(s) of the one or more metabolites in the biological sample to organ failure-positive and/or organ failure-negative reference levels. The level(s) of the one or more metabolites in the biological sample may also be compared to organ failure-positive and/or organ failure-negative reference levels using one or more statistical analyses (e.g., t-test, Welch's t-test, Wilcoxon's rank sum test, random forests, support vector machines, linear discriminant analysis, k nearest neighbours).

Compositions for use (e.g., sufficient for, necessary for, or useful for) in the diagnostic methods of some embodiments of the present invention include reagents for detecting the presence or absence of organ failure specific metabolites. Any of these compositions, alone or in combination with other compositions of the present invention, may be provided in the form of a kit. Kits may further comprise appropriate controls and/or detection reagents.

Embodiments of the present invention provide for multiplex or panel assays that simultaneously detect one or more of the markers of the present invention depicted in tables 1 to 3, alone or in combination with additional organ failure markers known in the art. For example, in some embodiments, panel or combination assays are provided that detected 2 or more, 3 or more, 4 or more, 5 or more, 6 or more, 7 or more, 8 or more, 9 or more, 10 or more, 15 or more, or 20 or more, 30 or more, 40 or more markers in a single assay. In some embodiments, assays are automated or high throughput.

A preferred embodiment of the present invention is the use of markers listed in tables 2 and 3 for prediction/diagnosis of organ failure and its duration/severity where said mammalian subject is a human being, said biological sample blood and/or blood cells.

In some embodiments, additional organ failure markers are included in multiplex or panel assays. Markers are selected for their predictive value alone or in combination with the metabolic markers described herein.

### Therapeutic Methods

In some embodiments, the present invention provides therapeutic methods (e.g., that target the organ failure specific metabolites described herein). In some embodiments, the therapeutic methods target enzymes or pathway components of the organ failure specific metabolites described herein.

For example, in some embodiments, the present invention provides compounds that target the organ failure specific metabolites of the present invention. The compounds may decrease the level of organ failure specific metabolite by, for example, interfering with synthesis of the organ failure specific metabolite (e.g., by blocking transcription or translation of an enzyme involved in the synthesis of a metabolite, by inactivating an enzyme involved in the synthesis of a metabolite (e.g., by post translational modification or binding to an irreversible inhibitor), or by otherwise inhibiting the activity of an enzyme involved in the synthesis of a metabolite) or a precursor or metabolite thereof, by binding to and inhibiting the function of the organ failure specific metabolite, by binding to the target of the organ failure specific metabolite (e.g., competitive or non competitive inhibitor), or by increasing the rate of break down or clearance of the metabolite.

The compounds may increase the level of organ failure specific metabolite by, for example, inhibiting the break down or clearance of the organ failure specific metabolite (e.g., by inhibiting an enzyme involved in the breakdown of the metabolite), by increasing the level of a precursor of the organ failure specific metabolite, or by increasing the affinity of the metabolite for its target.

Dosing is dependent on severity and responsiveness of the disease state to be treated, with the course of treatment lasting from several days to several months, or until a cure is effected or a diminution of the disease state is achieved. Optimal dosing schedules can be calculated from measurements of drug accumulation in the body of the patient. The administering physician can easily determine optimum dosages, dosing methodologies and repetition rates.

In some embodiments, the present invention provides drug screening assays (e.g., to screen for anti - organ failure drugs). The screening methods of the present invention utilize organ failure specific metabolites described herein. As described above, in some embodiments, test compounds are small molecules, nucleic acids, or antibodies. In some embodiments, test compounds target organ failure specific metabolites directly. In other embodiments, they target enzymes involved in metabolic pathways of organ failure specific metabolites.

### EXPERIMENTAL

The following examples are provided in order to demonstrate and further illustrate certain preferred embodiments and aspects of the present invention and are not to be construed as limiting the scope thereof.

### General Analytics:

Sample preparation and metabolomic analyses were performed at BIOCRATES life sciences AG, Innsbruck, Austria. We used a multi-parametric, highly robust, sensitive and high-throughput targeted metabolomic platform consisting of flow injection analysis (FIA)-MS/MS and LC-MS/MS methods for the simultaneous quantification of a broad range of endogenous intermediates namely from the panel disclosed in table 1. All procedures (sample handling, analytics) were performed by co-workers blinded to the groups.

### Plasma homogenization

Plasma samples were prepared by standard procedures and stored at (-70°C). To enable analysis of all samples simultaneously within one batch, samples were thawed on ice (1 h) on the day of analysis and centrifuged at 18000 g at 2°C for 5 min. All tubes were prepared with 0.001% BHT (butylated hydroxytoluene; Sigma-Aldrich, Vienna, Austria) to prevent artificial formation of prostaglandins caused by autooxidation .

Liver tissue samples were homogenized using a Precellys® 24 homogenizer with Cryolys cooling module before analysis. Typically 50 mg of tissue were homogenized in ethanol : phosphate buffer 9:1 (v/v) for 30 min and unsolved material and beads for tissue desintegration removed by 5 min centrifugation at 10 000g.

### Acylcarnitines, Sphingomyelins, Hexoses, Glycerophospholipids (FIA-MS/MS)

To determine the concentration of acylcarnitines, sphingomyelins and glycerophospholipids in brain homogenates and in plasma the Absolute/*DQ* kit p150 (Biocrates Life Sciences AG) was prepared as described in the manufacturer's protocol. In brief, 10 µL of brain homogenate was added to the center of the filter on the upper 96-well kit plate, and the samples were dried using a nitrogen evaporator (VLM Laboratories). Subsequently, 20 µL of a 5 % solution of phenyl-isothiocyanate was added for derivatization. After incubation, the filter spots were dried again using an evaporator. The metabolites were extracted using 300 µL of a 5 mM ammonium acetate solution in methanol. The extracts were obtained by centrifugation into the lower 96-deep well plate followed by a dilution step with 600 µL of kit MS running solvent. Mass spectrometric analysis was performed on an API4000 QTrap® tandem mass spectrometry instrument (Applied Biosystems/MDS Analytical Technologies) equipped with an electro-spray ionization (ESI)-source using the analysis acquisition method as provided in the Absolute/*DQ* kit. The standard FIA-MS/MS method was applied for all measurements with two subsequent 20 µL injections (one for positive and one for negative mode analysis). Multiple reaction monitoring (MRM) detection was used for quantification applying the spectra parsing algorithm integrated into the MetIQ software (Biocrates Life Sciences AG). Concentration values for 148 metabolites (all analytes determined with the metabolomics kit besides of the amino acids, which were determined by a different method) obtained by internal calibration were exported for comprehensive statistical analysis.

### Amino acids, Biogenic amines (LC-MS/MS)

Amino acids and biogenic amines were quantitatively analyzed by reversed phase LC-MS/MS to obtain chromatographic separation of isobaric (same MRM ion pairs) metabolites for individual quantitation performed by external calibration and by use of internal standards. 10 µL sample volume (plasma, brain homogenate) is required for the analysis using the following sample preparation procedure. Samples were added on filter spots placed in a 96- solvinert well plate (internal standards were placed and dried down under nitrogen before), fixed above a 96 deep well plate (capture plate). 20 µL of 5% phenyl-isothiocyanate derivatization reagent was added. The derivatized samples were extracted after incubation by aqueous methanol into the capture plate. Sample extracts were analyzed by LC-ESI-MS/MS in positive MRM detection mode with an AP14000 QTrap® tandem mass spectrometry instrument (Applied Biosystems/MDS Analytical Technologies). The analyzed individual metabolite concentrations (Analyst 1.4.2 software, Applied Biosystems) were exported for comprehensive statistical analysis.

### Bile acids (LC-MSlMS)

A highly selective reversed phase LC-MS/MS analysis method in negative MRM detection mode was applied to determine the concentration of bile acids in plasma samples. Samples were extracted via dried filter spot technique in 96 well plate format, which is well suitable for high throughput analysis. For highly accurate quantitation internal standards and external calibration were applied. In brief, internal standards and 20 µL sample volume placed onto filter spots were extracted and simultaneously protein precipitated with aqueous methanol. These sample extracts were measured by LC-ESI-MS/MS with an API4000 QTrap® tandem mass spectrometry instrument (Applied Biosystems/MDS Analytical Technologies). Data of bile acids were quantified with Analyst 1.4.2 software (Applied Biosystems) and finally exported for comprehensive statistical analysis.

### Prostanoids, oxidized fatty acids (LC-MS/MS)

Prostanoids - a term summarizing prostaglandins (PG), thromboxanes (TX) and prostacylines - and oxidised fatty acid metabolites were analyzed in plasma extracts by LC-ES1-MS/MS *[*Unterwurzacher at al. Clin Chem Lab Med 2008; 46 (11):1589-1597*]* and in brain homogenate extracts by online solid phase extraction (SPE)-LC-MS/MS *[*Unterwurzacher et al. Rapid Commun Mass Spec submitted*]* with an API4000 QTrap® tandem mass spectrometry instrument (Applied Biosystems/MDS Analytical Technologies) in negative MRM detection mode. The sample preparation was the same for both, plasma and brain homogenates. In brief, filter spots in a 96 well plate were spiked with internal standard; 20 µL of plasma or tissue homogenates were added and extracted with aqueous methanol, the individual extracts then were analysed. Data of prostanoids and oxidized fatty acids were quantified with Analyst 1.4.2 software (Applied Biosystems) and finally exported for statistical analysis.

### Oxysterols

Oxysterols are determined after extraction and saponification by HPLC-Tandem mass spectrometer (HPLC-API-MS/MS) in positive detection mode using Multiple Reaction Mode (MRM).

Samples (20 µL), calibrators and internal standard mixture were placed into a capture plate and were protein precipitated in the first step by means of addition of 200 µL acetonitrile and centrifugation. 180 µL of the appropriate supernatants were transferred on a new filter plate with 7 mm filter spots, dried down, hydrolysed with 0.35 M KOH in 95 % Ethanol and after washing steps extracted with 100 µL aqueous MeOH. An aliquot of the extracted sample is injected onto the HPLC-MS/MS system. Chromatographic separation and detection is performed by using a Zorbax Eclipse XDB C18, 150 x 2.0 mm, 3.5 µm HPLC-Column at a flow rate of 0.3 mL/min followed by electrospray ionization on the API4000/QTRAP4000 tandem mass spectrometer. For the quantitation the Analyst Quantitation software from Applied Bioystems was used.

### Energy metabolism (Organic Acids) (LC-MS/MS)

For the quantitative analysis of energy metabolism intermediates (glycolysis, citrate cycle, pentose phosphate pathway, urea cycle) hdyrophilic interaction liquid chromatography (HILIC)-ESI-MS/MS method in highly selective negative MRM detection mode was used. The MRM detection was performed using an API4000 QTrap® tandem mass spectrometry instrument (Applied Biosystems/MDS Analytical Technologies). 20 µL sample volume (plasma, brain homogenate) was protein precipitated and extracted simultaneously with aqueous methanol in a 96 well plate format. Internal standards (ratio external to internal standard) and external calibration were used for highly accurate quantitation. Data were quantified with Analyst 1.4.2 software (Applied Biosystems) and finally exported for statistical analysis.

**Table 1 summarizes analyzed metabolites and respective abbreviations; Glycero-phospholipids are further differentiated with respect to the presence of ester (a) and ether (e) bonds in the glycerol moiety, where two letters (aa, ea, or ee) denote that the first and the second position of the glycerol scaffold are bound to a fatty acid residue, whereas a single letter (a or e) indicates a bond with only one fatty acid residue; e.g. PC_ea_33:1 denotes a plasmalogen phosphatidylcholine with 33 carbons in the two fatty acid side chains and a single double bond in one of them.**

| **Lab name** | **Family** |
|---|---|
| C0 | Ac.Ca. |
| C10 | Ac.Ca. |
| C10:1 | Ac.Ca. |
| C10:2 | Ac.Ca. |
| C12 | Ac.Ca. |
| C12-DC | Ac.Ca. |
| C12:1 | Ac.Ca. |
| C14 | Ac.Ca. |
| C14:1 | Ac.Ca. |
| C14:1-OH | Ac.Ca. |
| C14:2 | Ac.Ca. |
| C14:2-OH | Ac.Ca. |
| C16 | Ac.Ca. |
| C16-OH | Ac.Ca. |
| C16:1 | Ac.Ca. |
| C16:1-OH | Ac.Ca. |
| C16:2 | Ac.Ca. |
| C16:2-OH | Ac.Ca. |
| C18 | Ac.Ca. |
| C18:1 | Ac.Ca. |
| C18:1-OH | Ac.Ca. |
| C18:2 | Ac.Ca. |
| C2 | Ac.Ca. |
| C3 | Ac.Ca. |
| C3-OH | Ac.Ca. |
| C3:1 | Ac.Ca. |
| C4 | Ac.Ca. |
| C4-OH (C3-DC) | Ac.Ca. |
| C4:1 | Ac.Ca: |
| C5 | Ac.Ca. |
| C5-DC (C6-OH) | Ac.Ca. |
| C5-M-DC | Ac.Ca. |
| C5-OH (C3-DC-M) | Ac.Ca. |
| C5:1 | Ac.Ca. |
| C5:1-DC | Ac.Ca. |
| C6 (C4:1-DC) | Ac.Ca. |
| C6:1 | Ac.Ca. |
| C7-DC | Ac.Ca. |
| C8 | Ac.Ca. |
| C8:1 | Ac.Ca. |
| C9 | Ac.Ca. |
| H1 | Sug. |
| SM (OH) C14:1 | S.L. |
| SM (OH) C16:1 | S.L. |
| SM (OH) C22:1 | S.L. |
| SM (OH) C22:2 | S.L. |
| SM (OH) C24:1 | S.L. |
| SM C26:0 | S.L. |
| SM C26:1 | S.L. |
| PC aa C24:0 | GP.L. |
| PC aa C26:0 | GP.L. |
| PC aa C28:1 | GP.L. |
| PC aa C32:3 | GP.L. |
| PC aa C34:4 | GP.L. |
| PC aa C36:6 | GP.L. |
| PC aa C38:0 | GP.L. |
| PC aa C40:1 | GP.L. |
| PC aa C40:2 | GP.L. |
| PC aa C40:3 | GP.L. |
| PC aa C42:0 | GP.L. |
| PC aa C42:1 | GP.L. |
| PC aa C42:2 | GP.L. |
| PC aa C42:4 | GP.L. |
| PC aa C42:5 | GP.L. |
| PC aa C42:6 | GP.L. |
| PC ae C30:0 | GP.L. |
| PC ae C30:1 | GP.L. |
| PC ae C30:2 | GP.L. |
| PC ae C32:2 | GP.L. |
| PC ae C36:0 | GP.L. |
| PC ae C38:0 | GP.L. |
| PC ae C40:0 | GP.L. |
| PC ae C40:1 | GP.L. |
| PC ae C40:2 | GP.L. |
| PC ae C40:3 | GP.L. |
| PC ae C40:4 | GP.L. |
| PC ae C40:6 | GP.L. |
| PC ae C42:0 | GP.L. |
| PC ae C42:1 | GP.L. |
| PC ae C42:2 | GP.L. |
| PC ae C42:3 | GP.L. |
| PC ae C42:4 | GP.L. |
| PC ae C42:5 | GP.L. |
| PC ae C44:3 | GP.L. |
| PC ae C44:4 | GP.L. |
| PC ae C44:5 | GP.L. |
| PC ae C44:6 | GP.L. |
| lysoPC a C14:0 | GP.L. |
| lysoPC a C16:1 | GP.L. |
| lysoPC a C17:0 | GP.L. |
| lysoPC a C20:3 | GP.L. |
| lysoPC a C24:0 | GP.L. |
| lysoPC a C26:0 | GP.L. |
| lysoPC a C26:1 | GP.L. |
| lysoPC a C28:0 | GP.L. |
| lysoPC a C28:1 | GP.L. |
| lysoPC a C6:0 | GP.L. |
| Gly | Am.Ac. |
| Ala | Am.Ac. |
| Ser | Am.Ac. |
| Pro | Am.Ac. |
| Val | Am.Ac. |
| Thr | Am.Ac. |
| Xle | Am.Ac. |
| Leu | Am.Ac. |
| Ile | Am.Ac. |
| Asn | Am.Ac. |
| Asp | Am.Ac. |
| Gln | Am.Ac. |
| Glu | Am.Ac. |
| Met | Am.Ac. |
| His | Am.Ac. |
| Phe | Am.Ac. |
| Arg | Am.Ac. |
| Cit | Am.Ac. |
| Tyr | Am.Ac. |
| Trp | Am.Ac. |
| Orn | Am.Ac. |
| Lys | Am.Ac. |
| ADMA | B.Am. |
| total DMA | B.Am. |
| Met-SO | Am.Ac. |
| Kyn | B.Am. |
| Putrescine | B.Am. |
| Spermidine | B.Am. |
| Spermine | B.Am. |
| Creatinine | B.Am. |
| 9-HODE | P.G. |
| 13S-HODE | P.G. |
| 12S-HETE | P.G. |
| 15S-HETE | P.G. |
| LTB4 | P.G. |
| DHA | P.G. |
| PGE2 | P.G. |
| PGD2 | P.G. |
| AA | P.G. |
| Lac | En.Met. |
| Suc | En.Met. |
| Hex | En. Met. |
| 22ROHC | Ox.St. |
| 24SOHC | Ox.St. |
| 250HC | Ox.St. |
| 270HC | Ox. St. |
| THC | Ox.St. |
| 7aOHC | Ox.St. |
| 7KC | Ox.St. |
| 5a,6a,EPC | Ox.St. |
| 4BOHC | Ox.St. |
| Desmosterol | Ox.St. |
| 7DHC | Ox.St. |
| Lanosterol | Ox.St. |
| PE a C16:0 | GP.L. |
| PE a C18:0 | GP.L. |
| PE a C18:1 | GP.L. |
| PE a C18:2 | GP.L. |
| PE a C20:4 | GP.L. |
| PE a C22:4 | GP.L. |
| PE a C22:5 | GP.L. |
| PE a C22:6 | GP.L. |
| PE e C18:0 | GP.L. |
| PG e C14:2 | GP.L. |
| PE aa C20:0 | GP.L. |
| PE aa C22:2 | GP.L. |
| PE aa C26:4 | GP.L. |
| PE aa C28:4 | GP.L. |
| PE aa C28:5 | GP.L. |
| PE aa C34:0 | GP.L. |
| PE aa C34:1 | GP.L. |
| PE aa C34:2 | GP.L. |
| PE aa C34:3 | GP.L. |
| PE aa C36:0 | GP.L. |
| PE aa C36:1 | GP.L. |
| PE aa C36:2 | GP.L. |
| PE aa C36:3 | GP.L. |
| PE aa C36:4 | GP.L. |
| PE aa C36:5 | GP.L. |
| PE aa C38:0 | GP.L. |
| PE aa C38:1 | GP.L. |
| PE aa C38:2 | GP.L. |
| PE aa C38:3 | GP.L. |
| PE aa C38:4 | GP.L. |
| PE aa C38:5 | GP.L. |
| PE aa C38:6 | GP.L. |
| PE aa C38:7 | GP.L. |
| PE aa C40:2 | GP.L. |
| PE aa C40:3 | GP.L. |
| PE aa C40:4 | GP.L. |
| PE aa C40:5 | GP.L. |
| PE aa C40:6 | GP.L. |
| PE aa C40:7 | GP.L. |
| PE aa C48:1 | GP.L. |
| PE ae C34:1 | GP.L. |
| PE ae C34:2 | GP.L. |
| PE ae C34:3 | GP.L. |
| PE ae C36:1 | GP.L. |
| PE ae C36:2 | GP.L. |
| PE ae C36:3 | GP.L. |
| PE ae C36:4 | GP.L. |
| PE ae C36:5 | GP.L. |
| PE ae C38:1 | GP.L. |
| PE ae C38:2 | GP.L. |
| PE ae C38:3 | GP.L. |
| PE ae C38:4 | GP.L. |
| PE ae C38:5 | GP.L. |
| PE ae C38:6 | GP.L. |
| PE ae C40:1 | GP.L. |
| PE ae C40:2 | GP.L. |
| PE ae C40:3 | GP.L. |
| PE ae C40:4 | GP.L. |
| PE ae C40:5 | GP.L. |
| PE ae C40:6 | GP.L. |
| PE ae C42:1 | GP.L. |
| PE ae C42:2 | GP.L. |
| PE ae C46:5 | GP.L. |
| PE ae C46:6 | GP.L. |
| PG aa C30:0 | GP.L. |
| PG aa C32:0 | GP.L. |
| PG aa C32:1 | GP.L. |
| PG aa C33:6 | GP.L. |
| PG aa C34:0 | GP.L. |
| PG aa C34:1 | GP.L. |
| PG aa C34:2 | GP.L. |
| PG aa C34:3 | GP.L. |
| PG aa C36:0 | GP.L. |
| PG aa C36:1 | GP.L. |
| PG aa C36:2 | GP.L. |
| PG aa C36:3 | GP.L. |
| PG aa C36:4 | GP.L. |
| PG aa C38:5 | GP.L. |
| PG ae C32:0 | GP.L. |
| PG ae C34:0 | GP.L. |
| PG ae C34:1 | GP.L. |
| PG ae C36:1 | GP.L. |
| PS aa C34:1 | GP.L. |
| PS aa C34:2 | GP.L. |
| PS aa C36:0 | GP.L. |
| PS aa C36:1 | GP.L. |
| PS aa C36:2 | GP.L. |
| PS aa C36:3 | GP.L. |
| PS aa C36:4 | GP.L. |
| PS aa C38:1 | GP.L. |
| PS aa C38:2 | GP.L. |
| PS aa C38:3 | GP.L. |
| PS aa C38:4 | GP.L. |
| PS aa C38:5 | GP.L. |
| PS aa C40:1 | GP.L. |
| PS aa C40:2 | GP.L. |
| PS aa C40:3 | GP.L. |
| PS aa C40:4 | GP.L. |
| PS aa C40:5 | GP.L. |
| PS aa C40:6 | GP.L. |
| PS aa C40:7 | GP.L. |
| PS aa C42:1 | GP.L. |
| PS aa C42:2 | GP.L. |
| PS aa C42:4 | GP.L. |
| PS aa C42:5 | GP.L. |
| PS ae C34:2 | GP.L. |
| PS ae C36:1 | GP.L. |
| PS ae C36:2 | GP.L: |
| PS ae C38:4 | GP.L. |
| SM C14:0 | S.L. |
| SM C16:0 | S.L. |
| SM C16:1 | S.L. |
| SM C17:0 | S.L. |
| SM C18:0 | S.L. |
| SM C18:1 | S.L. |
| SM C19:0 | S.L. |
| SM C19:1 | S.L. |
| SM C19:2 | S.L. |
| SM C20:0 | S.L. |
| SM C20:1 | S.L. |
| SM C20:2 | S.L. |
| SM C21:0 | S.L. |
| SM C21:1 | S.L. |
| SM C21:2 | S.L. |
| SM C21:3 | S.L. |
| SM C22:0 | S.L. |
| SM C22:1 | S.L. |
| SM C22:2 | S.L. |
| SM C22:3 | S.L. |
| SM C23:0 | S.L. |
| SM C23:1 | S.L. |
| SM C23:2 | S.L. |
| SM C23:3 | S.L. |
| SM C24:0 | S.L. |
| SM C24:1 | S.L. |
| SM C24:2 | S.L. |
| SM C24:3 | S.L. |
| SM C24:4 | S.L. |
| SM C26:3 | S.L. |
| SM C26:4 | S.L. |
| SM C3:0 | S.L. |
| lysoPC a C16:0 | GP.L. |
| lysoPC a C18:0 | GP.L. |
| lysoPC a C 18:1 | GP.L. |
| lysoPC a C 18:2 | GP.L. |
| lysoPC a C20:4 | GP.L. |
| PC e C18:0 | GP.L. |
| PC aa C30:0 | GP.L. |
| PC aa C30:1 | GP.L. |
| PC aa C30:2 | GP.L. |
| PC aa C32:0 | GP.L. |
| PC aa C32:1 | GP.L. |
| PC aa C32:2 | GP.L. |
| PC aa C34:0 | GP.L. |
| PC aa C34:1 | GP.L. |
| PC aa C34:2 | GP.L. |
| PC aa C34:3 | GP.L. |
| PC aa C36:0 | GP.L. |
| PC aa C36:1 | GP.L. |
| PC aa C36:2 | GP.L. |
| PC aa C36:3 | GP.L. |
| PC aa C36:4 | GP.L. |
| PC aa C36:5 | GP.L. |
| PC aa C38:1 | GP.L. |
| PC aa C38:2 | GP.L. |
| PC aa C38:3 | GP.L. |
| PC aa C38:4 | GP.L. |
| PC aa C38:5 | GP.L. |
| PC aa C38:6 | GP.L. |
| PC aa C40:4 | GP.L. |
| PC aa C40:5 | GP.L. |
| PC aa C40:6 | GP.L. |
| PC aa C40:7 | GP.L. |
| PC aa C40:8 | GP.L. |
| PC ae C32:0 | GP.L. |
| PC ae C32:1 | GP.L. |
| PC ae C32:6 | GP.L. |
| PC ae C34:0 | GP.L. |
| PC ae C34:1 | GP.L. |
| PC ae C34:2 | GP.L. |
| PC ae C34:3 | GP.L. |
| PC ae C34:6 | GP.L. |
| PC ae C36:1 | GP.L. |
| PC ae C36:2 | GP.L. |
| PC ae C36:3 | GP.L. |
| PC ae C36:4 | GP.L. |
| PC ae C36:5 | GP.L. |
| PC ae C38:1 | GP.L. |
| PC ae C38:2 | GP.L. |
| PC ae C38:3 | GP.L. |
| PC ae C38:4 | GP.L. |
| PC ae C38:5 | GP.L. |
| PC ae C38:6 | GP.L. |
| PC ae C40:5 | GP.L. |
| N-C2:0-Cer | Cer. |
| N-C3:1-Cer | Cer. |
| N-C3:0-Cerr | Cer. |
| N-C4:1-Cer | Cer. |
| N-C4:0-Cer | Cer. |
| N-C5:1-Cer | Cer. |
| N-C5:0-Cer | Cer. |
| N-C6:1-Cer | Cer. |
| N-C6:0-Cer | Cer. |
| N-C7:1-Cer | Cer. |
| N-C7:0-Cer | Cer. |
| N-C8:1-Cer | Cer. |
| N-C8:0-Cer | Cer. |
| N-C9:3-Cer | Cer. |
| N-C9:1-Cer | Cer. |
| N-C9:0-Cer | Cer. |
| N-C10:1-Cer | Cer. |
| N-C10:0-Cer | Cer. |
| N-C 11:1-Cer | Cer. |
| N-C11:0-Cer | Cer. |
| N-C12:1-Cer | Cer. |
| N-C12:0-Cer | Cer. |
| N-(OH)C11:0-Cer | Cer. |
| N-C13:1-Cer | Cer. |
| N-C13:0-Cer | Cer. |
| N-C14:1-Cer | Cer. |
| N-C14:0-Cer | Cer. |
| N-C15:1-Cer | Cer. |
| N-C15:0-Cer | Cer. |
| N-C16:1-Cer | Cer. |
| N-C16:0-Cer | Cer. |
| N-C17:1-Cer | Cer. |
| N-C17:0-Cer | Cer. |
| N-(2xOH)C15:0-Cer | Cer. |
| N-C18:1-Cer | Cer. |
| N-C18:0-Cer | Cer. |
| N-C19:1-Cer | Cer. |
| N-C19:0-Cer | Cer. |
| N-C20:1-Cer | Cer. |
| N-C20:0-Cer | Cer. |
| N-C21:1-Cer | Cer. |
| N-C21:0-Cer | Cer. |
| N-C22:1-Cer | Cer. |
| N-C22:0-Cer | Cer. |
| N-C23:1-Cer | Cer. |
| N-C23:0-Cer | Cer. |
| N-C24:1-Cer | Cer. |
| N-C24:0-Cer | Cer. |
| N-C25:1-Cer | Cer. |
| N-C25:0-Cer | Cer. |
| N-C26:1-Cer | Cer. |
| N-C26:0-Cer | Cer. |
| N-C27:1-Cer | Cer. |
| N-C27:0-Cer | Cer. |
| N-C28:1-Cer | Cer. |
| N-C28:0-Cer | Cer. |
| N-C2:0-Cer(2H) | Cer. |
| N-C3:1-Cer(2H) | Cer. |
| N-C3:0-Cer(2H) | Cer. |
| N-C4:1-Cer(2H) | Cer. |
| N-C4:0-Cer(2H) | Cer. |
| N-C5:1-Cer(2H) | Cer. |
| N-C5:0-Cer(2H) | Cer. |
| N-C6:1-Cer(2H) | Cer. |
| N-C6:0-Cer(2H) | Cer. |
| N-C7:1-Cer(2H) | Cer. |
| N-C7:0-Cer(2H) | Cer. |
| N-C8:1-Cer(2H) | Cer. |
| N-C8:0-Cer(2H) | Cer. |
| N-C9:1-Cer(2H) | Cer. |
| N-C9:0-Cer(2H) | Cer. |
| N-C10:1-Cer(2H) | Cer. |
| N-C10:0-Cer(2H) | Cer. |
| N-C11:1-Cer(2H) | Cer. |
| N-C11:0-Cer(2H) | Cer. |
| N-C12:1-Cer(2H) | Cer. |
| N-C12:0-Cer(2H) | Cer. |
| N-C13:1-Cer(2H) | Cer. |
| N-C13:0-Cer(2H) | Cer. |
| N-C14:1-Cer(2H) | Cer. |
| N-C14:0-Cer(2H) | Cer. |
| N-C15:1-Cer(2H) | Cer. |
| N-C15:0-Cer(2H) | Cer. |
| N-C16:1-Cer(2H) | Cer. |
| N-C16:0-Cer(2H) | Cer. |
| N-C17:1-Cer(2H) | Cer. |
| N-C17:0-Cer(2H) | Cer. |
| N-C18:1-Cer(2H) | Cer. |
| N-C18:0-Cer(2H) | Cer. |
| N-C19:1-Cer(2H) | Cer. |
| N-C19:0-Cer(2H) | Cer. |
| N-C18:0-Cer(2H) | Cer. |
| N-C20:0-Cer(2H) | Cer. |
| N-C21:1-Cer(2H) | Cer. |
| N-C21:0-Cer(2H) | Cer. |
| N-C22:1-Cer(2H) | Cer. |
| N-C22:0-Cer(2H) | Cer. |
| N-C23:1-Cer(2H) | Cer. |
| N-C23:0-Cer(2H) | Cer. |
| N-C24:1-Cer(2H) | Cer. |
| N-C24:0-Cer(2H) | Cer. |
| N-C25:1-Cer(2H) | Cer. |
| N-C25:0-Cer(2H) | Cer. |
| N-C26:1-Cer(2H) | Cer. |
| N-C26:0-Cer(2H) | Cer. |
| N-C27:1-Cer(2H) | Cer. |
| N-C27:0-Cer(2H) | Cer. |
| N-C28:1-Cer(2H) | Cer. |
| N-C28:0-Cer(2H) | Cer. |
| N-C3:0(OH)-Cer | Cer. |
| N-C4:0(OH)-Cer | Cer. |
| N-(2xOH)C3:0-Cer | Cer. |
| N-C5:0(OH)-Cer | Cer. |
| N-C6:0(OH)-Cer | Cer. |
| N-C7:2(OH)-Cer | Cer. |
| N-C7:1 (OH)-Cer | Cer. |
| N-C7:0(OH)-Cer | Cer. |
| N-C8:0(OH)-Cer | Cer. |
| N-C9:0(OH)-Cer | Cer. |
| N-C10:0(OH)-Cer | Cer. |
| N-C11:1 (OH)-Cer | Cer. |
| N-C11:0(OH)-Cer | Cer. |
| N-C12:0(OH)-Cer | Cer. |
| N-C13:0(OH)-Cer | Cer. |
| N-C14:0(OH)-Cer | Cer. |
| N-C15:0(OH)-Cer | Cer. |
| N-C16:0(OH)-Cer | Cer. |
| N-C17:1(OH)-Cer | Cer. |
| N-C17:0(OH)-Cer | Cer. |
| N-C18:0(OH)-Cer | Cer. |
| N-C19:0(OH)-Cer | Cer. |
| N-C20:0(OH)-Cer | Cer. |
| N-C19:0(2xOH)-Cer | Cer. |
| N-C21:0(OH)-Cer | Cer. |
| N-C22:0(OH)-Cer | Cer. |
| N-C23:0(OH)-Cer | Cer. |
| N-C24:0(OH)-Cer | Cer. |
| N-C23:0(2xOH)-Cer | Cer. |
| N-C25:0(OH)-Cer | Cer. |
| N-C26:1(OH)-Cer | Cer. |
| N-C26:0(OH)-Cer | Cer. |
| N-C27:0(OH)-Cer | Cer. |
| N-C28:0(OH)-Cer | Cer. |
| N-C3:0(OH)-Cer(2H) | Cer. |
| N-C4:0(OH)-Cer(2H) | Cer. |
| N-C5:0(OH)-Cer(2H) | Cer. |
| N-C6:0(OH)-Cer(2H) | Cer. |
| N-C7:0(OH)-Cer(2H) | Cer. |
| N-C8:0(OH)-Cer(2H) | Cer. |
| N-C9:0(OH)-Cer(2H) | Cer. |
| N-C10:0(OH)-Cer(2H) | Cer. |
| N-C11:0(OH)-Cer(2H) | Cer. |
| N-C13:0(OH)-Cer(2H) | Cer. |
| N-C14:0(OH)-Cer(2H) | Cer. |
| N-C15:0(OH)-Cer(2H) | Cer. |
| N-C16:0(OH)-Cer(2H) | Cer. |
| N-C17:0(OH)-Cer(2H) | Cer. |
| N-C18:0(OH)-Cer(2H) | Cer. |
| N-C19:0(OH)-Cer(2H) | Cer. |
| N-C20:0(OH)-Cer(2H) | Cer. |
| N-C21:0(OH)-Cer(2H) | Cer. |
| N-C22:0(OH)-Cer(2H) | Cer. |
| N-C23:0(OH)-Cer(2H) | Cer. |
| N-C24:0(OH)-Cer(2H) | Cer. |
| N-C25:0(OH)-Cer(2H) | Cer. |
| N-C26:0(OH)-Cer(2H) | Cer. |
| N-C27:0(OH)-Cer(2H) | Cer. |
| N-C28:0(OH)-Cer(2H) | Cer. |
| Histamine | B.Am. |
| Serotonin | B.Am. |
| PEA | B.Am. |
| TXB2 | P.G. |
| PGF2a | P.G. |
| 24,25,EPC | Ox.St. |
| 5B,6B,EPC | Ox.St. |
| 24DH Lan | Ox.St. |
| GCDCA | Bi.Ac. |
| GLCA | Bi.Ac. |
| TCDCA | Bi.Ac. |
| TLCA | Bi.Ac. |
| GCA | Bi.Ac. |
| CA | Bi.Ac. |
| UDCA | Bi.Ac. |
| CDCA | Bi.Ac. |
| DCA | Bi.Ac. |
| TDCA | Bi.Ac. |
| TLCAS | Bi.Ac. |
| GDCA | Bi.Ac. |
| GUDCA | Bi.Ac. |

### Detailed Examples

### 1. Human

We use data of 29 subjects where data are obtained by 17 patients with mixed sepsis (i.e., sepsis with mixed foci including peritonitis (4), pneumonia (5) and also unidentified foci (12 patients with mixed sepsis) developing a systemic infection (sepsis) associated organ failure. Diagnosis was confirmed diagnosis clinical criteria and microbiological evidence for infection (blood culture, PCR for pathogens).

### Statistical Analysis

All statistical calculations have been performed using the statistics software R (R: A Language and Environment for Statistical Computing, R Development Core Team, R Foundation for Statistical Computing,Vienna, Austria, 2009, ISBN 3-900051-07-0). Analytes that were detected in at least 15% of the samples were selected for further analyses resulting in a list of 521 unique compounds/metabolites (Table 1). The metabolic data is left censored due to thresholding of the mass spectrometer data resulting in non detected peak/signals. By a combination of metabolic pathway dynamism, complex sample molecular interaction and overall efficiency of the analytical protocol, replacement of missing data by means of a multivariate algorithm is preferred to a naive imputation by a pre-specified value like for instance zero. Hence, missing metabolite concentrations are replaced by the average value of the 6 closest samples to the one where the measurement is missing (impute: Imputation for microarray data, Hastie T., Tibshirani R., Narasimhan B. and Chu G., R package version 1.14.0). At the exception of fold change (FC) determination, all statistical analyses are performed on preprocessed - that is, log transformed - data.

The ImFit function in the package limma (Limma: linear models for microarray data, Smyth G.K. In: Bioinformatics and Computational Biology Solutions using R and Bioconductor, Springer, New York, pp 397-420, R package version 2.16.5) is used to compute the moderated statistics between measurements from septic patients samples and samples from patient developing organ failure. Resulting p values are adjusted by the method described in Benjamini and Hochberg (Benjamini Y. and Hochberg Y., Controlling the false discovery rate: a practical and powerful approach to multiple testing, Journal of the Royal Statistical Society Series B, 1995, 57, 289-300) leading to so-called q values.

Sensitivity/specificity properties of a classifier comprising one analyte or a combination of analytes are summarised in terms of Area Under the Receiver Operating Characteristic Curve (AUC). The function colAUC (caTools: Tools: moving window statistics, GIF, Base64, ROC AUC, etc., Tuszynski J., 2008, R package version 1.9) is used to compute and plot ROC curves. From the three univariate statistics (adjusted p value (q value), fold change and AUC), features are ranked according to a 2 step strategy: 1) the 3 measures are first used as input to the multiple objective algorithm described by Chen et al. (Chen J.J., Tsai C.-A., Tzeng S.-L.and Chen C.-H., Gene selection with multiple ordering criteria, BMC Bioinformatics 2007, 8:74) 2) ties (i.e. metabolites belonging to the same front) are broken according by simple Borda count. The function vennDiagram from the R package limma (Limma: linear models for microarray data, Smyth G.K. In: Bioinformatics and Computational Biology Solutions using R and Bioconductor, Springer, New York, pp 397-420, R package version 2.16.5) is employed to display the number of features selected by each ranking technique; confer Figure 1. Numbers in dark (resp. grey) express the count of metabolites that exhibit higher (resp. lower) concentration in the samples of those patients developing organ failure than in the septic patients samples. Following thresholds are used: adjusted p value (q-value) less than 0.01, absolute fold change higher than 50% and AUC greater than 0.8.

In addition to univariate statistics, additional ranking that take into account multivariate interactions is computing from boosted regression tree models. Similarly to the variable importance measures in Breiman's Random Forests, feature relative influence is determined as the effect of class labels permutation on reducing the loss function (Friedman J.H., Greedy Function Approximation: A Gradient Boosting Maof Statistics, 2001, 29(5):1189-1232). gbm function from gbm R package (gbm: Generalized Boosted Regression Models, Ridgeway G., 2007, R package version 1.6-3) was used to perform tree based gradient boosting specifying a gaussian loss function, a shrinkage parameter of 0.05 and allowing trees with up to 3 trees splits. To reduce variance in the ranking, feature relevance score is presented as the average rank calculated by leaving one set out on the training set.

Performance of single markers as well as of combinations of markers is assessed by three classification algorithms that rely on different mechanisms to ensure that the outcome is not dependent on the modelling technique: support vector machine (SVM) with linear kernel using the R function svm in package e1071 (e1071: Misc Functions of the Department of Statistics (e1071), Dimitriadou E., Hornik k., Leisch F., Meyer D. and Weingessel A., R package version 1.5-19); diagonal discriminant analysis (DLDA) using the R function dDa in package sfsmisc (sfsmisc: Utilities from Seminar fuer Statistik ETH Zurich, Maechler M., R package version 1.0-7) and the nearest neighbour algorithm(KNN) with k equal to one using the R function knn in package class (Modern Applied Statistics with S, Venables W.N. And Ripley B.D., Springer, New York, R package version 7.2-47). Predictive abilities of the models are computed using stratified boostrap (B=20), repeated 10 times to obtain a performance estimate and its associated variance (FIEmspro: Flow Injection Electrospray Mass Spectrometry Processing: data processing, classification modelling and variable selection in metabolite fingerprinting, Beckmann M., Enot D. and Lin W., 2007, R package version 1.1-0).

Based on the accuracy computations for the three classification algorithms SVM, DLDA, and KNN (cf. Figures 2 and 3) we select the top 60 metabolites for the ranker combining adjusted p values, fold change and AUC as well as for the multivariate wrapper which uses boosted regression trees leading to 97 different analytes and metabolites; confer Table 2.

**Table 2 depicts the ranks of the individual analytes and metabolites in terms of discriminatory power for detecting the onset of infection associated organ failure. Ranking was performed using a ranker combining adjusted p values, fold changes and AUCs as well as using a multivariate wrapper which is based on boosted regression trees as described above. For additional information see Fig 1-3.**

| **Name** | **Univariate rank** | **Adjusted p value** | **Fold change** | **AUC** | **Multivariate rank** |
|---|---|---|---|---|---|
| C0 | 290 | 9,85E-001 | 40,23 | 0,50 | 27 |
| C12-DC | 386 | 6,06E-001 | -0,79 | 0,58 | 43 |
| C14:1 | 4 | 1,64E-003 | 106,12 | 0,93 | 13 |
| C14:1-OH | 326 | 5,75E-001 | 13,33 | 0,63 | 56 |
| C14:2 | 60 | 2,25E-001 | 90,48 | 0,82 | 16 |
| C14:2-OH | 214 | 3,45E-001 | 44,44 | 0,70 | 29 |
| C18 | 200 | 6,06E-001 | 56,00 | 0,66 | 26 |
| C6:1 | 31 | 6,06E-001 | -325,41 | 0,64 | 124 |
| SM (OH) C22:1 | 2 | 4,39E-005 | 111,63 | 0,92 | 39 |
| SM (OH) C22:2 | 24 | 1,03E-004 | 87,48 | 0,90 | 254 |
| SM (OH) C24:1 | 50 | 1,25E-004 | 77,60 | 0,88 | 38 |
| SM C26:0 | 57 | 2,79E-003 | 89,00 | 0,83 | 298 |
| SM C26:1 | 19 | 4,44E-005 | 84,43 | 0,91 | 169 |
| PC aa C28:1 | 256 | 1,48E-001 | 10,75 | 0,64 | 52 |
| PC aa C38:0 | 27 | 2,57E-003 | 103,52 | 0,85 | 209 |
| PC aa C42:0 | 58 | 1,55E-002 | 91,30 | 0,80 | 154 |
| PC aa C42:1 | 36 | 2,73E-003 | 102,52 | 0,85 | 253 |
| PC ae C40:1 | 33 | 1,83E-003 | 96,56 | 0,88 | 500 |
| PC ae C40:2 | 39 | 2,73E-003 | 91,53 | 0,87 | 455 |
| PC ae C40:6 | 32 | 2,22E-004 | 81,86 | 0,92 | 108 |
| PC ae C42:2 | 10 | 2,57E-003 | 147,86 | 0,84 | 419 |
| PC ae C42:3 | 8 | 2,96E-003 | 134,67 | 0,87 | 331 |
| PC ae C42:4 | 41 | 1,37E-002 | 126,49 | 0,79 | 50 |
| PC ae C44:5 | 42 | 9,27E-002 | 182,51 | 0,74 | 141 |
| PC ae C44:6 | 29 | 1,90E-002 | 120,88 | 0,81 | 61 |
| lysoPC a C20:3 | 54 | 4,48E-002 | 118,52 | 0,73 | 93 |
| lysoPC a C26:0 | 298 | 4,27E-001 | 18,11 | 0,56 | 41 |
| Phe | 251 | 9,40E-001 | -27,92 | 0,70 | 60 |
| THC | 15 | 7,04E-002 | -380,12 | 0,80 | 6 |
| 7KC | 17 | 7,04E-002 | -437,25 | 0,76 | 74 |
| 5a,6a,EPC | 18 | 7,04E-002 | -224,71 | 0,75 | 37 |
| PE a C18:1 . | 53 | 8,50E-002 | 144,30 | 0,74 | 487 |
| PE a C18:2 | 30 | 9,15E-002 | 248,48 | 0,75 | 389 |
| PE a C20:4 | 49 | 5,45E-002 | 122,02 | 0,77 | 334 |
| PE a C22:5 | 47 | 1,02E-001 | 136,84 | 0,76 | 394 |
| PE a C22:6 | 16 | 4,74E-002 | 195,51 | 0,74 | 281 |
| PE aa C38:0 | 119 | 5,41E-003 | 52,04 | 0,85 | 58 |
| PE aa C38:2 | 59 | 7,01E-002 | 108,83 | 0,76 | 395 |
| SM C16:0 | 46 | 1,97E-005 | 60,14 | 0,93 | 64 |
| SM C17:0 | 56 | 7,25E-005 | 64,61 | 0,91 | 3 |
| SM C18:0 | 83 | 2,11E-004 | 54,73 | 0,88 | 40 |
| SM C19:0 | 52 | 4,44E-005 | 48,58 | 0,94 | 36 |
| SM C21:1 | 48 | 4,44E-005 | 62,77 | 0,90 | 63 |
| SM C21:3 | 45 | 6,41E-005 | 69,05 | 0,95 | 20 |
| SM C22:2 | 28 | 5,09E-006 | 58,61 | 0,96 | 14 |
| SM C23:0 | 6 | 1,56E-005 | 75,15 | 0,96 | 4 |
| SM C23:1 | 25 | 6,88E-005 | 79,68 | 0,91 | 161 |
| SM C23:2 | 26 | 9,32E-006 | 70,13 | 0,94 | 62 |
| SM C23:3 | 44 | 9,97E-005 | 73,55 | 0,92 | 197 |
| SM C24:0 | 3 | 3,89E-006 | 78,55 | 0,96 | 42 |
| SM C24:1 | 20 | 9,99E-006 | 77,52 | 0,95 | 35 |
| SM C24:2 | 5 | 2,71E-006 | 73,35 | 0,98 | 9 |
| SM C24:3 | 11 | 2,71E-006 | 55,12 | 0,99 | 21 |
| SM C24:4 | 38 | 2,64E-004 | 85,17 | 0,86 | 137 |
| SM C26:4 | 43 | 2,11E-004 | 83,13 | 0,89 | 104 |
| SM C3:0 | 13 | 2,08E-003 | 171,48 | 0,80 | 66 |
| lysoPC a C18:2 | 14 | 1,06E-002 | 180,95 | 0,78 | 178 |
| lysoPC a C20:4 | 23 | 8,22E-003 | 153,07 | 0,80 | 17 |
| PC aa C36:4 | 35 | 4,82E-005 | 64,50 | 0,95 | 8 |
| PC aa C38:1 | 37 | 1,39E-004 | 77,32 | 0,93 | 267 |
| PC aa C38:2 | 21 | 1,39E-004 | 86,17 | 0,93 | 215 |
| PC aa C38:4 | 79 | 7,00E-004 | 60,00 | 0,90 | 18 |
| PC aa C38:5 | 12 | 4,71E-005 | 58,58 | 0,99 | 15 |
| PC aa C38:6 | 40 | 2,10E-003 | 90,17 | 0,86 | 120 |
| PC aa C40:5 | 68 | 2,79E-004 | 73,08 | 0,90 | 28 |
| PC aa C40:6 | 51 | 1,83E-003 | 84,16 | 0,89 | 55 |
| PC aa C40:7 | 55 | 2,22E-004 | 73,36 | 0,91 | 182 |
| PC aa C40:8 | 9 | 2,57E-003 | 119,32 | 0,86 | 151 |
| PC ae C36:4 | 70 | 1,31E-003 | 70,81 | 0,90 | 30 |
| PC ae C36:5 | 22 | 2,91E-004 | 87,31 | 0,94 | 10 |
| PC ae C38:4 | 7 | 4,82E-005 | 79,47 | 0,94 | 98 |
| PC ae C38:6 | 1 | 4,82E-005 | 96,66 | 0,97 | 59 |
| N-C2:0-Cer | 312 | 8,48E-001 | 20,06 | 0,65 | 25 |
| N-C7:0-Cer | 209 | 6,02E-001 | 44,44 | 0,71 | 46 |
| N-C9:3-Cer? | 144 | 4,45E-001 | 71,25 | 0,73 | 57 |
| N-C17:1-Cer | 354 | 9,99E-001 | -22,50 | 0,61 | 49 |
| N-C22:1-Cer | 364 | 9,99E-001 | -27,07 | 0,51 | 23 |
| N-C25:0-Cer | 34 | 2,95E-003 | 88,98 | 0,91 | 12 |
| N-C27:1-Cer | 253 | 4,68E-001 | 17,49 | 0,70 | 19 |
| N-C5:1-Cer(2H) | 178 | 9,52E-001 | 62,93 | 0,68 | 5 |
| N-C7:1-Cer(2H) | 289 | 9,52E-001 | 31,68 | 0,67 | 48 |
| N-C8:1-Cer(2H) | 254 | 9,52E-001 | 41,96 | 0,66 | 22 |
| N-C11:1-Cer(2H) | 311 | 9,99E-001 | 31,82 | 0,62 | 53 |
| N-C20:0-Cer(2H) | 103 | 1,29E-001 | 80,11 | 0,76 | 33 |
| N-C21:0-Cer(2H) | 457 | 9,99E-001 | 4,89 | 0,58 | 24 |
| N-C22:1-Cer(2H) | 223 | 4,45E-001 | 48,84 | 0,67 | 54 |
| N-C25:1-Cer(2H) | 228 | 4,45E-001 | 38,12 | 0,71 | 11 |
| N-C26:1-Cer(2H) | 140 | 3,45E-001 | 59,22 | 0,80 | 31 |
| N-C6:0(OH)-Cer | 276 | 9,99E-001 | 38,31 | 0,62 | 51 |
| N-C24:0(OH)-Cer | 236 | 4,45E-001 | 32,03 | 0,71 | 1 |
| N-C26:0(OH)-Cer | 260 | 4,45E-001 | -14,17 | 0,69 | 45 |
| N-C8:0(OH)-Cer(2H) | 415 | 7,98E-001 | -11,72 | 0,56 | 32 |
| N-C10:0(OH)-Cer(2H) | 100 | 6,61E-002 | -74,99 | 0,82 | 47 |
| N-C25:0(OH)-Cer(2H) | 318 | 9,52E-001 | 20,16 | 0,65 | 7 |
| N-C26:0(OH)-Cer(2H) | 462 | 9.99E-001 | 2,38 | 0,58 | 34 |
| N-C27:0(OH)-Cer(2H) | 493 | 9,99E-001 | 8,35 | 0,52 | 44 |
| N-C28:0(OH)-Cer(2H) | 151 | 4,45E-001 | 28,55 | 0,82 | 2 |

### 2. Mouse

We use data of 11 (BL6) mice obtained from 5 animals with sepsis and induced liver failure and 6 mice with sepsis. Sepsis and organ failure were induced by intraperitoneal injection of an extract of human faeces. Typically 20 g of human stool (weight determined without further treatment) were homogenized in 40 ml of ice-cooled (4 C) sterile phosphate buffered saline (pH 7.4) using a Potter homogenizer or an Ultra Turrax, briefly centrifuged to remove bigger particles and the extract stored as frozen aliquots.

The effective dosis of the extract (to induce either sepsis or organ failure) has to be predetermined for each batch (of stool from one individual human subject). Depending of the dosage, sepsis can be induced within 24 h with a complete recovery of the animals > 48 h or septic organ failure can be induced by applying a higher dosage; for instance sepsis can be induced by injection of 0.5 ml of extract and organ failure by injection of 1.0 ml intraperitoneally. All samples of liver tissue were drawn 24 h after intraperitoneal injection of the extract.

### Statistical Analysis

All statistical calculations have been performed using the statistics software R (R: A Language and Environment for Statistical Computing, R Development Core Team, R Foundation for Statistical Computing,Vienna, Austria, 2009, ISBN 3-900051-07-0). Analytes that were detected in at least 15% of the samples were selected for further analyses resulting in a list of 218 unique compounds/metabolites (Table 1). The metabolic data is left censored due to thresholding of the mass spectrometer data resulting in non detected peak/signals. By a combination of metabolic pathway dynamism, complex sample molecular interaction and overall efficiency of the analytical protocol, replacement of missing data by means of a multivariate algorithm is preferred to a naive imputation by a pre-specified value like for instance zero. Hence, missing metabolite concentrations are replaced by the average value of the 6 closest samples to the one where the measurement is missing (impute: Imputation for microarray data, Hastie T., Tibshirani R., Narasimhan B. and Chu G., R package version 1.14.0). At the exception of fold change (FC) determination, all statistical analyses are performed on preprocessed - that is, log transformed - data.

The ImFit function in the package limma (Limma: linear models for microarray data, Smyth G.K. In: Bioinformatics and Computational Biology Solutions using R and Bioconductor, Springer, New York, pp 397-420, R package version 2.16.5) is used to compute the moderated statistics between measurements from septic patients samples and samples from patient developing organ failure. Resulting p values are adjusted by the method described in Benjamini and Hochberg (Benjamini Y. and Hochberg Y., Controlling the false discovery rate: a practical and powerful approach to multiple testing, Journal of the Royal Statistical Society Series B, 1995, 57, 289-300) leading to so-called q values.

Sensitivity/specificity properties of a classifier comprising one analyte or a combination of analytes are summarised in terms of Area Under the Receiver Operating Characteristic Curve (AUC). The function colAUC (caTools: Tools: moving window statistics, GIF, Base64, ROC AUC, etc., Tuszynski J., 2008, R package version 1.9) is used to compute and plot ROC curves. From the three univariate statistics (adjusted p value (q value), fold change and AUC), features are ranked according to a 2 step strategy: 1) the 3 measures are first used as input to the multiple objective algorithm described by Chen et al. (Chen J.J., Tsai C.-A., Tzeng S.-L.and Chen C.-H., Gene selection with multiple ordering criteria, BMC Bioinformatics 2007, 8:74) 2) ties (i.e. metabolites belonging to the same front) are broken according by simple Borda count. The function vennDiagram from the R package limma (Limma: linear models for microarray data, Smyth G.K. In: Bioinformatics and Computational Biology Solutions using R and Bioconductor, Springer, New York, pp 397-420, R package version 2.16.5) is employed to display the number of features selected by each ranking technique; confer Figure 4. Numbers in dark (resp. grey) express the count of metabolites that exhibit higher (resp. lower) concentration in the samples of those patients developing organ failure than in the septic patients samples. Following thresholds are used: adjusted p value (q-value) less than 0.05, absolute fold change higher than 50% and AUC greater than 0.8.

Due to the relatively small number of samples we performed no multivariate analyses avoiding overfitting.

We select the top 60 metabolites for the ranker combining adjusted p values, fold changes and AUCs; confer Table 3.

**Table 3 depicts the ranks of the individual analytes and metabolites in terms of discriminatory power for detecting the onset of infection associated organ failure. Ranking was performed using a univariate ranker which combines adjusted p values, fold changes and AUCs. For additional information see Fig 4.**

| **Name** | **Univariate rank** | **Adjusted p value** | **Fold change** | **AUC** |
|---|---|---|---|---|
| Putrescine | 1 | 6,75E-005 | 166,67 | 1,00 |
| Lanosterol | 2 | 3,50E-003 | -186,85 | 0,97 |
| C5-DC (C6-OH) | 3 | 3,39E-002 | 90,38 | 1,00 |
| 25OHC | 4 | 1,14E-003 | 122,16 | 0,87 |
| SM C16:1 | 5 | 9,74E-003 | 47,95 | 0,98 |
| 24SOHC | 6 | 2,07E-004 | 104,06 | 0,80 |
| C14 | 7 | 3,06E-003 | -163,87 | 0,63 |
| C4-OH (C3-DC) | 8 | 2,65E-002 | 129,92 | 0,93 |
| C0 | 9 | 2,17E-002 | 82,21 | 0,93 |
| C5-M-DC | 10 | 3,49E-002 | 71,15 | 0,98 |
| C6 (C4:1-DC) | 11 | 2,14E-001 | 134,29 | 0,75 |
| PC aa C38:4 | 12 | 6,03E-003 | 14,29 | 0,87 |
| GLCA | 13 | 6,57E-001 | -150,89 | 0,60 |
| Ala | 14 | 3,91E-001 | -144,80 | 0,50 |
| 4BOHC | 15 | 8,26E-002 | 59,11 | 0,93 |
| 24DHLan | 16 | 1,23E-001 | -51,66 | 0,93 |
| TLCA | 17 | 1,35E-001 | 87,93 | 0,87 |
| Serotonin | 18 | 1,48E-001 | 84,52 | 0,87 |
| ADMA | 19 | 7,50E-002 | -114,30 | 0,67 |
| PC aa C36:1 | 20 | 3,12E-003 | -20,78 | 0,53 |
| SM C16:0 | 21 | 3,52E-002 | 35,88 | 0,93 |
| C5:1-DC | 22 | 2,90E-001 | 88,46 | 0,83 |
| 7aOHC | 23 | 1,39E-001 | -26,38 | 0,93 |
| 27OHC | 24 | 3,87E-001 | -94,61 | 0,77 |
| Cit | 25 | 3,17E-001 | -126,99 | 0,50 |
| lysoPC a C20:4 | 26 | 2,90E-001 | 59,50 | 0,87 |
| GCA | 27 | 3,00E-001 | 98,25 | 0,67 |
| lysoPC a C16:0 | 28 | 1,59E-001 | 51,93 | 0,90 |
| Ile | 29 | 5,49E-002 | 42,99 | 0,87 |
| Desmosterol | 30 | 5,26E-002 | -68,61 | 0,80 |
| PEA | 31 | 5,06E-001 | -112,16 | 0,60 |
| total DMA | 32 | 2,50E-002 | -35,97 | 0,53 |
| Trp | 33 | 7,03E-002 | 28,10 | 0,90 |
| C3:1 | 34 | 8,68E-001 | 50,00 | 0,90 |
| IysoPC a C18:0 | 35 | 2,76E-001 | 50,86 | 0,87 |
| Val | 36 | 3,40E-001 | 38,05 | 0,90 |
| PC ae C38:0 | 37 | 6,05E-002 | -50,52 | 0,67 |
| PGF2a | 38 | 5,38E-001 | -96,77 | 0,60 |
| SM (OH) C14:1 | 39 | 2,68E-001 | 35,29 | 0,90 |
| lysoPC a C18:2 | 40 | 3,57E-001 | 39,10 | 0,87 |
| THC | 41 | 3,15E-001 | 26,62 | 0,90 |
| PC ae C40:4 | 42 | 1,17E-001 | 12,60 | 0,87 |
| 24,25,EPC | 43 | 1,71E-001 | -84,00 | 0,53 |
| PC ae C36:5 | 44 | 2,10E-001 | 24,65 | 0,90 |
| PGD2 | 45 | 4,49E-001 | 56,29 | 0,80 |
| Gly | 46 | 2,00E-001 | 45,29 | 0,83 |
| 5B,6B,EPC | 47 | 1,30E-001 | -16,12 | 0,80 |
| PC ae C40:0 | 48 | 9,41 E-002 | -24,60 | 0,67 |
| PC ae C36:1 | 49 | 1,21E-001 | -37,70 | 0,53 |
| C18 | 50 | 2,07E-001 | 44,24 | 0,73 |
| C16:2 | 51 | 4,96E-001 | 55,26 | 0,75 |
| PC aa C36:5 | 52 | 1,41 E-001 | -36,11 | 0,63 |
| PC aa C38:5 | 53 | 1,46E-001 | -27,05 | 0,67 |
| PC aa C30:2 | 54 | 5,91 E-001 | 57,78 | 0,73 |
| 13S-HODE | 55 | 5,25E-001 | -72,09 | 0,57 |
| C9 | 56 | 4,81 E-001 | 16,22 | 0,87 |
| 15S-HETE | 57 | 4,58E-001 | -66,46 | 0,53 |
| SM C22:3 | 58 | 1,80E-001 | -36,27 | 0,53 |
| C5:1 | 59 | 4,16E-001 | 32,69 | 0,83 |
| lysoPC a C17:0 | 60 | 6,28E-001 | 36,24 | 0,80 |

Table 4 shows the endogenous organ failure predictive targe metabolites as used in the present invention with their abbreviations and chemical names

**Table 4**

| **No. Name** | **Common Name** |
|---|---|
| 1 C0 | Carnitine (free) |
| 2 C10 | Decanoylcarnitine [Caprylcarnitine] (Fumarylcarnitine) |
| 3 C10:1 | Decenoylcarnitine |
| 4 C 10:2 | Decadienoylcarnitine |
| 5 C12 | Dodecanoylcarnitine [Laurylcarnitine] |
| 6 C12-DC | Dodecanedioylcarnitine |
| 7 C 12:1 | Dodecenoylcarnitine |
| 8 C14 | Tetradecanoylcarnitine |
| 9 C14:1 | Tetradecenoylcarnitine [Myristoleylcarnitine] |
| 10 C14:1-OH | 3-Hydroxytetradecenoylcarnitine [3-Hydroxymyristoleylcarnitine] |
| 11 C14:2 | Tetradecadienoylcarnitine |
| 12 C14:2-OH | 3-Hydroxytetradecadienoylcarnitine |
| 13 C16 | Hexadecanoylcarnitine [Pah-nitoylcamitine] |
| 14 C16-OH | 3-Hydroxyhexadecanolycarnitine [3-Hydroxypalmitoylcarnitine] |
| 15 C16:1 | Hexadecenoylcamitine [Palmitoleylcarnitine] |
| | 3-Hydroxyhexadecenoylcarnitine [3-Hydroxypalmitoleylcarnitine] |
| 16 C16:1-OH | |
| 17 C16:2 | Hexadecadienoylcarnitine |
| 18 C16:2-OH | 3-Hydroxyhexadecadienoylcarnitine |
| 19 C 18 | Octadecanoylcarnitine [Stearylcarnitine] |
| 20 C18:1 | Octadecenoylcarnitine [Oleylcarnitine] |
| 21 C18:1-OH | 3-Hydroxyoctadecenoylcarnitine [3-Hydroxyoleylcarnitine] |
| 22 C 18:2 | Octadecadienoylcarnitine [Linoleylcarnitine] |
| 23 C2 | Acetylcarnitine |
| 24 C3 | Propionylcarnitine |
| 25 C3-OH | Hydroxypropionylcarnitine |
| 26 C3:1 | Propenoylcarnitine |
| 27 C4 | Butyrylcarnitine / Isobutyrylcarnitine |
| 28 C4-OH (C3-DC) | 3-Hydroxybutyrylcarnitine / Malonylcarnitine |
| 29 C4:1 | Butenoylcarnitine |
| 30 C5 | Isovalerylcarnitine / 2-Methylbutyrylcarnitine / Valerylcarnitine |
| 31 C5-DC (C6-OH) | Glutarylcarnitine / Hydroxycaproylcarnitine |
| 32 C5-M-DC | Methylglutarylcarnitine |
| C5-OH (C3-DC- | |
| 33 M) | 3-Hydroxyisovalerylcarnitine / 3-Hydroxy-2-methylbutyryl |
| 34 C5:1 | Tiglylcarnitine / 3-Methyl-crotonylcarnitine |
| 35 C5:1-DC | Tiglylcarnitine / 3-Methyl-crotonylcarnitine |
| 36 C6 (C4:1-DC) | Hexanoylcarnitine [Caproylcarnitine] |
| 37 C6:1 | Hexenoylcarnitine |
| 38 C7-DC | Pimelylcarnitine |
| 39 C8 | Octanoylcarnitine [Caprylylcarnitine] |
| 40 C8:1 | Octenoylcarnitine |
| 41 C9 | Nonoylcarnitine [Pelargonylcarnitine] |
| 42 H1 | Hexose pool |
| 43 SM (OH) C14:1 | Sphingomyelin with acyl residue sum (OH) C14:1 |
| 44 SM (OH) C16:1 | Sphingomyelin with acyl residue sum (OH) C16:1 |
| 45 SM (OH) C22:1 | Sphingomyelin with acyl residue sum (OH) C22:1 |
| 46 SM (OH) C22:2 | Sphingomyelin with acyl residue sum (OH) C22:2 |
| 47 SM (OH) C24:1 | Sphingomyelin with acyl residue sum (OH) C24:1 |
| 48 SM C26:0 | Sphingomyelin with acyl residue sum C26:0 |
| 49 SM C26:1 | Sphingomyelin with acyl residue sum C26:1 |
| 50 PC aa C24:0 | Phosphatidylcholine with diacyl residue sum C24:0 |
| 51 PC aa C26:0 | Phosphatidylcholine with diacyl residue sum C26:0 |
| 52 PC aa C28:1 | Phosphatidylcholine with diacyl residue sum C28:1 |
| 53 PC aa C32:3 | Phosphatidylcholine with diacyl residue sum C32:3 |
| 54 PC aa C34:4 | Phosphatidylcholine with diacyl residue sum C34:4 |
| 55 PC aa C36:6 | Phosphatidylcholine with diacyl residue sum C36:6 |
| 56 PC aa C38:0 | Phosphatidylcholine with diacyl residue sum C38:0 |
| 57 PC aa C40:1 | Phosphatidylcholine with diacyl residue sum C40:1 |
| 58 PC aa C40:2 | Phosphatidylcholine with diacyl residue sum C40:2 |
| 59 PC aa C40:3 | Phosphatidylcholine with diacyl residue sum C40:3 |
| 60 PC aa C42:0 | Phosphatidylcholine with diacyl residue sum C42:0 |
| 61 PC aa C42:1 | Phosphatidylcholine with diacyl residue sum C42:1 |
| 62 PC aa C42:2 | Phosphatidylcholine with diacyl residue sum C42:2 |
| 63 PC aa C42:4 | Phosphatidylcholine with diacyl residue sum C42:4 |
| 64 PC aa C42:5 | Phosphatidylcholine with diacyl residue sum C42:5 |
| 65 PC aa C42:6 | Phosphatidylcholine with diacyl residue sum C42:6 |
| 66 PC ae C30:0 | Phosphatidylcholine with acyl-alkyl residue sum C30:0 |
| 67 PC ae C30:1 | Phosphatidylcholine with acyl-alkyl residue sum C30:1 |
| 68 PC ae C30:2 | Phosphatidylcholine with acyl-alkyl residue sum C30:2 |
| 69 PC ae C32:2 | Phosphatidylcholine with acyl-alkyl residue sum C32:2 |
| 70 PC ae C36:0 | Phosphatidylcholine with acyl-alkyl residue sum C36:0 |
| 71 PC ae C38:0 | Phosphatidylcholine with acyl-alkyl residue sum C38:0 |
| 72 PC ae C40:0 | Phosphatidylcholine with acyl-alkyl residue sum C40:0 |
| 73 PC ae C40:1 | Phosphatidylcholine with acyl-alkyl residue sum C40:1 |
| 74 PC ae C40:2 | Phosphatidylcholine with acyl-alkyl residue sum C40:2 |
| 75 PC ae C40:3 | Phosphatidylcholine with acyl-alkyl residue sum C40:3 |
| 76 PC ae C40:4 | Phosphatidylcholine with acyl-alkyl residue sum C40:4 |
| 77 PC ae C40:6 | Phosphatidylcholine with acyl-alkyl residue sum C40:6 |
| 78 PC ae C42:0 | Phosphatidylcholine with acyl-alkyl residue sum C42:0 |
| 79 PC ae C42:1 | Phosphatidylcholine with acyl-alkyl residue sum C42:1 |
| 80 PC ae C42:2 | Phosphatidylcholine with acyl-alkyl residue sum C42:2 |
| 81 PC ae C42:3 | Phosphatidylcholine with acyl-alkyl residue sum C42:3 |
| 82 PC ae C42:4 | Phosphatidylcholine with acyl-alkyl residue sum C42:4 |
| 83 PC ae C42:5 | Phosphatidylcholine with acyl-alkyl residue sum C42:5 |
| 84 PC ae C44:3 | Phosphatidylcholine with acyl-alkyl residue sum C44:3 |
| 85 PC ae C44:4 | Phosphatidylcholine with acyl-alkyl residue sum C44:4 |
| 86 PC ae C44:5 | Phosphatidylcholine with acyl-alkyl residue sum C44:5 |
| 87 PC ae C44:6 | Phosphatidylcholine with acyl-alkyl residue sum C44:6 |
| 88 lysoPC a C14:0 | Lysophosphatidylcholine with acyl residue sum C14:0 |
| 89 lysoPC a C16:1 | Lysophosphatidylcholine with acyl residue sum C16:1 |
| 90 lysoPC a C17:0 | Lysophosphatidylcholine with acyl residue sum C17:0 |
| 91 lysoPC a C20:3 | Lysophosphatidylcholine with acyl residue sum C20:3 |
| 92 lysoPC a C24:0 | Lysophosphatidylcholine with acyl residue sum C24:0 |
| 93 lysoPC a C26:0 | Lysophosphatidylcholine with acyl residue sum C26:0 |
| 94 lysoPC a C26:1 | Lysophosphatidylcholine with acyl residue sum C26:1 |
| 95 lysoPC a C28:0 | Lysophosphatidylcholine with acyl residue sum C28:0 |
| 96 lysoPC a C28:1 | Lysophosphatidylcholine with acyl residue sum C28:1 |
| 97 lysoPC a C6:0 | Lysophosphatidylcholine with acyl residue sum C6:0 |
| 98 Gly | Glycine |
| 99 Ala | Alanine |
| 100 Ser | Serine |
| 101 Pro | Proline |
| 102 Val | Valine |
| 103 Thr | Threonine |
| 104 Xle | Leucine + Isoleucine |
| 105 Leu | Leucine |
| 106 Ile | Isoleucine |
| 107 Asn | Asparagine |
| 108 Asp | Aspartate |
| 109 Gln | Glutamine |
| 110 Glu | Glutamate |
| 111 Met | Methionine |
| 112 His | Histidine |
| 113 Phe | Phenylalanine |
| 114 Arg | Arginine |
| 115 Cit | Citrulline |
| 116 Tyr | Tyrosine |
| 117 Trp | Tryptophan |
| 118 Orn | Ornithine |
| 119 Lys | Lysine |
| 120 ADMA | asymmetrical Dimethylarginin |
| 121 total DMA | Total dimethylarginine: sum ADMA + SDMA |
| 122 Met-SO | Methionine-Sulfoxide |
| 123 Kyn | Kynurenine |
| 124 Putrescine | Putrescine |
| 125 Spermidine | Spermidine |
| 126 Spermine | Spermine |
| 127 Creatinine | Creatinine |
| 128 9-HODE | (±)9-hydroxy-10E,12Z-octadecadienoic acid |
| 129 13S-HODE | 13(S)-hydroxy-9Z,11E-octadecadienoic acid |
| 130 12S-HETE | 12(S)-hydroxy-SZ,8Z,10E,14Z-eicosatetraenoic acid |
| 131 15S-HETE | 15(S)-hydroxy-5Z,8Z,11Z,13E-eicosatetraenoic acid |
| 132 LTB4 | Leukotriene B4 |
| 133 DHA | Docosahexaenoic acid |
| 134 PGE2 | Prostaglandin E2 |
| 135 PGD2 | Prostaglandin D2 |
| 136 AA | Arachidonic acid |
| 137 Lac | Lactate |
| 138 Suc | Succinic acid (succite) |
| 139 Hex | Hexose pool |
| 140 22ROHC | 22-R-Hydroxycholesterol |
| 14124SOHC | 24-S-Hydroxycholesterol |
| 142 250HC | 25-Hydroxycholesterol |
| 143 27OHC | 27-Hydroxycholesterol |
| 144 THC | 3β,5α,6β-Trihydroxycholestan |
| 145 7aOHC | 7α-Hydroxycholesterol |
| 146 7KC | 7-Ketocholesterol |
| 147 5a,6a,EPC | 5α,6α-Epoxycholesterol |
| 148 4BOHC | 4β-Hydroxycholesterol |
| 149 Desmosterol | Desmosterol |
| 150 7DHC | 7-Dehydrocholesterol (Vitamin D3) |
| 151 Lanosterol | Lanosterol |
| 152 PE a C16:0 | Lysophosphatidylethanolamine with acyl residue sum C16:0 |
| 153 PE a C18:0 | Lysophosphatidylethanolamine with acyl residue sum C18:0 |
| 154 PE a C18:1 | Lysophosphatidylethanolamine with acyl residue sum C 18:1 |
| 155 PE a C18:2 | Lysophosphatidylethanolamine with acyl residue sum C 18:2 |
| 156 PE a C20:4 | Lysophosphatidylethanolamine with acyl residue sum C20:4 |
| 157 PE a C22:4 | Lysophosphatidylethanolamine with acyl residue sum C22:4 |
| 158 PE a C22:5 | Lysophosphatidylethanolamine with acyl residue sum C22:5 |
| 159 PE a C22:6 | Lysophosphatidylethanolamine with acyl residue sum C22:6 |
| 160 PE e C18:0 | Lysophosphatidylethanolamine with alkyl residue sum C18:0 |
| 161 PG e C14:2 | Lysophosphatidylglycerol with alkyl residue sum C14:2 |
| 162 PE aa C20:0 | Phosphatidylethanolamine with diacyl residue sum C20:0 |
| 163 PE aa C22:2 | Phosphatidylethanolamine with diacyl residue sum C22:2 |
| 164 PE aa C26:4 | Phosphatidylethanolamine with diacyl residue sum C26:4 |
| 165 PE aa C28:4 | Phosphatidylethanolamine with diacyl residue sum C28:4 |
| 166 PE aa C28:5 | Phosphatidylethanolamine with diacyl residue sum C28:5 |
| 167 PE aa C34:0 | Phosphatidylethanolamine with diacyl residue sum C34:0 |
| 168 PE aa C34:1 | Phosphatidylethanolamine with diacyl residue sum C34:1 |
| 169 PE aa C34:2 | Phosphatidylethanolamine with diacyl residue sum C34:2 |
| 170 PE aa C34:3 | Phosphatidylethanolamine with diacyl residue sum C34:3 |
| 171 PE aa C36:0 | Phosphatidylethanolamine with diacyl residue sum C36:0 |
| 172 PE aa C36:1 | Phosphatidylethanolamine with diacyl residue sum C36:1 |
| 173 PE aa C36:2 | Phosphatidylethanolamine with diacyl residue sum C36:2 |
| 174 PE aa C36:3 | Phosphatidylethanolamine with diacyl residue sum C36:3 |
| 175 PE aa C36:4 | Phosphatidylethanolamine with diacyl residue sum C36:4 |
| 176 PE aa C36:5 | Phosphatidylethanolamine with diacyl residue sum C36:5 |
| 177 PE aa C38:0 | Phosphatidylethanolamine with diacyl residue sum C38:0 |
| 178 PE aa C38:1 | Phosphatidylethanolamine with diacyl residue sum C38:1 |
| 179 PE aa C38:2 | Phosphatidylethanolamine with diacyl residue sum C38:2 |
| 180 PE aa C38:3 | Phosphatidylethanolamine with diacyl residue sum C38:3 |
| 181 PE aa C38:4 | Phosphatidylethanolamine with diacyl residue sum C38:4 |
| 182 PE aa C38:5 | Phosphatidylethanolamine with diacyl residue sum C38:5 |
| 183 PE aa C38:6 | Phosphatidylethanolamine with diacyl residue sum C38:6 |
| 184 PE aa C38:7 | Phosphatidylethanolamine with diacyl residue sum C38:7 |
| 185 PE aa C40:2 | Phosphatidylethanolamine with diacyl residue sum C40:2 |
| 186 PE aa C40:3 | Phosphatidylethanolamine with diacyl residue sum C40:3 |
| 187 PE aa C40:4 | Phosphatidylethanolamine with diacyl residue sum C40:4 |
| 188 PE aa C40:5 | Phosphatidylethanolamine with diacyl residue sum C40:5 |
| 189 PE aa C40:6 | Phosphatidylethanolamine with diacyl residue sum C40:6 |
| 190 PE aa C40:7 | Phosphatidylethanolamine with diacyl residue sum C40:7 |
| 191 PE aa C48:1 | Phosphatidylethanolamine with diacyl residue sum C48:1 |
| 192 PE ae C34:1 | Phosphatidylethanolamine with acyl-alkyl residue sum C34:1 |
| 193 PE ae C34:2 | Phosphatidylethanolamine with acyl-alkyl residue sum C34:2 |
| 194 PE ae C34:3 | Phosphatidylethanolamine with acyl-alkyl residue sum C34:3 |
| 195 PE ae C36:1 | Phosphatidylethanolamine with acyl-alkyl residue sum C36:1 |
| 196 PE ae C36:2 | Phosphatidylethanolamine with acyl-alkyl residue sum C36:2 |
| 197 PE ae C36:3 | Phosphatidylethanolamine with acyl-alkyl residue sum C36:3 |
| 198 PE ae C36:4 | Phosphatidylethanolamine with acyl-alkyl residue sum C36:4 |
| 199 PE ae C36:5 | Phosphatidylethanolamine with acyl-alkyl residue sum C36:5 |
| 200 PE ae C38:1 | Phosphatidylethanolamine with acyl-alkyl residue sum C38:1 |
| 201 PE ae C38:2 | Phosphatidylethanolamine with acyl-alkyl residue sum C38:2 |
| 202 PE ae C38:3 | Phosphatidylethanolamine with acyl-alkyl residue sum C38:3 |
| 203 PE ae C38:4 | Phosphatidylethanolamine with acyl-alkyl residue sum C38:4 |
| 204 PE ae C38:5 | Phosphatidylethanolamine with acyl-alkyl residue sum C38:5 |
| 205 PE ae C38:6 | Phosphatidylethanolamine with acyl-alkyl residue sum C38:6 |
| 206 PE ae C40:1 | Phosphatidylethanolamine with acyl-alkyl residue sum C40:1 |
| 207 PE ae C40:2 | Phosphatidylethanolamine with acyl-alkyl residue sum C40:2 |
| 208 PE ae C40:3 | Phosphatidylethanolamine with acyl-alkyl residue sum C40:3 |
| 209 PE ae C40:4 | Phosphatidylethanolamine with acyl-alkyl residue sum C40:4 |
| 210 PE ae C40:5 | Phosphatidylethanolamine with acyl-alkyl residue sum C40:5 |
| 211 PE ae C40:6 | Phosphatidylethanolamine with acyl-alkyl residue sum C40:6 |
| 212 PE ae C42:1 | Phosphatidylethanolamine with acyl-alkyl residue sum C42:1 |
| 213 PE ae C42:2 | Phosphatidylethanolamine with acyl-alkyl residue sum C42:2 |
| 214 PE ae C46:5 | Phosphatidylethanolamine with acyl-alkyl residue sum C46:5 |
| 215 PE ae C46:6 | Phosphatidylethanolamine with acyl-alkyl residue sum C46:6 |
| 216 PG aa C30:0 | Phosphatidylglycerol with diacyl residue sum C30:0 |
| 217 PG aa C32:0 | Phosphatidylglycerol with diacyl residue sum C32:0 |
| 218 PG aa C32:1 | Phosphatidylglycerol with diacyl residue sum C32:1 |
| 219 PG aa C33:6 | Phosphatidylglycerol with diacyl residue sum C33:6 |
| 220 PG aa C34:0 | Phosphatidylglycerol with diacyl residue sum C34:0 |
| 221 PG aa C34:1 | Phosphatidylglycerol with diacyl residue sum C34:1 |
| 222 PG aa C34:2 | Phosphatidylglycerol with diacyl residue sum C34:2 |
| 223 PG aa C34:3 | Phosphatidylglycerol with diacyl residue sum C34:3 |
| 224 PG aa C36:0 | Phosphatidylglycerol with diacyl residue sum C36:0 |
| 225 PG aa C36:1 | Phosphatidylglycerol with diacyl residue sum C36:1 |
| 226 PG aa C36:2 | Phosphatidylglycerol with diacyl residue sum C36:2 |
| 227 PG aa C36:3 | Phosphatidylglycerol with diacyl residue sum C36:3 |
| 228 PG aa C36:4 | Phosphatidylglycerol with diacyl residue sum C36:4 |
| 229 PG aa C38:5 | Phosphatidylglycerol with diacyl residue sum C38:5 |
| 230 PG ae C32:0 | Phosphatidylglycerol with acyl-alkyl residue sum C32:0 |
| 231 PG ae C34:0 | Phosphatidylglycerol with acyl-alkyl residue sum C34:0 |
| 232 PG ae C34:1 | Phosphatidylglycerol with acyl-alkyl residue sum C34:1 |
| 233 PG ae C36:1 | Phosphatidylglycerol with acyl-alkyl residue sum C36:1 |
| 234 PS aa C34:1 | Phosphatidylserine with diacyl residue sum C34:1 |
| 235 PS aa C34:2 | Phosphatidylserine with diacyl residue sum C34:2 |
| 236 PS aa C36:0 | Phosphatidylserine with diacyl residue sum C36:0 |
| 237 PS aa C36:1 | Phosphatidylserine with diacyl residue sum C36:1 |
| 238 PS aa C36:2 | Phosphatidylserine with diacyl residue sum C36:2 |
| 239 PS aa C36:3 | Phosphatidylserine with diacyl residue sum C36:3 |
| 240 PS aa C36:4 | Phosphatidylserine with diacyl residue sum C36:4 |
| 241 PS aa C38:1 | Phosphatidylserine with diacyl residue sum C38:1 |
| 242 PS aa C38:2 | Phosphatidylserine with diacyl residue sum C38:2 |
| 243 PS aa C38:3 | Phosphatidylserine with diacyl residue sum C38:3 |
| 244 PS aa C38:4 | Phosphatidylserine with diacyl residue sum C38:4 |
| 245 PS aa C38:5 | Phosphatidylserine with diacyl residue sum C38:5 |
| 246 PS aa C40:1 | Phosphatidylserine with diacyl residue sum C40:1 |
| 247 PS aa C40:2 | Phosphatidylserine with diacyl residue sum C40:2 |
| 248 PS aa C40:3 | Phosphatidylserine with diacyl residue sum C40:3 |
| 249 PS aa C40:4 | Phosphatidylserine with diacyl residue sum C40:4 |
| 250 PS aa C40:5 | Phosphatidylserine with diacyl residue sum C40:5 |
| 251 PS aa C40:6 | Phosphatidylserine with diacyl residue sum C40:6 |
| 252 PS aa C40:7 | Phosphatidylserine with diacyl residue sum C40:7 |
| 253 PS aa C42:1 | Phosphatidylserine with diacyl residue sum C42:1 |
| 254 PS aa C42:2 | Phosphatidylserine with diacyl residue sum C42:2 |
| 255 PS aa C42:4 | Phosphatidylserine with diacyl residue sum C42:4 |
| 256 PS aa C42:5 | Phosphatidylserine with diacyl residue sum C42:5 |
| 257 PS ae C34:2 | Phosphatidylserine with acyl-alkyl residue sum C34:2 |
| 258 PS ae C36:1 | Phosphatidylserine with acyl-alkyl residue sum C36:1 |
| 259 PS ae C36:2 | Phosphatidylserine with acyl-alkyl residue sum C36:2 |
| 260 PS ae C38:4 | Phosphatidylserine with acyl-alkyl residue sum C38:4 |
| 261 SM C 4:0 | Sphingomyelin with acyl residue sum C14:0 |
| 262 SM C16:0 | Sphingomyelin with acyl residue sum C16:0 |
| 263 SM C16:1 | Sphingomyelin with acyl residue sum C16:1 |
| 264 SM C 17:0 | Sphingomyelin with acyl residue sum C 17:0 |
| 265 SM C18:0 | Sphingomyelin with acyl residue sum C18:0 |
| 266 SM C 18:1 | Sphingomyelin with acyl residue sum C18:1 |
| 267 SM C 19:0 | Sphingomyelin with acyl residue sum C19:0 |
| 268 SM C 19:1 | Sphingomyelin with acyl residue sum C 19:1 |
| 269 SM C19:2 | Sphingomyelin with acyl residue sum C19:2 |
| 270 SM C20:0 | Sphingomyelin with acyl residue sum C20:0 |
| 271 SM C20:1 | Sphingomyelin with acyl residue sum C20:1 |
| 272 SM C20:2 | Sphingomyelin with acyl residue sum C20:2 |
| 273 SM C21:0 | Sphingomyelin with acyl residue sum C21:0 |
| 274 SM C21:1 | Sphingomyelin with acyl residue sum C21:1 |
| 275 SM C21:2 | Sphingomyelin with acyl residue sum C21:2 |
| 276 SM C21:3 | Sphingomyelin with acyl residue sum C21:3 |
| 277 SM C22:0 | Sphingomyelin with acyl residue sum C22:0 |
| 278 SM C22:1 | Sphingomyelin with acyl residue sum C22:1 |
| 279 SM C22:2 | Sphingomyelin with acyl residue sum C22:2 |
| 280 SM C22:3 | Sphingomyelin with acyl residue sum C22:3 |
| 281 SM C23:0 | Sphingomyelin with acyl residue sum C23:0 |
| 282 SM C23:1 | Sphingomyelin with acyl residue sum C23:1 |
| 283 SM C23:2 | Sphingomyelin with acyl residue sum C23:2 |
| 284 SM C23:3 | Sphingomyelin with acyl residue sum C23:3 |
| 285 SM C24:0 | Sphingomyelin with acyl residue sum C24:0 |
| 286 SM C24:1 | Sphingomyelin with acyl residue sum C24:1 |
| 287 SM C24:2 | Sphingomyelin with acyl residue sum C24:2 |
| 288 SM C24:3 | Sphingomyelin with acyl residue sum C24:3 |
| 289 SM C24:4 | Sphingomyelin with acyl residue sum C24:4 |
| 290 SM C26:3 | Sphingomyelin with acyl residue sum C26:3 |
| 291 SM C26:4 | Sphingomyelin with acyl residue sum C26:4 |
| 292 SM C3:0 | Sphingomyelin with acyl residue sum C3:0 |
| 293 lysoPC a C16:0 | Lysophosphatidylcholine with acyl residue sum C16:0 |
| 294 lysoPC a C18:0 | Lysophosphatidylcholine with acyl residue sum C18:0 |
| 295 lysoPC a C18:1 | Lysophosphatidylcholine with acyl residue sum C18:1 |
| 296 lysoPC a C 18:2 | Lysophosphatidylcholine with acyl residue sum C 18:2 |
| 297 lysoPC a C20:4 | Lysophosphatidylcholine with acyl residue sum C20:4 |
| 298 PC e C18:0 | Lysophosphatidylcholine with alkyl residue sum C18:0 |
| 299 PC aa C30:0 | Phosphatidylcholine with diacyl residue sum C30:0 |
| 300 PC aa C30:1 | Phosphatidylcholine with diacyl residue sum C30:1 |
| 301 PC aa C30:2 | Phosphatidylcholine with diacyl residue sum C30:2 |
| 302 PC aa C32:0 | Phosphatidylcholine with diacyl residue sum C32:0 |
| 303 PC aa C32:1 | Phosphatidylcholine with diacyl residue sum C32:1 |
| 304 PC aa C32:2 | Phosphatidylcholine with diacyl residue sum C32:2 |
| 305 PC aa C34:0 | Phosphatidylcholine with diacyl residue sum C34:0 |
| 306 PC aa C34:1 | Phosphatidylcholine with diacyl residue sum C34:1 |
| 307 PC aa C34:2 | Phosphatidylcholine with diacyl residue sum C34:2 |
| 308 PC aa C34:3 | Phosphatidylcholine with diacyl residue sum C34:3 |
| 309 PC aa C36:0 | Phosphatidylcholine with diacyl residue sum C36:0 |
| 310 PC aa C36:1 | Phosphatidylcholine with diacyl residue sum C36:1 |
| 311 PC aa C36:2 | Phosphatidylcholine with diacyl residue sum C36:2 |
| 312 PC aa C36:3 | Phosphatidylcholine with diacyl residue sum C36:3 |
| 313 PC aa C36:4 | Phosphatidylcholine with diacyl residue sum C36:4 |
| 314 PC aa C36:5 | Phosphatidylcholine with diacyl residue sum C36:5 |
| 315 PC aa C38:1 | Phosphatidylcholine with diacyl residue sum C38:1 |
| 316 PC aa C38:2 | Phosphatidylcholine with diacyl residue sum C38:2 |
| 317 PC aa C38:3 | Phosphatidylcholine with diacyl residue sum C38:3 |
| 318 PC aa C38:4 | Phosphatidylcholine with diacyl residue sum C38:4 |
| 319 PC aa C38:5 | Phosphatidylcholine with diacyl residue sum C38:5 |
| 320 PC aa C38:6 | Phosphatidylcholine with diacyl residue sum C38:6 |
| 321 PC aa C40:4 | Phosphatidylcholine with diacyl residue sum C40:4 |
| 322 PC aa C40:5 | Phosphatidylcholine with diacyl residue sum C40:5 |
| 323 PC aa C40:6 | Phosphatidylcholine with diacyl residue sum C40:6 |
| 324 PC aa C40:7 | Phosphatidylcholine with diacyl residue sum C40:7 |
| 325 PC aa C40:8 | Phosphatidylcholine with diacyl residue sum C40:8 |
| 326 PC ae C32:0 | Phosphatidylcholine with acyl-alkyl residue sum C32:0 |
| 327 PC ae C32:1 | Phosphatidylcholine with acyl-alkyl residue sum C32:1 |
| 328 PC ae C32:6 | Phosphatidylcholine with acyl-alkyl residue sum C32:6 |
| 329 PC ae C34:0 | Phosphatidylcholine with acyl-alkyl residue sum C34:0 |
| 330 PC ae C34:1 | Phosphatidylcholine with acyl-alkyl residue sum C34:1 |
| 331 PC ae C34:2 | Phosphatidylcholine with acyl-alkyl residue sum C34:2 |
| 332 PC ae C34:3 | Phosphatidylcholine with acyl-alkyl residue sum C34:3 |
| 333 PC ae C34:6 | Phosphatidylcholine with acyl-alkyl residue sum C34:6 |
| 334 PC ae C36:1 | Phosphatidylcholine with acyl-alkyl residue sum C36:1 |
| 335 PC ae C36:2 | Phosphatidylcholine with acyl-alkyl residue sum C36:2 |
| 336 PC ae C36:3 | Phosphatidylcholine with acyl-alkyl residue sum C36:3 |
| 337 PC ae C36:4 | Phosphatidylcholine with acyl-alkyl residue sum C36:4 |
| 338 PC ae C36:5 | Phosphatidylcholine with acyl-alkyl residue sum C36:5 |
| 339 PC ae C38:1 | Phosphatidylcholine with acyl-alkyl residue sum C38:1 |
| 340 PC ae C38:2 | Phosphatidylcholine with acyl-alkyl residue sum C38:2 |
| 341 PC ae C38:3 | Phosphatidylcholine with acyl-alkyl residue sum C38:3 |
| 342 PC ae C38:4 | Phosphatidylcholine with acyl-alkyl residue sum C38:4 |
| 343 PC ae C38:5 | Phosphatidylcholine with acyl-alkyl residue sum C38:5 |
| 344 PC ae C38:6 | Phosphatidylcholine with acyl-alkyl residue sum C38:6 |
| 345 PC ae C40:5 | Phosphatidylcholine with acyl-alkyl residue sum C40:5 |
| | Ceramide: chain length and number of double bonds is |
| 346 N-C2:0-Cer | determined by the measured mass C2:0 |
| | Ceramide: chain length and number of double bonds is |
| 347 N-C3:1-Cer | determined by the measured mass C3:1 |
| | Ceramide: chain length and number of double bonds is |
| 348 N-C3:0-Cerr | determined by the measured mass C3:0 |
| | Ceramide: chain length and number of double bonds is |
| 349 N-C4:1-Cer | determined by the measured mass C4:1 |
| | Ceramide: chain length and number of double bonds is |
| 350 N-C4:0-Cer | determined by the measured mass C4:0 |
| | Ceramide: chain length and number of double bonds is |
| 351 N-C5:1-Cer | determined by the measured mass C5:1 |
| | Ceramide: chain length and number of double bonds is |
| 352 N-C5:0-Cer | determined by the measured mass C5:0 |
| | Ceramide: chain length and number of double bonds is |
| 353 N-C6:1-Cer | determined by the measured mass C6:1 |
| | Ceramide: chain length and number of double bonds is |
| 354 N-C6:0-Cer | determined by the measured mass C6:0 |
| | Ceramide: chain length and number of double bonds is |
| 355 N-C7:1-Cer | determined by the measured mass C7:1 |
| | Ceramide: chain length and number of double bonds is |
| 356 N-C7:0-Cer | determined by the measured mass C7:0 |
| | Ceramide: chain length and number of double bonds is |
| 357 N-C8:1-Cer | determined by the measured mass C8:1 |
| | Ceramide: chain length and number of double bonds is |
| 358 N-C8:0-Cer | determined by the measured mass C8:0 |
| | Ceramide: chain length and number of double bonds is |
| 359 N-C9:3-Cer | determined by the measured mass C9:3 |
| | Ceramide: chain length and number of double bonds is |
| 360 N-C9:1-Cer | determined by the measured mass C9:1 |
| | Ceramide: chain length and number of double bonds is |
| 361 N-C9:0-Cer | determined by the measured mass C9:0 |
| | Ceramide: chain length and number of double bonds is |
| 362 N-C10:1-Cer | determined by the measured mass C10:1 |
| | Ceramide: chain length and number of double bonds is |
| 363 N-C10:0-Cer | determined by the measured mass C10:0 |
| | Ceramide: chain length and number of double bonds is |
| 3 64 N-C 11:1-Cer | determined by the measured mass C 11:1 |
| | Ceramide: chain length and number of double bonds is |
| 365 N-C11:0-Cer | determined by the measured mass C11:0 |
| | Ceramide: chain length and number of double bonds is |
| 366 N-C12:1-Cer | determined by the measured mass C12:1 |
| | Ceramide: chain length and number of double bonds is |
| 367 N-C12:0-Cer | determined by the measured mass C12:0 |
| | Ceramide: chain length and number of double bonds is |
| 368 N-(OH)C11:0-Cer | determined by the measured mass (OH)C11:0 |
| | Ceramide: chain length and number of double bonds is |
| 369 N-C13:1-Cer | determined by the measured mass C13:1 |
| | Ceramide: chain length and number of double bonds is |
| 3 70 N-C13:0-Cer | determined by the measured mass C13:0 |
| | Ceramide: chain length and number of double bonds is |
| 3 71 N-C14:1-Cer | determined by the measured mass C14:1 |
| | Ceramide: chain length and number of double bonds is |
| 372 N-C14:0-Cer | determined by the measured mass C14:0 |
| | Ceramide: chain length and number of double bonds is |
| 373 N-C15:1-Cer | determined by the measured mass C15:1 |
| | Ceramide: chain length and number of double bonds is |
| 374 N-C15:0-Cer | determined by the measured mass C 15:0 |
| | Ceramide: chain length and number of double bonds is |
| 375 N-C16:1-Cer | determined by the measured mass C16:1 |
| | Ceramide: chain length and number of double bonds is |
| 376 N-C16:0-Cer | determined by the measured mass C16:0 |
| | Ceramide: chain length and number of double bonds is |
| 377 N-C17:1-Cer | determined by the measured mass C17:1 |
| | Ceramide: chain length and number of double bonds is |
| 378 N-C17:0-Cer | determined by the measured mass C17:0 |
| N-(2xOH)C15:0- | Ceramide: chain length and number of double bonds is |
| 379 Cer | determined by the measured mass (2xOH)C15:0 |
| | Ceramide: chain length and number of double bonds is |
| 380 N-C18:1-Cer | determined by the measured mass C18:1 |
| | Ceramide: chain length and number of double bonds is |
| 381 N-C 18:0-Cer | determined by the measured mass C18:0 |
| | Ceramide: chain length and number of double bonds is |
| 3 82 N-C19:1-Cer | determined by the measured mass C19:1 |
| | Ceramide: chain length and number of double bonds is |
| 383 N-C19:0-Cer | determined by the measured mass C19:0 |
| | Ceramide: chain length and number of double bonds is |
| 384 N-C20:1-Cer | determined by the measured mass C20:1 |
| | Ceramide: chain length and number of double bonds is |
| 385 N-C20:0-Cer | determined by the measured mass C20:0 |
| | Ceramide: chain length and number of double bonds is |
| 386 N-C21:1-Cer | determined by the measured mass C21:1 |
| | Ceramide: chain length and number of double bonds is |
| 387 N-C21:0-Cer | determined by the measured mass C21:0 |
| | Ceramide: chain length and number of double bonds is |
| 388 N-C22:1-Cer | determined by the measured mass C22:1 |
| | Ceramide: chain length and number of double bonds is |
| 389 N-C22:0-Cer | determined by the measured mass C22:0 |
| | Ceramide: chain length and number of double bonds is |
| 3 90 N-C23:1-Cer | determined by the measured mass C23:1 |
| | Ceramide: chain length and number of double bonds is |
| 391 N-C23:0-Cer | determined by the measured mass C23:0 |
| | Ceramide: chain length and number of double bonds is |
| 392 N-C24:1-Cer | determined by the measured mass C24:1 |
| 393 N-C24:0-Cer | Ceramide: chain length and number of double bonds is |
| | determined by the measured mass C24:0 |
| | Ceramide: chain length and number of double bonds is |
| 3 94 N-C25:1-Cer | determined by the measured mass C25:1 |
| | Ceramide: chain length and number of double bonds is |
| 395 N-C25:0-Cer | determined by the measured mass C25:0 |
| | Ceramide: chain length and number of double bonds is |
| 3 96 N-C26:1-Cer | determined by the measured mass C26:1 |
| | Ceramide: chain length and number of double bonds is |
| 397 N-C26:0-Cer | determined by the measured mass C26:0 |
| | Ceramide: chain length and number of double bonds is |
| 398 N-C27:1-Cer | determined by the measured mass C27:1 |
| | Ceramide: chain length and number of double bonds is |
| 399 N-C27:0-Cer | determined by the measured mass C27:0 |
| | Ceramide: chain length and number of double bonds is |
| 400 N-C28:1-Cer | determined by the measured mass C28:1 |
| | Ceramide: chain length and number of double bonds is |
| 401 N-C28:0-Cer | determined by the measured mass C28:0 |
| | Dihydroceramide: chain length and number of double bonds is |
| 402 N-C2:0-Cer(2H) | determined by the measured mass C2:0 |
| | Dihydroceramide: chain length and number of double bonds is |
| 403 N-C3:1-Cer(2H) | determined by the measured mass C3:1 |
| | Dihydroceramide: chain length and number of double bonds is |
| 404 N-C3:0-Cer(2H) | determined by the measured mass C3:0 |
| | Dihydroceramide: chain length and number of double bonds is |
| 405 N-C4:1-Cer(2H) | determined by the measured mass C4:1 |
| | Dihydroceramide: chain length and number of double bonds is |
| 406 N-C4:0-Cer(2H) | determined by the measured mass C4:0 |
| | Dihydroceramide: chain length and number of double bonds is |
| 407 N-C5:1-Cer(2H) | determined by the measured mass C5:1 |
| | Dihydroceramide: chain length and number of double bonds is |
| 408 N-C5:0-Cer(2H) | determined by the measured mass C5:0 |
| | Dihydroceramide: chain length and number of double bonds is |
| 409 N-C6:1-Cer(2H) | determined by the measured mass C6:1 |
| | Dihydroceramide: chain length and number of double bonds is |
| 410 N-C6:0-Cer(2H) | determined by the measured mass C6:0 |
| | Dihydroceramide: chain length and number of double bonds is |
| 411 N-C7:1-Cer(2H) | determined by the measured mass C7:1 |
| | Dihydroceramide: chain length and number of double bonds is |
| 412 N-C7:0-Cer(2H) | determined by the measured mass C7:0 |
| | Dihydroceramide: chain length and number of double bonds is |
| 413 N-C8:1-Cer(2H) | determined by the measured mass C8:1 |
| | Dihydroceramide: chain length and number of double bonds is |
| 414 N-C8:0-Cer(2H) | determined by the measured mass C8:0 |
| | Dihydroceramide: chain length and number of double bonds is |
| 415 N-C9:1-Cer(2H) | determined by the measured mass C9:1 |
| N-C9:0-Cer(2H) | Dihydroceramide: chain length and number of double bonds is |
| 416 Q3+NL cor | determined by the measured mass C9:0 |
| | Dihydroceramide: chain length and number of double bonds is |
| 417 N-C10:1-Cer(2H) | determined by the measured mass C10:1 |
| | Dihydroceramide: chain length and number of double bonds is |
| 418 N-C 10:0-Cer(2H) | determined by the measured mass C10:0 |
| | Dihydroceramide: chain length and number of double bonds is |
| 419 N-C 11:1-Cer(2H) | determined by the measured mass C11:1 |
| | Dihydroceramide: chain length and number of double bonds is |
| 420 N-C11:0-Cer(2H) | determined by the measured mass C11:0 |
| | Dihydroceramide: chain length and number of double bonds is |
| 421 N-C12:1-Cer(2H) | determined by the measured mass C12:1 |
| | Dihydroceramide: chain length and number of double bonds is |
| 422 N-C12:0-Cer(2H) | determined by the measured mass C12:0 |
| | Dihydroceramide: chain length and number of double bonds is |
| 423 N-C 13:1-Cer(2H) | determined by the measured mass C 13:1 |
| | Dihydroceramide: chain length and number of double bonds is |
| 424 N-C13:0-Cer(2H) | determined by the measured mass C13:0 |
| | Dihydroceramide: chain length and number of double bonds is |
| 425 N-C 14:1-Cer(2H) | determined by the measured mass C14:1 |
| | Dihydroceramide: chain length and number of double bonds is |
| 426 N-C14:0-Cer(2H) | determined by the measured mass C14:0 |
| | Dihydroceramide: chain length and number of double bonds is |
| 427 N-C 15:1-Cer(2H) | determined by the measured mass C15:1 |
| | Dihydroceramide: chain length and number of double bonds is |
| 428 N-C15:0-Cer(2H) | determined by the measured mass C 15:0 |
| | Dihydroceramide: chain length and number of double bonds is |
| 429 N-C16:1-Cer(2H) | determined by the measured mass C16:1 |
| | Dihydroceramide: chain length and number of double bonds is |
| 43 0 N-C16:0-Cer(2H) | determined by the measured mass C16:0 |
| | Dihydroceramide: chain length and number of double bonds is |
| 431 N-C 17:1-Cer(2H) | determined by the measured mass C17:1 |
| | Dihydroceramide: chain length and number of double bonds is |
| 432 N-C17:0-Cer(2H) | determined by the measured mass C17:0 |
| | Dihydroceramide: chain length and number of double bonds is |
| 433 N-C18:1-Cer(2H) | determined by the measured mass C18:1 |
| | Dihydroceramide: chain length and number of double bonds is |
| 434 N-C18:0-Cer(2H) | determined by the measured mass C 18:0 |
| | Dihydroceramide: chain length and number of double bonds is |
| 435 N-C19:1-Cer(2H) | determined by the measured mass C19:1 |
| | Dihydroceramide: chain length and number of double bonds is |
| 436 N-C19:0-Cer(2H) | determined by the measured mass C19:0 |
| | Dihydroceramide: chain length and number of double bonds is |
| 437 N-C18:0-Cer(2H) | determined by the measured mass C18:0 |
| | Dihydroceramide: chain length and number of double bonds is |
| 438 N-C20:0-Cer(2H) | determined by the measured mass C20:0 |
| | Dihydroceramide: chain length and number of double bonds is |
| 439 N-C21:1-Cer(2H) | determined by the measured mass C21:1 |
| | Dihydroceramide: chain length and number of double bonds is |
| 440 N-C21:0-Cer(2H) | determined by the measured mass C21:0 |
| | Dihydroceramide: chain length and number of double bonds is |
| 441 N-C22:1-Cer(2H) | determined by the measured mass C22:1 |
| 442 N-C22:0-Cer(2H) | Dihydroceramide: chain length and number of double bonds is |
| | determined by the measured mass C22:0 |
| | Dihydroceramide: chain length and number of double bonds is |
| 443 N-C23:1-Cer(2H) | determined by the measured mass C23:1 |
| | Dihydroceramide: chain length and number of double bonds is |
| 444 N-C23:0-Cer(2H) | determined by the measured mass C23:0 |
| | Dihydroceramide: chain length and number of double bonds is |
| 445 N-C24:1-Cer(2H) | determined by the measured mass C24:1 |
| | Dihydroceramide: chain length and number of double bonds is |
| 446 N-C24:0-Cer(2H) | determined by the measured mass C24:0 |
| | Dihydroceramide: chain length and number of double bonds is |
| 447 N-C25:1-Cer(2H) | determined by the measured mass C25:1 |
| | Dihydroceramide: chain length and number of double bonds is |
| 448 N-C25:0-Cer(2H) | determined by the measured mass C25:0 |
| | Dihydroceramide: chain length and number of double bonds is |
| 449 N-C26:1-Cer(2H) | determined by the measured mass C26:1 |
| | Dihydroceramide: chain length and number of double bonds is |
| 450 N-C26:0-Cer(2H) | determined by the measured mass C26:0 |
| | Dihydroceramide: chain length and number of double bonds is |
| 451 N-C27:1-Cer(2H) | determined by the measured mass C27:1 |
| | Dihydroceramide: chain length and number of double bonds is |
| 452 N-C27:0-Cer(2H) | determined by the measured mass C27:0 |
| | Dihydroceramide: chain length and number of double bonds is |
| 453 N-C28:1-Cer(2H) | determined by the measured mass C28:1 |
| | Dihydroceramide: chain length and number of double bonds is |
| 454 N-C28:0-Cer(2H) | determined by the measured mass C28:0 |
| | Ceramide: chain length and number of double bonds is |
| 455 N-C3:0(OH)-Cer | determined by the measured mass C3:0(OH) |
| | Ceramide: chain length and number of double bonds is |
| 456 N-C4:0(OH)-Cer | determined by the measured mass C4:0(OH) |
| | Ceramide: chain length and number of double bonds is |
| 457 N-(2xOH)C3:0-Cer | determined by the measured mass (2xOH)C3:0 |
| | Ceramide: chain length and number of double bonds is |
| 458 N-C5:0(OH)-Cer | determined by the measured mass C5:0(OH) |
| | Ceramide: chain length and number of double bonds is |
| 459 N-C6:0(OH)-Cer | determined by the measured mass C6:0(OH) |
| | Ceramide: chain length and number of double bonds is |
| 460 N-C7:2(OH)-Cer | determined by the measured mass C7:2(OH) |
| | Ceramide: chain length and number of double bonds is |
| 461 N-C7:1(OH)-Cer | determined by the measured mass C7:1 (OH) |
| | Ceramide: chain length and number of double bonds is |
| 462 N-C7:0(OH)-Cer | determined by the measured mass C7:0(OH) |
| | Ceramide: chain length and number of double bonds is |
| 463 N-C8:0(OH)-Cer | determined by the measured mass C8:0(OH) |
| | Ceramide: chain length and number of double bonds is |
| 464 N-C9:0(OH)-Cer | determined by the measured mass C9:0(OH) |
| | Ceramide: chain length and number of double bonds is |
| 465 N-C10:0(OH)-Cer | determined by the measured mass C10:0(OH) |
| | Ceramide: chain length and number of double bonds is |
| 466 N-C11:1(OH)-Cer | determined by the measured mass C11:1(OH) |
| | Ceramide: chain length and number of double bonds is |
| 467 N-C11:0(OH)-Cer | determined by the measured mass C11:0(OH) |
| | Ceramide: chain length and number of double bonds is |
| 468 N-C12:0(OH)-Cer | determined by the measured mass C12:0(OH) |
| | Ceramide: chain length and number of double bonds is |
| 469 N-C 13:0(OH)-Cer | determined by the measured mass C 13:0(OH) |
| | Ceramide: chain length and number of double bonds is |
| 470 N-C14:0(OH)-Cer | determined by the measured mass C14:0(OH) |
| | Ceramide: chain length and number of double bonds is |
| 471 N-C 15:0(OH)-Cer | determined by the measured mass C 15:0(OH) |
| | Ceramide: chain length and number of double bonds is |
| 472 N-C16:0(OH)-Cer | determined by the measured mass C16:0(OH) |
| | Ceramide: chain length and number of double bonds is |
| 473 N-C17:1(OH)-Cer | determined by the measured mass C17:1(OH) |
| | Ceramide: chain length and number of double bonds is |
| 474 N-C17:0(OH)-Cer | determined by the measured mass C 17:0(OH) |
| | Ceramide: chain length and number of double bonds is |
| 475 N-C18:0(OH)-Cer | determined by the measured mass C18:0(OH) |
| | Ceramide: chain length and number of double bonds is |
| 476 N-C19:0(OH)-Cer | determined by the measured mass C19:0(OH) |
| | Ceramide: chain length and number of double bonds is |
| 477 N-C20:0(OH)-Cer | determined by the measured mass C20:0(OH) |
| N-C19:0(2xOH)- | Ceramide: chain length and number of double bonds is |
| 478 Cer | determined by the measured mass C19:0(2xOH) |
| | Ceramide: chain length and number of double bonds is |
| 479 N-C21:0(OH)-Cer | determined by the measured mass C21:0(OH) |
| | Ceramide: chain length and number of double bonds is |
| 480 N-C22:0(OH)-Cer | determined by the measured mass C22:0(OH) |
| | Ceramide: chain length and number of double bonds is |
| 481 N-C23:0(OH)-Cer | determined by the measured mass C23:0(OH) |
| | Ceramide: chain length and number of double bonds is |
| 482 N-C24:0(OH)-Cer | determined by the measured mass C24:0(OH) |
| N-C23:0(2xOH)- | Ceramide: chain length and number of double bonds is |
| 483 Cer | determined by the measured mass C23:0(2xOH) |
| | Ceramide: chain length and number of double bonds is |
| 484 N-C25:0(OH)-Cer | determined by the measured mass C25:0(OH) |
| | Ceramide: chain length and number of double bonds is |
| 485 N-C26:1(OH)-Cer | determined by the measured mass C26:1(OH) |
| | Ceramide: chain length and number of double bonds is |
| 486 N-C26:0(OH)-Cer | determined by the measured mass C26:0(OH) |
| | Ceramide: chain length and number of double bonds is |
| 487 N-C27:0(OH)-Cer | determined by the measured mass C27:0(OH) |
| | Ceramide: chain length and number of double bonds is |
| 488 N-C28:0(OH)-Cer | determined by the measured mass C28:0(OH) |
| N-C3:0(OH)- | Dihydroceramide: chain length and number of double bonds is |
| 489 Cer(2H) | determined by the measured mass C3:0(OH) |
| N-C4:0(OH)- | Dihydroceramide: chain length and number of double bonds is |
| 490 Cer(2H) | determined by the measured mass C4:0(OH) |
| 491 N-C5:0(OH)- | Dihydroceramide: chain length and number of double bonds is |
| Cer(2H) | determined by the measured mass C5:0(OH) |
| N-C6:0(OH)- | Dihydroceramide: chain length and number of double bonds is |
| 492 Cer(2H) | determined by the measured mass C6:0(OH) |
| N-C7:0(OH)- | Dihydroceramide: chain length and number of double bonds is |
| 493 Cer(2H) | determined by the measured mass C7:0(OH) |
| N-C8:0(OH)- | Dihydroceramide: chain length and number of double bonds is |
| 494 Cer(2H) | determined by the measured mass C8:0(OH) |
| N-C9:0(OH)- | Dihydroceramide: chain length and number of double bonds is |
| 495 Cer(2H) | determined by the measured mass C9:0(OH) |
| N-C 10:0(OH)- | Dihydroceramide: chain length and number of double bonds is |
| 496 Cer(2H) | determined by the measured mass C10:0(OH) |
| N-C11:0(OH)- | Dihydroceramide: chain length and number of double bonds is |
| 497 Cer(2H) | determined by the measured mass C11:0(OH) |
| N-C 13:0(OH)- | Dihydroceramide: chain length and number of double bonds is |
| 498 Cer(2H) | determined by the measured mass C13:0(OH) |
| N-C14:0(OH)- | Dihydroceramide: chain length and number of double bonds is |
| 499 Cer(2H) | determined by the measured mass C14:0(OH) |
| N-C 15:0(OH)- | Dihydroceramide: chain length and number of double bonds is |
| 500 Cer(2H) | determined by the measured mass C15:0(OH) |
| N-C 16:0(OH)- | Dihydroceramide: chain length and number of double bonds is |
| 501 Cer(2H) | determined by the measured mass C16:0(OH) |
| N-C17:0(OH)- | Dihydroceramide: chain length and number of double bonds is |
| 502 Cer(2H) | determined by the measured mass C17:0(OH) |
| N-C18:0(OH)- | Dihydroceramide: chain length and number of double bonds is |
| 503 Cer(2H) | determined by the measured mass C18:0(OH) |
| N-C 19:0(OH)- | Dihydroceramide: chain length and number of double bonds is |
| 504 Cer(2H) | determined by the measured mass C19:0(OH) |
| N-C20:0(OH)- | Dihydroceramide: chain length and number of double bonds is |
| 505 Cer(2H) | determined by the measured mass C20:0(OH) |
| N-C21:0(OH)- | Dihydroceramide: chain length and number of double bonds is |
| 506 Cer(2H) | determined by the measured mass C21:0(OH) |
| N-C22:0(OH)- | Dihydroceramide: chain length and number of double bonds is |
| 507 Cer(2H) | determined by the measured mass C22:0(OH) |
| N-C23:0(OH)- | Dihydroceramide: chain length and number of double bonds is |
| 508 Cer(2H) | determined by the measured mass C23:0(OH) |
| N-C24:0(OH)- | Dihydroceramide: chain length and number of double bonds is |
| 509 Cer(2H) | determined by the measured mass C24:0(OH) |
| N-C25:0(OH)- | Dihydroceramide: chain length and number of double bonds is |
| 510 Cer(2H) | determined by the measured mass C25:0(OH) |
| N-C26:0(OH)- | Dihydroceramide: chain length and number of double bonds is |
| 511 Cer(2H) | determined by the measured mass C26:0(OH) |
| N-C27:0(OH)- | Dihydroceramide: chain length and number of double bonds is |
| 512 Cer(2H) | determined by the measured mass C27:0(OH) |
| N-C28:0(OH)- | Dihydroceramide: chain length and number of double bonds is |
| 513 Cer(2H) | determined by the measured mass C28:0(OH) |
| 514 Histamine | Histamine |
| 515 Serotonin | Serotonin |
| 516 PEA | Phenylethylamine |
| 517 TXB2 | Tromboxane B2 |
| 518 PGF2a | Prostaglandin F2alpha |
| 519 24,25,EPC | 24,25-Epoxycholesterol |
| 520 5B,6B,EPC | 5β,6β-Epoxycholesterol |
| 52124DHLan | 24-Dihydrolanosterol |
| 522 GCDCA | Glycochenodeoxycholic Acid |
| 523 GLCA | Glycolithocholic Acid |
| 524 TCDCA | Taurochenodeoxycholic Acid |
| 525 TLCA | Taurolithocholic Acid |
| 526 GCA | Glycocholic Acid |
| 527 CA | Cholic Acid |
| 528 UDCA | Ursodeoxycholic Acid |
| 529 CDCA | Chenodeoxycholic Acid |
| 530 DCA | Deoxycholic Acid |
| 531 TDCA | Taurodeoxycholic Acid |
| 532 TLCAS | Taurolithocholic Acid sulfate |
| 533 GDCA | Glycodeoxycholic Acid |
| 534 GUDCA | Glycoursodeoxycholic Acid |

## Claims

1. Method for predicting the likelihood of onset of an inflammation associated organ failure from a biological sample of a mammalian subject in vitro, wherein
a. the subject's quantitative metabolomics profile comprising one or a plurality of endogenous metabolites, is detected in the biological sample by means of quantitative metabolomics analysis, and
b. the quantitative metabolomics profile of the subject's sample is compared with a quantitative reference metabolomics profile of one or a plurality of endogenous organ failure predictive target metabolites in order to predict whether the subject is likely or unlikely to develop an organ failure; and
c. wherein said endogenous organ failure predictive target metabolites have a molecular mass less than 1500 Da and are selected from the group consisting of: Amino acids, in particular, arginine, aspartic acid, citrulline, glutamic acid (glutamate), glutamine, leucine, isoleucine, histidine, ornithine, proline, phenylalanine, serine, tryptophane, tyrosine, valine, kynurenine;
phenylthio carbamyl amino acids (PTC-amino acids), in particular, PCT-arginine, PTC-glutamine, PTC-histidine, PTC-methionine, PTC-ornithine, PTC-phenylalanine, PTC-proline, PTC-serine, PTC-tryptophane, PTC-tyrosine, PTC-valine;
dimethylarginine, in particular N,N-dimethyl-L-arginine;
carboxylic acids, namely 15(S)-hydroxy-5Z,8Z,11Z,13E-eicosatetraenoic acid [(5Z,8Z,11Z,13E,15S)-15-Hydroxyicosa-5,8,11,13-tetraenoic acid], succinic acid (succinate);
Ceramides, with an N-acyl residue having from 2 to 30 Carbon atoms in the acyl residue and having from 0 to 5 double bonds and having from 0 to 5 hydroxy groups;
carnitine; acylcarnitines having from 1 to 20 carbon atoms in the acyl residue; acylcarnitines having from 3 to 20 carbon atoms in the acyl residue and having 1 to 4 double bonds in the acyl residue; acylcarnitines having from 1 to 20 carbon atoms in the acyl residue and having from 1 to 3 OH-groups in the acyl residue; acylcarnitines having from 3 to 20 carbon atoms in the acyl residue with 1 to 4 double bonds and 1 to 3 OH-groups in the acyl residue;
phospholipides, in particular lysophosphatidylcholines (monoacylphospha-tidylcholines) having from 1 to 30 carbon atoms in the acyl residue; lysophosphatidylcholines having from 3 to 30 carbon atoms in the acyl residue and having 1 to 6 double bonds in the acyl residue;
phosphatidylcholines (diacylphosphatidylcholines) having a total of from 1 to 50 carbon atoms in the acyl residues; phosphatidylcholines having a total from 3 to 50 carbon atoms in the acyl residues and having a total of 1 to 8 double bonds in the acyl residues;
sphingolipids, in particular sphingomyelines having a total number of carbon atoms in the acyl chains from 10 to 30; sphingomyelines having a total number of carbon atoms in the acyl chains from 10 to 30 and 1 to 5 double bonds; hydroxysphinogomyelines having a total number of carbon atoms in the acyl residues from 10 to 30; hydroxysphingoyelines having a total number of carbon atoms in the acyl residues from 10 to 30 and 1 to 5 double bonds;
prostaglandines, namely 6-keto-prostaglandin F1 alpha, prostaglandin D2, thromboxane B2;
putrescine;
oxysterols, namely 22-R-hydroxycholesterol, 24-S-hydroxycholesterol, 25-hydroxycholesterol, 27- hydroxycholesterol, 20α- hydroxycholesterol, 22-S-hydroxycholesterol, 24,25- epoxycholesterol,3β,5α,6β- trihydroxycholesterol, 7α-hydroxycholesterol, 7-Ketocholesterol, 5β,6β- epoxycholesterol, 5α,6α-epoxycholesterol, 4β- hydroxycholesterol, desmosterol (vitamin D3), 7-dehydrocholesterol, cholestenone, lanosterol, 24-dehydrolanosterol;
bile acids, namely cholic acid, chenodeoxycholic acid, deoxycholic acid, glycocholic acid, glycochenodeoxycholic acid, glycodeoxycholic acid, glycolithocholic acid, glycolithocholic acid sulfate, glycoursodeoxycholic acid, lithocholic acid, taurocholic acid, taurochenodeoxycholic acid taurodeoxycholic acid, taurolithocholic acid, taurolithocholic acid sulfate, tauroursodeoxycholic acid, ursodeoxycholic acid; biogenic amines, namely histamine, serotonine, palmitoyl ethanolamine.

2. Method according to claim 1, wherein inflammation associated organ failure comprises infection associated organ failure and/or sepsis associated organ failure.

3. Method according to claim1 or 2, wherein the biological sample is selected from the group consisting of stool; body fluids, in particular blood, liquor, cerebrospinal fluid, urine, ascitic fluid, seminal fluid, saliva, puncture fluid, cell content, tissue samples, in particular liver biopsy material; or a mixture thereof.

4. Method according to anyone of claims 1 to 3, wherein said quantitative metabolomics profile is achieved by a quantitative metabolomics profile analysis method comprising the generation of intensity data for the quantitation of endogenous metabolites by mass spectrometry (MS), in particular, by high- throughput mass spectrometry, preferably by MS-technologies such as Matrix Assisted Laser Desorption/lonisation (MALDI), Electro Spray Ionization (ESI), Atmospheric Pressure Chemical Ionization (APCI), ¹H-, ¹³C- and/or ³¹P- Nuclear Magnetic Resonance spectroscopy (NMR), optionally coupled to MS, determination of metabolite concentrations by use of MS-technologies and/or methods coupled to separation, in particular Liquid Chromatography (LC-MS), Gas Chromatography (GC-MS), or Capillary Electrophoresis (CE-MS).

5. Method according to anyone of claims 1 to 4, wherein intensity data of said metabolomics profile are normalized with a set of endogenous housekeeper metabolites by relating detected intensities of the selected endogenous organ failure predictive target metabolites to intensities of said endogenous housekeeper metabolites.

6. Method according to claim 5, wherein said endogenous housekeeper metabolites are selected from the group consisting of such endogeneous metabolites which show stability in accordance with statistical stability measures being selected from the group consisting of coefficient of variation (CV) of raw intensity data, standard deviation (SD) of logarithmic intensity data, stability measure (M) of geNorm - algorithm or stability measure value (rho) of NormFinder-algorithm.

7. Method according to anyone of claims 1 to 6, wherein said quantitative metabolomics profile comprises the results of measuring at least one of the parameters selected from the group consisting of: concentration, level or amount of each individual endogenous metabolite of said plurality of endogenous metabolites in said sample, qualitative and/or quantitative molecular pattern and/or molecular signature; and using and storing the obtained set of values in a database.

8. Method according to anyone of claims 1 to 7, wherein a panel of reference endogenous organ failure predictive target metabolites or derivatives thereof is established by:
a) mathematically preprocessing intensity values obtained for generating the metabolomics profiles in order to reduce technical errors being inherent to the measuring procedures used to generate the metabolomics profiles;
b) selecting at least one suitable classifying algorithm from the group consisting of logistic regression, (diagonal) linear or quadratic discriminant analysis (LDA, QDA, DLDA, DQDA), perceptron, shrunken centroids regularized discriminant analysis (RDA), random forests (RF), neural networks (NN), Bayesian networks, hidden Markov models, support vector machines (SVM), generalized partial least squares (GPLS), partitioning around medoids (PAM), inductive logic programming (ILP), generalized additive models, gaussian processes, regularized least square regression, self organizing maps (SOM), recursive partitioning and regression trees, K-nearest neighbour classifiers (K-NN), fuzzy classifiers, bagging, boosting, and naïve Bayes; and applying said selected classifier algorithm to said preprocessed data of step a);
c) said classifier algorithms of step b) being trained on at least one training data set containing preprocessed data from subjects being divided into classes according to their likelihood to develop an organ failure, in order to select a classifier function to map said preprocessed data to said likelihood;
d) applying said trained classifier algorithms of step c) to a preprocessed data set of a subject with unknown organ failure likelihood, and using the trained classifier algorithms to predict the class label of said data set in order to predict the likelihood for a subject to develop an organ failure.

9. Method according to anyone of claims 1 to 8, wherein said endogenous organ failure predictive target metabolites for easier and/or more sensitive detection are detected by means of chemically modified derivatives thereof, such as phenyisothiocyanates for amino acids.

10. Method according to anyone of claims 1 to 9, wherein said endogenous organ failure predictive target metabolites are selected from the group consisting of:
Carnitin, acylcarnitines (C chain length:total number of double bonds), in particular, C12-DC, C14:1, C14:1-OH, C14:2, C14:2-OH, C18, C6:1;
sphingomyelins (SM chain length:total number of double bonds), in particular, SM C16:0, SM C17:0, SM C18:0, SM C19:0, SM C21:1, SM C21:3, SM C22:2, SM C23:0, SM C23:1, SM C23:2, SM C23:3, SM C24:0, SM C24:1, SM C24:2, SM C24:3, SM C24:4, SM C26:4, SM C3:0, SM (OH) C22:1, SM (OH) C22:2, SM (OH) C24:1, SM C26:0, SM C26:1;
phosphatidylcholines, (diacylphosphatidylcholines, PC aa chain length:total number of double bonds or PC ae) in particular, PC aa C28:1, PC aa C38:0, PC aa C42:0, PC aa C42:1, PC ae C40:1, PC ae C40:2, PC ae C40:6, PC ae C42:2, PC ae C42:3, PC ae C42:4, PC ae C44:5, PC ae C44:6, PC aa C36:4, PC aa C38:1, PC aa C38:2, PC aa C38:4, PC aa C38:5, PC aa C38:6, PC aa C40:5, PC aa C40:6, PC aa C40:7, PC aa C40:8, PC ae C36:4, PC ae C36:5, PC ae C38:4, PC ae C38:6;
lysophosphatidylcholines (monoacylphosphatidylcholines, PC a chain length:total number of double bonds), in particular, PC a C18:2, PC a C20:4, PC a C20:3, PC a C26:0;
Phe;
oxycholesterols, in particular, 3β,5α,6β-trihydroxycholestan, 7-ketocholesterol, 5α,6α-epoxycholesterol;
lysophosphatidylethanolamins (monoacylphosphatidylcholins, PE a chain length:total number of double bonds), in particular, PE a C18:1, PE a C18:2, PE a C20:4, PE a C22:5, PE a C22:6;
phosphatidylethanolamins, (diacylphosphatidylcholins, PE aa chain length:total number of double bonds),in particular, PE aa C38:0, PE aa C38:2;
ceramids, (N-chain length:total number of double bonds),in particular, N-C2:0-Cer, N-C7:0-Cer, N-C9:3-Cer, N-C17:1-Cer, N-C22:1-Cer, N-C25:0-Cer, N-C27:1-Cer, N-C5:1-Cer(2H), N-C7:1-Cer(2H), N-C8:1-Cer(2H), N-C11:1-Cer(2H), N-C20:0-Cer(2H), N-C21:0-Cer(2H), N-C22:1-Cer(2H), N-C25:1-Cer(2H), N-C26:1-Cer(2H), N-C24:0(OH)-Cer, N-C26:0(OH)-Cer, N-C6:0(OH)-Cer, N-C8:0(OH)-Cer(2H), N-C10:0(OH)-Cer(2H), N-C25:0(OH)-Cer(2H), N-C26:0(OH)-Cer(2H), N-C27:0(OH)-Cer(2H), N-C28:0(OH)-Cer(2H).

11. Method according to anyone of claims 1 to 10, wherein said plurality of endogenous organ failure predictive target metabolites or derivatives thereof comprises 2 to 80, in particular 2 to 60, preferably 2 to 50, preferred 2 to 30, more preferred 2 to 20, particularly preferred 2 to 10 endogenous metabolites

12. Use of one or a plurality of endogenous metabolites for predicting of an onset of an infection associated organ failure from a biological sample of a mammalian subject in vitro, wherein the metabolites are selected from the group consisting of :
Amino acids, in particular, arginine, aspartic acid, citrulline, glutamic acid (glutamate), glutamine, leucine, isoleucine, histidine, ornithine, proline, phenylalanine, serine, tryptophane, tyrosine, valine, kynurenine;
phenylthio carbamyl amino acids (PTC-amino acids), in particular, PCT-arginine, PTC-glutamine, PTC-histidine, PTC-methionine, PTC-ornithine, PTC-phenylalanine, PTC-proline, PTC-serine, PTC-tryptophane, PTC-tyrosine, PTC-valine;
dimethylarginine, in particular N,N-dimethyl-L-arginine;
carboxylic acids, namely 15(S)-hydroxy-5Z,8Z,11Z,13E-eicosatetraenoic acid [(5Z,8Z,11Z,13E,15S)-15-Hydroxyicosa-5,8,11,13-tetraenoic acid], succinic acid (succinate);
Ceramides, with an N-acyl residue having from 2 to 30 Carbon atoms in the acyl residue and having from 0 to 5 double bonds and having from 0 to 5 hydroxy groups;
carnitine; acylcarnitines having from 1 to 20 carbon atoms in the acyl residue; acylcarnitines having from 3 to 20 carbon atoms in the acyl residue and having 1 to 4 double bonds in the acyl residue; acylcarnitines having from 1 to 20 carbon atoms in the acyl residue and having from 1 to 3 OH-groups in the acyl residue; acylcarnitines having from 3 to 20 carbon atoms in the acyl residue with 1 to 4 double bonds and 1 to 3 OH-groups in the acyl residue;
phospholipides, in particular lysophosphatidylcholines (monoacylphospha-tidylcholines) having from 1 to 30 carbon atoms in the acyl residue; lysophosphatidylcholines having from 3 to 30 carbon atoms in the acyl residue and having 1 to 6 double bonds in the acyl residue;
phosphatidylcholines (diacylphosphatidylcholines) having a total of from 1 to 50 carbon atoms in the acyl residues; phosphatidylcholines having a total from 3 to 50 carbon atoms in the acyl residues and having a total of 1 to 8 double bonds in the acyl residues;
sphingolipids, in particular sphingomyelines having a total number of carbon atoms in the acyl chains from 10 to 30; sphingomyelines having a total number of carbon atoms in the acyl chains from 10 to 30 and 1 to 5 double bonds; hydroxysphinogomyelines having a total number of carbon atoms in the acyl residues from 10 to 30;
hydroxysphingoyelines having a total number of carbon atoms in the acyl residues from 10 to 30 and 1 to 5 double bonds;
prostaglandines, namely 6-keto-prostaglandin F1 alpha, prostaglandin D2, thromboxane B2;
putrescine;
oxysterols, namely 22-R-hydroxycholesterol, 24-S-hydroxycholesterol, 25-hydroxycholesterol, 27- hydroxycholesterol, 20α- hydroxycholesterol, 22-S-hydroxycholesterol, 24,25- epoxycholesterol,3β,5α,6β- trihydroxycholesterol, 7α-hydroxycholesterol, 7-Ketocholesterol, 5β,6β- epoxycholesterol, 5α,6α-epoxycholesterol, 4β- hydroxycholesterol, desmosterol (vitamin D3), 7-dehydrocholesterol, cholestenone, lanosterol, 24-dehydrolanosterol;
bile acids, namely cholic acid, chenodeoxycholic acid, deoxycholic acid, glycocholic acid, glycochenodeoxycholic acid, glycodeoxycholic acid, glycolithocholic acid, glycolithocholic acid sulfate, glycoursodeoxycholic acid, lithocholic acid, taurocholic acid, taurochenodeoxycholic acid taurodeoxycholic acid, taurolithocholic acid, taurolithocholic acid sulfate, tauroursodeoxycholic acid, ursodeoxycholic acid;
biogenic amines, namely histamine, serotonine, palmitoyl ethanolamine.

13. Use according to claim 12, wherein said endogenous organ failure predictive target metabolites for easier and/or more sensitive detection are detected by means of chemically modified derivatives thereof, such as phenylisothiocyanates for amino acids;

14. Use according to claim 11 to 13, wherein said endogenous organ failure predictive target metabolites are selected from the group consisting of:
Carnitin, acylcarnitines (C chain length:total number of double bonds), in particular, C12-DC, C14:1, C14:1-OH, C14:2, C14:2-OH, C18, C6:1;
sphingomyelins (SM chain length:total number of double bonds), in particular, SM C16:0, SM C17:0, SMC18:0, SM C19:0, SM C21:1, SM C21:3, SM C22:2, SM C23:0, SM C23:1, SM C23:2, SM C23:3, SM C24:0, SM C24:1, SM C24:2, SM C24:3, SM C24:4, SM C26:4, SM C3:0, SM (OH) C22:1, SM (OH) C22:2, SM (OH) C24:1, SM C26:0, SM C26:1;
phosphatidylcholines, (diacylphosphatidylcholines, PC aa chain length:total number of double bonds or PC ae) in particular, PC aa C28:1, PC aa C38:0, PC aa C42:0, PC aa C42:1, PC ae C40:1, PC ae C40:2, PC ae C40:6, PC ae C42:2, PC ae C42:3, PC ae C42:4, PC ae C44:5, PC ae C44:6, PC aa C36:4, PC aa C38:1, PC aa C38:2, PC aa C38:4, PC aa C38:5, PC aa C38:6, PC aa C40:5, PC aa C40:6, PC aa C40:7, PC aa C40:8, PC ae C36:4, PC ae C36:5, PC ae C38:4, PC ae C38:6;
lysophosphatidylcholines (monoacylphosphatidylcholines, PC a chain length:total number of double bonds), in particular, PC a C18:2, PC a C20:4, PC a C20:3, PC a C26:0;
Phe;
oxycholesterols, in particular, 3β,5α,6β-trihydroxychotestan, 7-ketocholesterol, 5α,6α-epoxycholesterol;
lysophosphatidylethanolamins (monoacylphosphatidylcholins, PE a chain length:total number of double bonds), in particular, PE a C18:1, PE a C18:2, PE a C20:4, PE a C22:5, PE a C22:6;
phosphatidylethanolamins, (diacylphosphatidylcholins, PE aa chain length:total number of double bonds),in particular, PE aa C38:0, PE aa C38:2;
ceramids, (N-chain length:total number of double bonds),in particular, N-C2:0-Cer, N-C7:0-Cer, N-C9:3-Cer, N-C17:1-Cer, N-C22:1-Cer, N-C25:0-Cer, N-C27:1-Cer, N-C5:1-Cer(2H), N-C7:1-Cer(2H), N-C8:1-Cer(2H), N-C11:1-Cer(2H), N-C20:0-Cer(2H), N-C21:0-Cer(2H)_{;} N-C22:1-Cer(2H), N-C25:1-Cer(2H), N-C26:1-Cer(2H), N-C24:0(OH)-Cer, N-C26:0(OH)-Cer, N-C6:0(OH)-Cer, N-C8:0(OH)-Cer(2H), N-C10:0(OH)-Cer(2H), N-C25:0(OH)-Cer(2H), N-C26:0(OH)-Cer(2H), N-C27:0(OH)-Cer(2H), N-C28:0(OH)-Cer(2H).

15. Kit for carrying out a method in accordance with anyone of claims 1 to 11, in a biological sample, comprising:
a) calibration agents for the quantitative detection of endogenous organ failure predictive target metabolites, wherein said metabolites are selected from the group consisting of: Amino acids, in particular, arginine, aspartic acid, citrulline, glutamic acid (glutamate), glutamine, leucine, isoleucine, histidine, ornithine, proline, phenylalanine, serine, tryptophane, tyrosine, valine, kynurenine;
phenylthio carbamyl amino acids (PTC-amino acids), in particular, PCT-arginine, PTC-glutamine, PTC-histidine, PTC-methionine, PTC-ornithine, PTC-phenylalanine, PTC-proline, PTC-serine, PTC-tryptophane, PTC-tyrosine, PTC-valine;
dimethylarginine, in particular N,N-dimethyl-L-arginine;
carboxylic acids, namely 15(S)-hydroxy-5Z,8Z,11Z,13E-eicosatetraenoic acid [(5Z,8Z,11Z,13E,15S)-15-Hydroxyicosa-5,8,11,13-tetraenoic acid], succinic acid (succinate);
Ceramides, with an N-acyl residue having from 2 to 30 Carbon atoms in the acyl residue and having from 0 to 5 double bonds and having from 0 to 5 hydroxy groups;
carnitine; acylcarnitines having from 1 to 20 carbon atoms in the acyl residue; acylcarnitines having from 3 to 20 carbon atoms in the acyl residue and having 1 to 4 double bonds in the acyl residue; acylcarnitines having from 1 to 20 carbon atoms in the acyl residue and having from 1 to 3 OH-groups in the acyl residue; acylcarnitines having from 3 to 20 carbon atoms in the acyl residue with 1 to 4 double bonds and 1 to 3 OH-groups in the acyl residue;
phospholipides, in particular lysophosphatidylcholines (monoacylphospha-tidylcholines) having from 1 to 30 carbon atoms in the acyl residue; lysophosphatidylcholines having from 3 to 30 carbon atoms in the acyl residue and having 1 to 6 double bonds in the acyl residue;
phosphatidylcholines (diacylphosphatidylcholines) having a total of from 1 to 50 carbon atoms in the acyl residues; phosphatidylcholines having a total from 3 to 50 carbon atoms in the acyl residues and having a total of 1 to 8 double bonds in the acyl residues;
sphingolipids, in particular sphingomyelines having a total number of carbon atoms in the acyl chains from 10 to 30; sphingomyelines having a total number of carbon atoms in the acyl chains from 10 to 30 and 1 to 5 double bonds; hydroxysphinogomyelines having a total number of carbon atoms in the acyl residues from 10 to 30;
hydroxysphingoyelines having a total number of carbon atoms in the acyl residues from 10 to 30 and 1 to 5 double bonds;
prostaglandines, namely 6-keto-prostaglandin F1alpha, prostaglandin D2, thromboxane B2;
putrescine;
oxysterols, namely 22-R-hydroxycholesterol, 24-S-hydroxycholesterol, 25-hydroxycholesterol, 27- hydroxycholesterol, 20α- hydroxycholesterol, 22-S-hydroxycholesterol, 24,25- epoxycholesterol,3β,5α,6β- trihydroxycholesterol, 7α-hydroxycholesterol, 7-Ketocholesterol, 5β,6β- epoxycholesterol, 5α,6α-epoxycholesterol, 4β- hydroxycholesterol, desmosterol (vitamin D3), 7-dehydrocholesterol, cholestenone, lanosterol, 24-dehydrolanosterol;
bile acids, namely cholic acid, chenodeoxycholic acid, deoxycholic acid, glycocholic acid, glycochenodeoxycholic acid, glycodeoxycholic acid, glycolithocholic acid, glycolithocholic acid sulfate, glycoursodeoxycholic acid, lithocholic acid, taurocholic acid, taurochenodeoxycholic acid taurodeoxycholic acid, taurolithocholic acid, taurolithocholic acid sulfate, tauroursodeoxycholic acid, ursodeoxycholic acid;
biogenic amines, namely histamine, serotonine, palmitoyl ethanolamine;
b) data base with processed data from healthy patients and patients who developed an infection associated organ failure;
c) classification software for generating the quantitative metabolomics profiles achieved with said calibration agents of step a) and classifying the results based on the processed data of step b).
